# EUROPEAN PATENT APPLICATION

(11) **EP 1 839 655 A1**
(43) Date of publication of application: **03.10.2007**
(21) Application number: 06711930.5
(22) Date of filing: 19.01.2006
(51) Int. Cl.: A61K 31/167, A61K 31/17, A61K 31/18, A61K 31/27, A61K 31/277, A61K 31/341, A61K 31/343, A61K 31/36, A61K 31/381, A61K 31/382, A61K 31/395, A61K 31/40

(54) **CTGF EXPRESSION INHIBITOR**

(30) Priority: 20.01.2005 JP 2005012529
(71) Applicant: Shionogi Co., Ltd., Osaka-shi, Osaka 541-0045 (JP)
(72) Inventor: SENO, Kaoru, hima-ku, Osaka-shi, Osaka, 5530002 (JP); SHINOSAKI, Toshihiro, onaka-shi, Osaka, 5610825 (JP); HATA, Satoshi, onaka-shi, Osaka, 5610825 (JP); YAMADA, Isamu, hima-ku, Osaka-shi, Osaka, 5530002 (JP); SATO, Hiroki, hima-ku, Osaka-shi, Osaka, 5530002 (JP); KATAOKA, Mikayo, hima-ku, Osaka-shi, Osaka, 5530002 (JP)
(74) Representative: Hayes, Adrian Chetwynd
(86) International application number: PCT/JP2006/300684
(87) International publication number: WO 2006/077901

(57) **Abstract**

A CTGF expression inhibitor comprising a compound of the formula I: 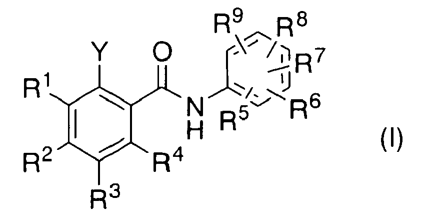 a pharmaceutically acceptable salt or solvate thereof as an active ingredient,
(wherein Y is hydroxy or a group of the formula: -NH-SO₂-Y' (wherein Y' is optionally substituted aryl or optionally substituted alkyl), and
R¹ to R⁹ are each independently hydrogen, halogen, optionally substituted alkyl, optionally substituted alkoxy or the like).

## Description

### Field of the Invention

This invention relates to a compound with inhibitory activity on a connective tissue growth factor (hereinafter referred to as CTGF) production and a pharmaceutical composition comprising it.

### Background Art

Transforming growth factor-β (TGF-β) is known as an important cytokine for organ fibrosis. In kidney, increased expression of TGF-β has been reported to correspond with fibrotic area in experimental animal models or human biopsy tissue. Additionally, suppression of renal stromal fibrosis with neutralizing antibody of TGF-β in experimental models has been confirmed. Furthermore, importance of TGF-β has been noted not only in kidney but also in each organ such as skin, liver, lung or heart.
However, as TGF-β has not only fibrotic effect but also various functions such as anti-inflammatory or immunosuppression, TGF-β1 knockout mice cannot survive so long after birth due to the induction of multiple organ dysfunction with remarkable inflammation. Therefore, reducing biological action of TGF-β for a long time is difficult to be adapted for clinical use. It is thought that specific suppression of other cytokine than TGF-β1 is clinically more desirable.
Recently, CTGF was identified as a downstream gene of TGF-β cell-signaling. CTGF is 38 kDa protein consisting of 349 amino acid residues isolated from human umbilical vein endothelial cells. In later reports, it was confirmed that CTGF is induced in fibroblasts as well as in endothelial cells, and a role of CTGF in organ fibrosis has been investigated.
It is known that CTGF is induced by TGF-β and has bioactivity for such as cell proliferation, increased chemotaxis, apoptosis induction or angiogenesis promotion other than production of extracellular matrix such as type I collagen or fibronectin. Additionally, it is known that CTGF expresses at a high level in diffuse or localized sclerema, keloid, atherosclerosis, biliary atresia or the like in human in addition to bleomycin-induced pulmonary fibrosis disease model in mice. Taken together, CTGF is thought to be implicated in tissue fibrosis specifically. It is hypothesized that TGF-β induces CTGF production in fibroblasts, mesangial cells or epithelial cells, eventually leading to the formation of tissue fibrosis by enhancing collagen or fibronectin production.
Therefore, CTGF is focused as a more specific therapeutic target for the treatment of organ fibrosis (Non-patent Document 1 and 2).

Compounds of the present invention are benzanilide derivatives, and the followings have been known as a benzanilide derivative.
For example, Non-patent Document 3 discloses benzanilide derivatives which are compounds of the present invention wherein R² and R³ are taken together with the neighboring carbon atom to form a ring. Non-patent Document 4 discloses benzanilide derivatives which are compounds of the present invention wherein Y is carboxamide. Patent Document 1 discloses benzanilide derivatives which are compounds of the present invention wherein Y is hydrazo. Patent Document 2 discloses benzanilide derivatives which are compounds of the present invention wherein R¹ is hydroxyl, and both R⁷ and R⁸ are hydrogen. Patent Document 3 discloses benzanilide derivatives can be used as an antidiabetic drug.
However, the above documents neither disclose nor suggest that these compounds have inhibitory activity on CTGF expression.
[Patent Document 1] JP2003-34671
[Patent Document 2] JP1996-143525
[Patent Document 3] WO03/10364
[Non-patent Document 1] Igakuno Ayumi, Vol. 190, No. 1, 1999.7.3
[Non-patent Document 2] Igakuno Ayumi, Vol. 201, No. 12, 2002.6.22
[Non-patent Document 3] Anal. Chem. 1994, 66, 1347-1353
[Non-patent Document 4] Journal of Magnetic resonance 72, 316-320 (1988)

### Disclosure of Invention

### Problems to be solved by the Invention

The present invention provides a compound with inhibitory activity on CTGF expression, pharmaceutically acceptable salt, solvate thereof and a pharmaceutical composition comprising them.

### Means for Solving the Problem

The present inventors found compounds with inhibitory activity on CTGF expression to accomplish the following invention.
(1) A CTGF expression inhibitor comprising a compound of the formula I: 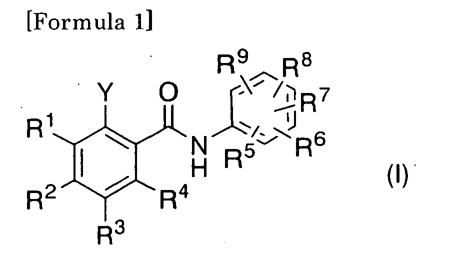 a pharmaceutically acceptable salt or solvate thereof as an active ingredient, (wherein Y is hydroxy or a group of the formula: -NH-SO₂-Y' (wherein Y' is optionally substituted aryl or optionally substituted alkyl),
   R¹ is hydrogen, optionally substituted alkyl, optionally substituted amino, nitro, optionally substituted alkoxy, halogen, optionally substituted alkenyl or optionally substituted alkynyl,
   R² is hydrogen, halogen, nitro, optionally substituted amino, cyano, optionally substituted alkyl, optionally substituted carbamoyl, hydroxy, optionally substituted alkenyl, optionally substituted alkynyl, optionally substituted aryl or
   a group of the formula: -O-R^{2'} (wherein R^{2'} is optionally substituted alkyl, alkylsulfonyl, cycloalkyl, optionally substituted nonaromatic heterocycle or heteroaryl), or
   R¹ and R² can be taken together with the neighboring carbon atom to form an optionally substituted 5 or 6-membered ring optionally containing heteroatom(s),
   R³ is hydrogen, halogen, cyano, optionally substituted sulfamoyl, optionally substituted carbamoyl, optionally substituted amino, nitro, optionally substituted alkyl, optionally substituted alkoxy, optionally substituted alkenyl, optionally substituted aryl, optionally substituted heteroaryl, optionally substituted nonaromatic heterocycle or
   a group of the formula: C≡C-R^{3'} (wherein R^{3'} is hydrogen, optionally substituted aryl, optionally substituted heteroaryl, hydroxy or optionally substituted alkyl),
   R⁴ is hydrogen, halogen, optionally substituted alkyl, optionally substituted alkoxy, optionally substituted alkenyl, optionally substituted alkynyl or optionally substituted alkylthio,
   R⁵ is hydrogen, carbamoyl, cyano, nitro, halogen, alkyl, alkenyl, optionally substituted alkoxycarbonylamino, alkoxy, haloalkoxy, alkylthio, alkylsulfinyl, alkylsulfonyl, optionally substituted amino, optionally substituted aryl, optionally substituted heteroaryl, optionally substituted nonaromatic heterocycle,
   a group of the formula: -X'-R^{5'} (wherein X' is -C≡C-, and R^{5'} is optionally substituted aryl, optionally substituted heteroaryl, optionally substituted nonaromatic heterocycle, optionally substituted alkyl, alkoxy, hydroxy or hydrogen) or
   a group of the formula: -X"-R^{5"} (wherein X" is -O-Z-, -S-Z-, -C(=O)-, -SO-Z-, -SO₂-Z-, -NRSO₂-, -NRC(=O)-, -SO₂NR-, -C(=O)NR-, -CR(OH)-, -SO₂O⁻ or -NR-, R^{5"} is optionally substituted aryl, optionally substituted heteroaryl or optionally substituted nonaromatic heterocycle, R is hydrogen or alkyl, and Z is a bond or alkylene), and
   R⁶, R⁷, R⁸ and R⁹ are each independently hydrogen, optionally substituted alkyl, optionally substituted alkenyl, optionally substituted alkynyl, halogen, optionally substituted alkoxy, cyano, nitro, optionally substituted amino, optionally substituted aryl or nonaromatic heterocycle).

Especially, the following embodiments are preferable.
(2) The CTGF expression inhibitor of (1), wherein a group of the formula: 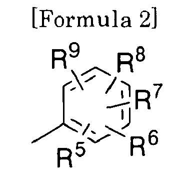 is a group of the formula: 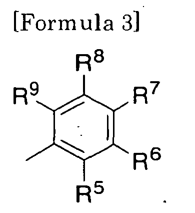
(3) The CTGF expression inhibitor of (2), wherein
   R⁶ is hydrogen, optionally substituted alkyl or halogen,
   R⁷ is hydrogen, optionally substituted alkoxy, halogen, cyano, nitro, optionally substituted alkyl, optionally substituted alkenyl, optionally substituted alkynyl or nonaromatic heterocycle,
   R⁸ is hydrogen, nitro, optionally substituted amino, halogen, optionally substituted alkyl, optionally substituted alkoxy, optionally substituted alkenyl, optionally substituted alkynyl, cyano or haloalkoxy, and
   R⁹ is hydrogen, alkyl, halogen or optionally substituted aryl.
(4) The CTGF expression inhibitor of (3), wherein
   R⁵ is hydrogen, halogen, optionally substituted alkyl, alkoxycarbonylamino, alkoxy, haloalkoxy, alkylthio, alkylsulfinyl, alkylsulfonyl, optionally substituted amino, optionally substituted aryl, optionally substituted heteroaryl, optionally substituted nonaromatic heterocycle,
   a group of the formula: -X'-R^{5'} (wherein X' is -C≡C-, and R^{5'} is optionally substituted aryl, optionally substituted heteroaryl, optionally substituted nonaromatic heterocycle, optionally substituted alkyl, alkoxy, hydroxy or hydrogen) or
   a group of the formula: -X"-R^{5"} (wherein X" is -O-Z-, -S-Z-, -C(=O)-, -SO-Z-, -SO₂-Z--NRSO₂-, -NRC(=O)-, -SO₂NR- -C(=O)NR-, -CR(OH)-, -SO₂O- or -NR-, R^{5"} is optionally substituted aryl, optionally substituted heteroaryl or optionally substituted nonaromatic heterocycle, R is hydrogen or alkyl and Z is a bond or alkylene).
(5) The CTGF expression inhibitor of (3), wherein
   R³ is hydrogen, halogen, optionally substituted aryl, optionally substituted heteroaryl, optionally substituted nonaromatic heterocycle or
   a group of the formula: -C≡C-R^{3'} (wherein R^{3'} is hydrogen, optionally substituted aryl, optionally substituted heteroaryl, hydroxy or optionally substituted alkyl).
(6) A compound of the formula II: 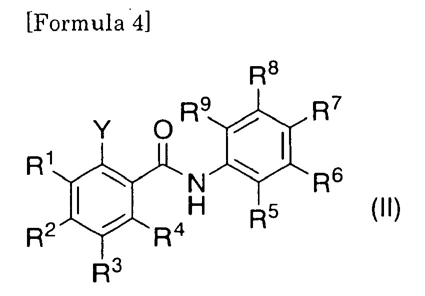 a pharmaceutically acceptable salt or solvate thereof, (wherein Y is hydroxy or a group of the formula: -NH-SO₂-Y' (wherein Y' is optionally substituted aryl or optionally substituted alkyl),
   R¹ is hydrogen, optionally substituted alkyl, optionally substituted amino, nitro, optionally substituted alkoxy, halogen, optionally substituted alkenyl or optionally substituted alkynyl,
   R² is hydrogen, halogen, nitro, optionally substituted amino, cyano, optionally substituted alkyl, optionally substituted carbamoyl, optionally substituted alkoxy, hydroxy, optionally substituted alkenyl, optionally substituted alkynyl or optionally substituted aryl, or
   R¹ and R² can be taken together with the neighboring carbon atom to form an optionally substituted 5 or 6-membered ring optionally containing heteroatom(s),
   R³ is a group of the formula: -C≡C-R^{3'} (wherein R^{3'} is hydrogen, optionally substituted aryl, optionally substituted heteroaryl, hydroxy or optionally substituted alkyl),
   R⁴ is hydrogen, halogen, optionally substituted alkyl, optionally substituted alkoxy, optionally substituted alkenyl, optionally substituted alkynyl or optionally substituted alkylthio,
   R⁵ is hydrogen, carbamoyl, cyano, nitro, halogen, alkyl, alkenyl, alkoxycarbonylamino, alkoxy, haloalkoxy, alkylthio, alkylsulfinyl, alkylsulfonyl, optionally substituted aryl, optionally substituted heteroaryl, optionally substituted nonaromatic heterocycle,
   a group of the formula: -X'-R^{5'} (wherein X' is -C≡C-, and R^{5'} is optionally substituted aryl, optionally substituted heteroaryl, optionally substituted nonaromatic heterocycle, optionally substituted alkyl, alkoxy, hydroxy or hydrogen) or
   a group of the formula: -X''-R^{5''} (wherein X" is -O-Z-, -S-Z-, -C(=O)-, -SO-Z-, -SO₂-Z-, -NRSO₂- -NRC(=O)-, -SO₂NR -C(=O)NR-, -CR(OH)-, -SO₂O- or -NR-, R^{5''} is optionally substituted aryl, optionally substituted heteroaryl or optionally substituted nonaromatic heterocycle, R is hydrogen or alkyl, and Z is a bond or alkylene),
   R⁶ is hydrogen, alkyl or halogen,
   R⁷ is hydrogen, optionally substituted alkoxy, halogen, cyano, nitro, optionally substituted alkyl, optionally substituted alkenyl or optionally substituted alkynyl,
   R⁸ is hydrogen, nitro, optionally substituted amino, halogen, optionally substituted alkyl, alkoxy, optionally substituted alkenyl, optionally substituted alkynyl, cyano or haloalkoxy, and
   R⁹ is hydrogen, alkyl, halogen or optionally substituted aryl).
(7) The compound of (6) wherein R⁸ is haloalkyl, a pharmaceutically acceptable salt or solvate thereof.
(8) The compound of (7) wherein R⁵ is substituted aryl, a pharmaceutically acceptable salt or solvate thereof.
(9) A compound of the formula II: 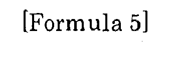 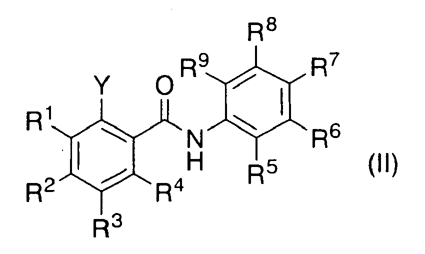 a pharmaceutically acceptable salt or solvate thereof, (wherein Y is hydroxy or a group of the formula: -NH-SO₂-Y' (wherein Y' is optionally substituted aryl or optionally substituted alkyl),
   R¹ is hydrogen, optionally substituted alkyl, optionally substituted amino, nitro, optionally substituted alkoxy, halogen, optionally substituted alkenyl or optionally substituted alkynyl,
   R² is hydrogen, halogen, nitro, optionally substituted amino, cyano, optionally substituted alkyl, optionally substituted carbamoyl, optionally substituted alkoxy, hydroxy, optionally substituted alkenyl, optionally substituted alkynyl or optionally substituted aryl, or
   R¹ and R² can be taken together with the neighboring carbon atom to form an optionally substituted 5 or 6-membered ring optionally containing heteroatom(s),
   R³ is hydrogen, halogen, cyano, optionally substituted sulfamoyl, optionally substituted carbamoyl, optionally substituted amino, nitro, optionally substituted alkyl, optionally substituted alkoxy, optionally substituted alkenyl, optionally substituted aryl, optionally substituted heteroaryl, optionally substituted nonaromatic heterocycle or
   a group of the formula: -C≡C-R^{3'} (wherein R^{3'} is hydrogen, optionally substituted aryl, optionally substituted heteroaryl, hydroxy or optionally substituted alkyl),
   R⁴ is hydrogen, halogen, optionally substituted alkyl, optionally substituted alkoxy, optionally substituted alkenyl, optionally substituted alkynyl or optionally substituted alkylthio,
   R⁵ is a group of the formula: -X'-R^{5'} (wherein X' is -C≡C-, and R⁵' is substituted aryl or optionally substituted alkyl), R⁶ is hydrogen, alkyl or halogen,
   R⁷ is hydrogen, optionally substituted alkoxy, halogen, cyano, nitro, optionally substituted alkyl, optionally substituted alkenyl or optionally substituted alkynyl,
   R⁸ is hydrogen, nitro, optionally substituted amino, halogen, optionally substituted alkyl, alkoxy, optionally substituted alkenyl, optionally substituted alkynyl, cyano or haloalkoxy, and
   R⁹ is hydrogen, alkyl, halogen or optionally substituted aryl).
(10) The compound of (9) wherein R² is halogen and R⁷ is haloalkyl, a pharmaceutically acceptable salt or solvate thereof.
(11) A compound of the formula II: 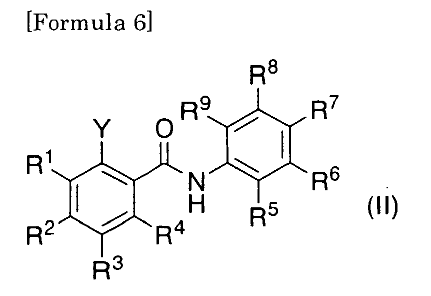 a pharmaceutically acceptable salt or solvate thereof,
   (wherein Y is hydroxy or a group of the formula: -NH-SO₂-Y' (wherein Y' is optionally substituted aryl or optionally substituted alkyl),
   R¹ is hydrogen, optionally substituted alkyl, optionally substituted amino, nitro, optionally substituted alkoxy, halogen, optionally substituted alkenyl or optionally substituted alkynyl,
   R² is hydrogen, halogen, nitro, optionally substituted amino, cyano, optionally substituted alkyl, optionally substituted carbamoyl, optionally substituted alkoxy, hydroxy, optionally substituted alkenyl, optionally substituted alkynyl or optionally substituted aryl, or
   R¹ and R² can be taken together with the neighboring carbon atom to form an optionally substituted 5 or 6-membered ring optionally containing heteroatom(s),
   R³ is substituted aryl or optionally substituted heteroaryl,
   R⁴ is hydrogen, halogen, optionally substituted alkyl, optionally substituted alkoxy, optionally substituted alkenyl, optionally substituted alkynyl or optionally substituted alkylthio,
   R⁵ is hydrogen, carbamoyl, cyano, nitro, halogen, alkyl, alkenyl, alkoxycarbonylamino, alkoxy, haloalkoxy, alkylthio, alkylsulfinyl, alkylsulfonyl, optionally substituted aryl, optionally substituted heteroaryl, optionally substituted nonaromatic heterocycle,
   a group of the formula: -X'-R^{5'} (wherein X' is -C=C-, and R^{5'} is optionally substituted aryl, optionally substituted heteroaryl, optionally substituted nonaromatic heterocycle, optionally substituted alkyl, alkoxy, hydroxy or hydrogen) or
   a group of the formula: -X"-R^{5"} (wherein X" is -O-Z-, -S-Z-, -C(=O)-, -SO-Z-, -SO₂-Z-, -NRSO₂-, -NRC(=O)-, -SO₂NR-, -C(=O)NR-, -CR(OH)-, -SO₂O- or -NR-, R⁵ is optionally substituted aryl, optionally substituted heteroaryl or optionally substituted nonaromatic heterocycle, R is hydrogen or alkyl, and Z is a bond or alkylene),
   R⁶ is hydrogen, alkyl or halogen,
   R⁷ is hydrogen, optionally substituted alkoxy, halogen, cyano, nitro, alkyl, haloalkyl, optionally substituted alkenyl or optionally substituted alkynyl,
   R⁸ is hydrogen, nitro, optionally substituted amino, halogen, optionally substituted alkyl, alkoxy, optionally substituted alkenyl, optionally substituted alkynyl, cyano or haloalkoxy, and
   R⁹ is hydrogen, alkyl, halogen or optionally substituted aryl, provided that, one of R⁷ and R⁸ is not hydrogen).
(12) The compound of (11) wherein R⁸ is haloalkyl, a pharmaceutically acceptable salt or solvate thereof.
(13) A compound of the formula II: 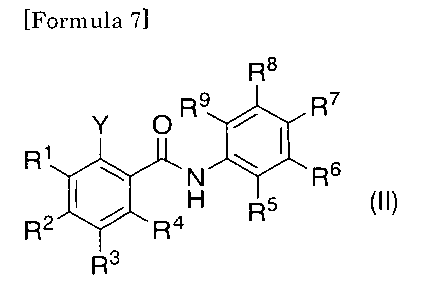 a pharmaceutically acceptable salt or solvate thereof, (wherein Y is hydroxy or a group of the formula: -NH-SO₂-Y' (wherein Y' is optionally substituted aryl or optionally substituted alkyl),
   R¹ is hydrogen, optionally substituted alkyl, optionally substituted amino, nitro, optionally substituted alkoxy, halogen, optionally substituted alkenyl or optionally substituted alkynyl,
   R² is hydrogen, halogen, nitro, optionally substituted amino, cyano, optionally substituted alkyl, optionally substituted carbamoyl, hydroxy, optionally substituted alkenyl, optionally substituted alkynyl, optionally substituted aryl or a group of the formula: -O-R^{2'} (wherein R^{2'} is optionally substituted alkyl, alkylsulfonyl, cycloalkyl, optionally substituted nonaromatic heterocycle or heteroaryl), or
   R¹ and R² can be taken together with the neighboring carbon atom to form an optionally substituted 5 or 6-membered ring optionally containing heteroatom(s),
   R³ is hydrogen, halogen, cyano, optionally substituted sulfamoyl, optionally substituted carbamoyl, optionally substituted amino, nitro, optionally substituted alkyl, optionally substituted alkoxy, optionally substituted alkenyl, optionally substituted aryl, optionally substituted heteroaryl, optionally substituted nonaromatic heterocycle or
   a group of the formula: -C≡C-R^{3'} (wherein R^{3'} is hydrogen, optionally substituted aryl, optionally substituted heteroaryl, hydroxy or optionally substituted alkyl),
   R⁴ is hydrogen, halogen, optionally substituted alkyl, optionally substituted alkoxy, optionally substituted alkenyl, optionally substituted alkynyl or optionally substituted alkylthio,
   R⁵ is substituted aryl,
   R⁶ is hydrogen, alkyl or halogen,
   R⁷ is hydrogen, optionally substituted alkoxy, halogen, cyano, nitro, optionally substituted alkyl, optionally substituted alkenyl or optionally substituted alkynyl,
   R⁸ is hydrogen, nitro, optionally substituted amino, halogen, optionally substituted alkyl, alkoxy, optionally substituted alkenyl, optionally substituted alkynyl, cyano or haloalkoxy, and
   R⁹ is hydrogen, alkyl, halogen or optionally substituted aryl).
(14) The compound of (13) wherein either R⁷ or R⁸ is haloalkyl or haloalkoxy, a pharmaceutically acceptable salt or solvate thereof.
(15) The compound of (14) wherein R² is halogen, a pharmaceutically acceptable salt or solvate thereof.
(16) The compound of (14) wherein R¹ is optionally substituted alkyl, a pharmaceutically acceptable salt or solvate thereof.
(17) The compound of (14) wherein R³ is halogen or substituted aryl, a pharmaceutically acceptable salt or solvate thereof.
(18) A compound of the formula II: 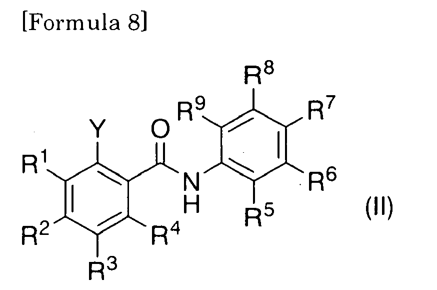 a pharmaceutically acceptable salt or solvate thereof, (wherein Y is hydroxy or a group of the formula: -NH-SO₂-Y' (wherein Y' is optionally substituted aryl or optionally substituted alkyl),
   R¹ is hydrogen, optionally substituted alkyl, optionally substituted amino, nitro, optionally substituted alkoxy, halogen, optionally substituted alkenyl or optionally substituted alkynyl,
   R² is halogen, nitro, optionally substituted amino, cyano, optionally substituted alkyl, optionally substituted carbamoyl, hydroxy, optionally substituted alkenyl, optionally substituted alkynyl, optionally substituted aryl or a group of the formula: -O-R^{2'} (wherein R^{2'} is optionally substituted alkyl, alkylsulfonyl, cycloalkyl, optionally substituted nonaromatic heterocycle or heteroaryl), or
   R¹ and R² can be taken together with the neighboring carbon atom to form an optionally substituted 5 or 6-membered ring optionally containing heteroatom(s),
   R³ is hydrogen, halogen, cyano, optionally substituted sulfamoyl, optionally substituted carbamoyl, optionally substituted amino, nitro, optionally substituted alkyl, optionally substituted alkoxy, optionally substituted alkenyl, optionally substituted aryl, optionally substituted heteroaryl, optionally substituted nonaromatic heterocycle or
   a group of the formula: -C≡C-R^{3'} (wherein R³' is hydrogen, optionally substituted aryl, optionally substituted heteroaryl, hydroxy or optionally substituted alkyl),
   R⁴ is hydrogen, halogen, optionally substituted alkyl, optionally substituted alkoxy, optionally substituted alkenyl, optionally substituted alkynyl or optionally substituted alkylthio,
   R⁵ is optionally substituted nonaromatic heterocycle,
   R⁶ is hydrogen, alkyl or halogen,
   R⁷ is hydrogen, optionally substituted alkoxy, halogen, cyano, nitro, optionally substituted alkyl, optionally substituted alkenyl or optionally substituted alkynyl,
   R⁸ is halogen or haloalkyl, and
   R⁹ is hydrogen, alkyl, halogen or optionally substituted aryl).
(19) The compound of (18) wherein R² is halogen, a pharmaceutically acceptable salt or solvate thereof.
(20) A compound of the formula II: 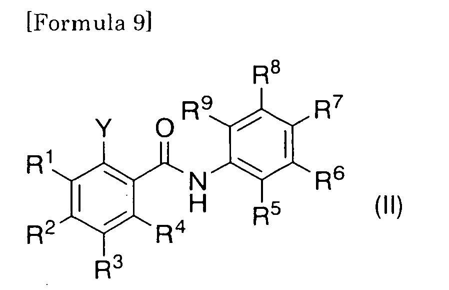 a pharmaceutically acceptable salt or solvate thereof,
   (wherein Y is hydroxy or a group of the formula: -NH-SO₂-Y' (wherein Y' is optionally substituted aryl or optionally substituted alkyl),
   R¹ is optionally substituted alkyl, optionally substituted amino, nitro, optionally substituted alkoxy, halogen, optionally substituted alkenyl or optionally substituted alkynyl,
   R² is hydrogen, halogen, nitro, optionally substituted amino, cyano, optionally substituted alkyl, optionally substituted carbamoyl, optionally substituted alkoxy, hydroxy, optionally substituted alkenyl, optionally substituted alkynyl or optionally substituted aryl, or
   R¹ and R² can be taken together with the neighboring carbon atom to form an optionally substituted 5 or 6-membered ring optionally containing heteroatom(s),
   R³ is hydrogen, halogen, cyano, optionally substituted sulfamoyl, optionally substituted carbamoyl, optionally substituted amino, nitro, optionally substituted alkyl, optionally substituted alkoxy, optionally substituted alkenyl, optionally substituted aryl, optionally substituted heteroaryl, optionally substituted nonaromatic heterocycle or
   a group of the formula: -C≡C-R^{3'} (wherein R^{3'} is hydrogen, optionally substituted aryl, optionally substituted heteroaryl, hydroxy or optionally substituted alkyl),
   R⁴ is hydrogen, halogen, optionally substituted alkyl, optionally substituted alkoxy, optionally substituted alkenyl, optionally substituted alkynyl or optionally substituted alkylthio,
   R⁵ is optionally substituted nonaromatic heterocycle,
   R⁶ is hydrogen, alkyl or halogen,
   R⁷ is hydrogen, optionally substituted alkoxy, halogen, cyano, nitro, optionally substituted alkyl, optionally substituted alkenyl or optionally substituted alkynyl,
   R⁸ is halogen or haloalkyl, and
   R⁹ is hydrogen, alkyl, halogen or optionally substituted aryl).
(21) The compound of (20) wherein R¹ is alkyl, a pharmaceutically acceptable salt or solvate thereof.
(22) The compound of (21) wherein R³ is halogen, a pharmaceutically acceptable salt or solvate thereof.
(23) A compound of the formula II: 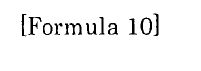 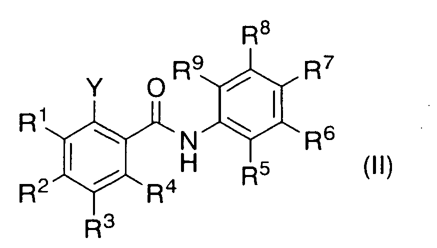 a pharmaceutically acceptable salt or solvate thereof, (wherein Y is hydroxy or a group of the formula: -NH-SO₂-Y' (wherein Y' is optionally substituted aryl or optionally substituted alkyl),
   R¹ is hydrogen, optionally substituted alkyl, optionally substituted amino, nitro, optionally substituted alkoxy, halogen, optionally substituted alkenyl or optionally substituted alkynyl,
   R² is halogen,
   R³ is hydrogen, halogen, cyano, optionally substituted sulfamoyl, optionally substituted carbamoyl, optionally substituted amino, nitro, optionally substituted alkyl, optionally substituted alkoxy, optionally substituted alkenyl, optionally substituted aryl, optionally substituted heteroaryl, optionally substituted nonaromatic heterocycle or
   a group of the formula: -C≡C-R^{3'} (wherein R^{3'} is hydrogen, optionally substituted aryl, optionally substituted heteroaryl, hydroxy or optionally substituted alkyl),
   R⁴ is hydrogen, halogen, optionally substituted alkyl, optionally substituted alkoxy, optionally substituted alkenyl, optionally substituted alkynyl or optionally substituted alkylthio,
   a group of the formula: -X"-R^{5"} (wherein X" is -C(=O)-, -NHSO₂-, -NHC(=O)-, -CH(OH)- or -NR-, R^{5"} is substituted aryl, and R is hydrogen or alkyl),
   R⁶ is hydrogen, alkyl or halogen,
   R⁷ is haloalkyl or haloalkoxy,
   R⁸ is hydrogen, nitro, optionally substituted amino, halogen, optionally substituted alkyl, alkoxy, optionally substituted alkenyl, optionally substituted alkynyl, cyano or haloalkoxy, and
   R⁹ is hydrogen, alkyl, halogen or optionally substituted aryl).
(24) A compound of the formula II: 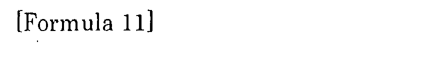 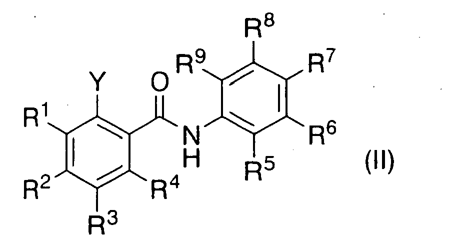 a pharmaceutically acceptable salt or solvate thereof,
   (wherein Y is hydroxy,
   R¹ is hydrogen,
   R² is a group of the formula: -O-R^{2'} (wherein R^{2'} is optionally substituted nonaromatic heterocycle),
   R³ is hydrogen,
   R⁴ is hydrogen,
   R⁵ is halogen, aryl or optionally substituted heteroaryl,
   R⁶ is hydrogen,
   R⁷ is hydrogen,
   R⁸ is haloalkyl, and
   R⁹ is hydrogen).
(25) A compound of the formula II: 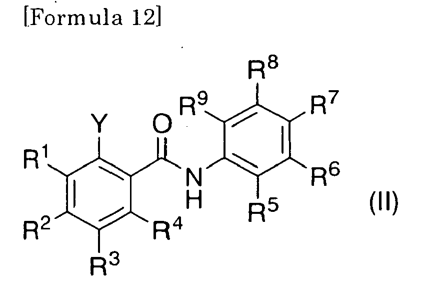 a pharmaceutically acceptable salt or solvate thereof,
   (wherein Y is a group of the formula: -NH-SO₂-Y' (wherein Y' is optionally substituted aryl),
   R¹ is hydrogen,
   R² is hydrogen, halogen, nitro, cyano, optionally substituted carbamoyl or a group of the formula: -O-R^{2'} (wherein R^{2'} is optionally substituted alkyl),
   R³ is hydrogen, halogen, nitro, cyano, optionally substituted aryl or nonaromatic heterocycle,
   R⁴ is hydrogen,
   R⁵ is hydrogen, halogen, optionally substituted alkyl, alkoxy, optionally substituted amino or optionally substituted nonaromatic heterocycle,
   R⁶ is hydrogen, optionally substituted alkyl or halogen,
   R⁷ is hydrogen, halogen or optionally substituted nonaromatic heterocycle,
   R⁸ is hydrogen, halogen, haloalkyl or haloalkoxy, and
   R⁹ is hydrogen).
(26) The compound of any one of (13) to (17) wherein R⁵ is 2,4-dihalogenophenyl, a pharmaceutically acceptable salt or solvate thereof.
(27) The compound of any one of (13) to (19), (24) and (26) wherein R² is a group of the formula: 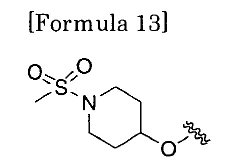 a pharmaceutically acceptable salt or solvate thereof.
(28) A pharmaceutical composition comprising the compound of any one of claims (6) to (27), a pharmaceutically acceptable salt or solvate thereof.
(29) The CTGF inhibitor of (1), wherein R² is a group of the formula:

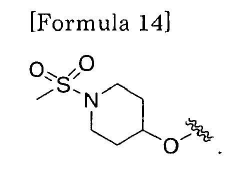

### Effect of the Invention

Compounds of the present invention have inhibitory activity on CTGF expression. Therefore, a pharmaceutical composition comprising the compound of the present invention is useful for therapy of a disease caused by overexpression of CTGF.

### Best Mode for Carrying Out the Invention

A compound of this invention is a compound of the formula I: 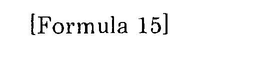 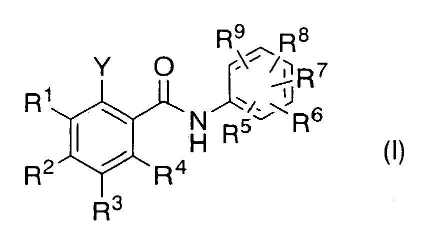 (wherein Y is hydroxy or a group of the formula: -NH-SO₂-Y' (wherein Y' is optionally substituted aryl or optionally substituted alkyl),
R¹ is hydrogen, optionally substituted alkyl, optionally substituted amino, nitro, optionally substituted alkoxy, halogen, optionally substituted alkenyl or optionally substituted alkynyl,
R² is hydrogen, halogen, nitro, optionally substituted amino, cyano, optionally substituted alkyl, optionally substituted carbamoyl, hydroxy, optionally substituted alkenyl, optionally substituted alkynyl, optionally substituted aryl or
a group of the formula: -O-R^{2'} (wherein R^{2'} is optionally substituted alkyl, alkylsulfonyl, cycloalkyl, optionally substituted nonaromatic heterocycle or heteroaryl), or
R¹ and R² can be taken together with the neighboring carbon atom to form an optionally substituted 5 or 6-membered ring optionally containing heteroatom(s),
R³ is hydrogen, halogen, cyano, optionally substituted sulfamoyl, optionally substituted carbamoyl, optionally substituted amino, nitro, optionally substituted alkyl, optionally substituted alkoxy, optionally substituted alkenyl, optionally substituted aryl, optionally substituted heteroaryl, optionally substituted nonaromatic heterocycle or
a group of the formula: -C≡C-R^{3'} (wherein R^{3'} is hydrogen, optionally substituted aryl, optionally substituted heteroaryl, hydroxy or optionally substituted alkyl),
R⁴ is hydrogen, halogen, optionally substituted alkyl, optionally substituted alkoxy, optionally substituted alkenyl, optionally substituted alkynyl or optionally substituted alkylthio,
R⁵ is hydrogen, carbamoyl, cyano, nitro, halogen, optionally substituted alkyl, alkenyl, alkoxycarbonylamino, alkoxy, haloalkoxy, alkylthio, alkylsulfinyl, alkylsulfonyl, optionally substituted amino, optionally substituted aryl, optionally substituted heteroaryl, optionally substituted nonaromatic heterocycle,
a group of the formula: -X'-R^{5'} (wherein X' is -C≡C-, and R^{5'} is optionally substituted aryl, optionally substituted heteroaryl, optionally substituted nonaromatic heterocycle, optionally substituted alkyl, alkoxy, hydroxy or hydrogen) or a group of the formula: -X''-R^{5''} (wherein X" is -O-Z-, -S-Z-, -C(=O)-, -SO-Z-, -SO₂-Z-, -NRSO₂-, -NRC(=O)-, -SO₂NR-, -C(=O)NR-, -CR(OH)-, -SO₂O- or -NR-, R^{5''} is optionally substituted aryl, optionally substituted heteroaryl or optionally substituted nonaromatic heterocycle, R is hydrogen or alkyl, and Z is a bond or alkylene), and
R⁶, R⁷, R⁸ and R⁹ are each independently hydrogen, optionally substituted alkyl, optionally substituted alkenyl, optionally substituted alkynyl, halogen, optionally substituted alkoxy, cyano, nitro, optionally substituted amino, optionally substituted aryl or nonaromatic heterocycle).

As to Y and R¹ to R⁹, the following substituents are preferable.
As to Y, hydroxy or a group of the formula: -NH-SO₂-Y' (wherein Y' is optionally substituted aryl or alkyl) is preferable.
As to R¹, hydrogen, optionally substituted alkyl, optionally substituted amino, nitro, alkoxy or halogen is preferable.
As to R², hydrogen, halogen, nitro, optionally substituted amino, cyano, alkyl, optionally substituted aryl or a group of the formula: -O-R^{2'} (wherein R^{2'} is optionally substituted alkyl, alkylsulfonyl, cycloalkyl, optionally substituted nonaromatic heterocycle or heteroaryl) is preferable.
As to R³, hydrogen, halogen, cyano, nitro, alkyl, alkoxy, optionally substituted aryl, heteroaryl or a group of the formula: -C≡C-R^{3'} (wherein R^{3'} is optionally substituted aryl or optionally substituted heteroaryl) is preferable.

As to R⁴, hydrogen, halogen, alkyl is preferable.
As to R⁵, hydrogen, halogen, optionally substituted alkyl, alkoxycarbonylamino, alkoxy, optionally substituted aryl, heteroaryl, optionally substituted nonaromatic heterocycle, a group of the formula: -X'-R^{5'} (wherein X' is -C≡C-, and R⁵' is optionally substituted aryl or optionally substituted alkyl) or a group of the formula: -X"-R^{5"} (wherein X" is -O-Z-, -C(=O)-, -NRSO₂-, -NRC(=O)-, -SO₂NR-, -CR(OH)-, -SO₂O- or -NR-, R^{5"} is optionally substituted aryl or heteroaryl, R is hydrogen or alkyl, and Z is a bond).
As to R⁶, hydrogen, optionally substituted alkyl is preferable.
As to R⁷, hydrogen, optionally substituted alkyl, halogen, optionally substituted alkoxy is preferable.
As to R⁸, hydrogen, optionally substituted alkyl, halogen, alkoxy is preferable.
As to R⁹, hydrogen is preferable.

Among compounds of the formula (II), especially preferable embodiments are as below. 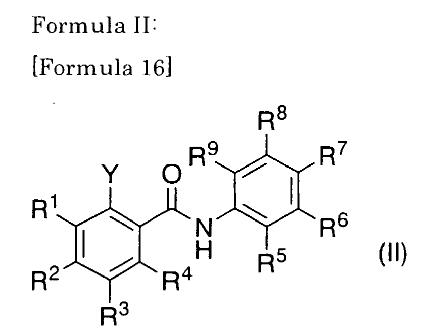
1) A compound wherein Y is hydroxy, R¹, R², R⁴, R⁵, R⁶, R⁷ and R⁹ is hydrogen, R³ is a group of the formula: -C≡C-R^{3'} (wherein R^{3'} is optionally substituted aryl), and R⁸ is haloalkyl. For example, it is a compound described in Table 1 to 3.
2) A compound wherein Y is hydroxy, R¹, R², R⁴, R⁵, R⁶ and R⁹ are hydrogen, R³ is a group of the formula: -C≡C-R^{3'} (wherein R^{3'} is optionally substituted aryl), R⁷ is halogen, and R⁸ is haloalkyl. For example, it is a compound described in Table 4.
3) A compound wherein Y is hydroxy, R¹, R², R⁴, R⁶, R⁷ and R⁹ are hydrogen, R³ is a group of the formula: -C≡C-R^{3'}(wherein R^{3'} is optionally substituted aryl), R⁵ is halogen, alkoxy or optionally substituted aryl, and R⁸ is haloalkyl. For example, it is a compound described in Table 4 or 5.
4) A compound wherein Y is hydroxy, R¹, R³, R⁴, R⁶, R⁸ and R⁹ are hydrogen, R² is halogen, R⁵ is a group of the formula: -X'-R^{5'} (wherein X' is -C≡C-, and R^{5'} is optionally substituted aryl or optionally substituted alkyl), and R⁷ is haloalkyl. For example, it is a compound described in Table 6.
5) A compound wherein Y is hydroxy, R¹ is alkyl, R², R⁴, R⁶, R⁸ and R⁹ are hydrogen, R³ is halogen, R⁵ is a group of the formula: -X'-R^{5'} (wherein X' is -C≡C-, and R⁵ is optionally substituted alkyl), and R⁷ is haloalkyl. For example, it is a compound described in Table 6.
6) A compound wherein Y is hydroxy, R¹, R⁴, R⁶, R⁷ and R⁹ are hydrogen, R² is hydrogen or optionally substituted aryl, R³ is optionally substituted aryl or heteroaryl, R⁵ is hydrogen or halogen, and R⁸ is haloalkyl. For example, it is a compound described in Table 7 or 8.
7) A compound wherein Y is hydroxy, R¹, R², R⁴, R⁵, R⁶ and R⁹ are hydrogen, R³ is optionally substituted aryl or heteroaryl, R⁷ is halogen, and R⁸ is haloalkyl.
8) A compound wherein Y is hydroxy, R¹, R³, R⁴, R⁶, R⁷ and R⁹ are hydrogen, R² is halogen, R⁵ is optionally substituted aryl, and R⁸ is haloalkyl. For example, it is a compound described in Table 9 or 10.
9) A compound wherein Y is hydroxy, R¹, R³, R⁴, R⁶, R⁸ and R⁹ are hydrogen, R² is halogen, R⁵ is optionally substituted aryl or heteroaryl, and R⁷ is haloalkoxy. For example, it is a compound described in Table 11 to 13.
10) A compound wherein Y is hydroxy, R¹, R³, R⁴, R⁶, R⁸ and R⁹ are hydrogen, R² is halogen, R⁵ is optionally substituted aryl or heteroaryl, and R⁷ is haloalkoxy or haloalkyl. For example, it is a compound described in Table 14 or 15.
11) A compound wherein Y is hydroxy, R¹, R², R⁴, R⁶, R⁷ and R⁹ are hydrogen, R³ is halogen or optionally substituted aryl, R⁵ is optionally substituted aryl, and R⁸ is haloalkyl. For example, it is a compound described in Table 16.
12) A compound wherein Y is hydroxy, R¹ is alkyl, R², R⁶, R⁷ and R⁹ are hydrogen, R³ is halogen, R⁴ is hydrogen or alkyl, R⁵ is optionally substituted aryl, and R⁸ is haloalkyl. For example, it is a compound described in Table 17.
13) A compound wherein Y is hydroxy, R¹ is alkyl, R², R⁴, R⁶, R⁸ and R⁹ are hydrogen, R³ is halogen, R⁵ is optionally substituted aryl, and R⁷ is haloalkoxy. For example, it is a compound described in Table 18.
14) A compound wherein Y is hydroxy, R¹, R², R⁴, R⁶, R⁸ and R⁹ are hydrogen, R³ is halogen or optionally substituted aryl, R⁵ is optionally substituted aryl, and R⁷ is haloalkoxy. For example, it is a compound described in Table 19 or 20.
15) A compound wherein Y is hydroxy, R¹ is hydrogen, alkyl, alkoxy, nitro or halogen, R², R⁴, R⁶, R⁷ and R⁹ are hydrogen, R³ is halogen, R⁵ is optionally substituted aryl, and R⁸ is haloalkyl. For example, it is a compound described in Table 21 to 24.
16) A compound wherein Y is hydroxy, R¹ is alkyl, R², R⁴, R⁶, R⁸ and R⁹ are hydrogen, R³ is halogen, R⁵ is optionally substituted aryl, and R⁷ is haloalkoxy. For example, it is a compound described in Table 25.
17) A compound wherein Y is hydroxy, R¹ is hydrogen, halogen, optionally substituted alkyl or optionally substituted amino, R² is hydrogen, nitro, alkoxy, alkyl, cyano or optionally substituted amino, R³ is hydrogen, halogen, nitro, alkyl, alkoxy or cyano, R⁴ is hydrogen or halogen, R⁵ is optionally substituted aryl, R⁶, R⁷, and R⁹ are hydrogen, and R⁸ is haloalkyl. For example, it is a compound described in Table 26 to 28.
18) A compound wherein Y is hydroxy, R¹, R³, R⁴, R⁶, R⁷ and R⁹ are hydrogen, R² is halogen, R⁵ is optionally substituted nonaromatic heterocycle, and R⁸ is haloalkyl. For example, it is a compound described in Table 29 or 30.
19) A compound wherein Y is hydroxy, R¹, R², R⁴, R⁶, R⁷ and R⁹ are hydrogen, R³ is halogen, R⁵ is optionally substituted nonaromatic heterocycle, and R⁸ is haloalkyl. For example, it is a compound described in Table 31.
20) A compound wherein Y is hydroxy, R¹ is alkyl, R², R⁴, R⁶, R⁷ and R⁹ are hydrogen, R³ is halogen, R⁵ is optionally substituted nonaromatic heterocycle, and R⁸ is haloalkyl. For example, it is a compound described in Table 32.
21) A compound wherein Y is hydroxy, R¹, R², R⁴, R⁶, R⁷ and R⁹ are hydrogen, R³ is halogen, R⁵ is optionally substituted nonaromatic heterocycle, and R⁸ is halogen. For example, it is a compound described in Table 33.
22) A compound wherein Y is hydroxy, R¹, R³, R⁴, R⁶, R⁷ and R⁹ are hydrogen, R² is halogen, R⁵ is optionally substituted aryl, and R⁸ halogen, alkyl or alkoxy. For example, it is a compound described in Table 34.
22) A compound wherein Y is hydroxy, R¹, R³, R⁴, R⁶, R⁸ and R⁹ are hydrogen, R² is halogen, R⁵ is optionally substituted aryl, and R⁷ is halogen. For example, it is a compound described in Table 34.
23) A compound wherein Y is hydroxy, R¹ is hydrogen or alkyl, R², R⁴, R⁶, R⁷ and R⁹ are hydrogen, R³ is halogen, R⁵ is optionally substituted aryl, and R⁸ is nitro or halogen. For example, it is a compound described in Table 35 or 36.
24) A compound wherein Y is hydroxy, R¹ is alkyl, R², R⁴, R⁶, R⁸ and R⁹ are hydrogen, R³ is halogen, R⁵ is optionally substituted aryl, and R⁷ is halogen or haloalkyl. For example, it is a compound described in Table 37.
25) A compound wherein Y is hydroxy, R¹, R⁴, R⁶, R⁷ and R⁹ are hydrogen, R² is hydrogen or halogen, R³ is hydrogen or halogen, R⁵ is alkoxy or a group of the formula: -X"-R^{5"} (wherein X" is -O-Z-, R^{5"} is optionally substituted aryl, and Z is a bond), and R⁸ is haloalkyl. For example, it is a compound described in Table 38.
26) A compound wherein Y is hydroxy, R¹, R³, R⁴, R⁶, R⁷ and R⁹ are hydrogen, R² is halogen, R⁵ is a group of the formula: -X"-R^{5"} (wherein X" is -S-Z-, -SO-Z-, -SO₂-Z- or -NR-, R^{5"} is optionally substituted aryl, R is alkyl, and Z is a bond or alkylene), and R⁸ is haloalkyl. For example, it is a compound described in Table 39.
27) A compound wherein Y is hydroxy, R¹, R³, R⁴, R⁶, R⁸ and R⁹ are hydrogen, R² is halogen, R⁵ is alkoxycarbonylamino or a group of the formula: -X"-R^{5"} (wherein X" is -C(=O)-, -CR(OH)- or -NRC(=O)-, R^{5"} is optionally substituted aryl or heteroaryl, and R is hydrogen), and R⁷ is haloalkyl or haloalkoxy. For example, it is a compound described in Table 40 to 43.
28) A compound wherein Y is hydroxy, R¹, R³, R⁴, R⁶, R⁷, and R⁹ are hydrogen, R² is halogen, R⁵ is a group of the formula: -X"-R^{5"}(wherein X" is -NRC(=O)-, R^{5"} is optionally substituted aryl, and R is hydrogen), and R⁸ is haloalkyl. For example, it is a compound described in Table 43.
29) A compound wherein Y is hydroxy, R¹, R³, R⁴, R⁶. R⁷ and R⁹ are hydrogen, R² is halogen, R⁵ is a group of the formula: -X^{"}-R^{5"} (wherein X" is -NRC(=O)- or -NRSO₂-, R^{5"} is optionally substituted aryl, and R is hydrogen), and R⁸ is haloalkyl. For example, it is a compound described in Table 44.
30) A compound wherein Y is hydroxy, R¹, R³, R⁴, R⁶, R⁸ and R⁹ are hydrogen, R² is halogen, R⁵ is a group of the formula: -X"-R^{5"} (wherein X" is -NRC(=O)- or -NRSO₂-, R^{5"} is optionally substituted aryl, and R is hydrogen), and R⁷ is haloalkyl. For example, it is a compound described in Table 45.
31) A compound wherein Y is hydroxy, R¹, R³, R⁴, R⁶, R⁷ and R⁹ are hydrogen, R² is halogen, R⁵ is a group of the formula: -X"-R^{5"} (wherein X" is -SO₂NR-, R⁵ is optionally substituted aryl, and R is hydrogen), and R⁸ is haloalkyl. For example, it is a compound described in Table 45.
32) A compound wherein Y is hydroxy, R¹, R³, R⁴, R⁶, R⁷, R⁸ and R⁹ are hydrogen, R² is halogen, and R⁵ is a group of the formula: -X"-R^{5"} (wherein X" is -SO₂O-, and R⁵ is optionally substituted aryl). For example, it is a compound described in Table 45.
33) A compound wherein Y is a group of the formula: -NH-SO₂-Y (wherein Y' is aryl or alkyl), R¹, R³, R⁴, R⁶, R⁷ and R⁹ are hydrogen, R² is halogen, R⁵ is optionally substituted aryl or nonaromatic heterocycle, and R⁸ is haloalkyl. For example, it is a compound described in Table 46.
34) A compound wherein Y is a group of the formula: -NH-SO₂-Y' (wherein Y' is aryl or alkyl), R¹, R², R⁴, R⁶, R⁷ and R⁹ are hydrogen, R³ is halogen, R⁵ is hydrogen or optionally substituted aryl, R⁸ is haloalkyl. For example, it is a compound described in Table 47.
35) A compound wherein Y is a group of the formula: -NH-SO₂-Y' (wherein Y' is aryl), R¹, R², R⁴, R⁵, R⁶, R⁷ and R⁹ are hydrogen, R³ is halogen, R⁶ is halogen, and R⁸ is halogen. For example, it is a compound described in Table 47.
36) A compound wherein Y is a group of the formula: -NH-SO₂-Y' (wherein Y' is optionally substituted aryl), R¹, R², R⁴, R⁵, R⁶, R⁷ and R⁹ are hydrogen, R³ is halogen, and R⁸ is haloalkyl. For example, it is a compound described in Table 48.
37) A compound wherein Y is hydroxy, R¹ and R² are taken together with the neighboring carbon atom to form an optionally substituted 5 or 6-membered ring comprising heteroatom(s), R³ is hydrogen or halogen, R⁴, R⁶, R⁷ and R⁹ are hydrogen, R⁵ is optionally substituted aryl, and R⁸ is haloalkyl. For example, it is a compound described in Table 49.
38) A compound wherein Y is hydroxy, R¹ and R² are taken together with the neighboring carbon atom to form an optionally substituted 5 or 6-membered ring comprising heteroatom(s), R³ is optionally substituted aryl, R⁴, R⁵, R⁷, R⁸ and R⁹ are hydrogen, and R⁶ is haloalkyl. For example, it is a compound described in Table 49.
39) A compound wherein Y is hydroxy, R¹, R³, R⁴, R⁶, R⁷ and R⁹ are hydrogen, R² is a group of the formula: -O-R^{2'} (wherein R²' is optionally substituted alkyl, alkylsulfonyl, cycloalkyloxy, optionally substituted nonaromatic heterocycle or heteroaryl), R⁵ is optionally substituted aryl or optionally substituted nonaromatic heterocycle, and R⁸ is haloalkyl. For example, it is a compound described in Table 50 to 55.
40) A compound wherein Y is hydroxy, R² is a group of the formula: -O-R^{2'} (wherein R²' is optionally substituted alkyl or optionally substituted nonaromatic heterocycle), R¹ is halogen, R³ is hydrogen or halogen, R⁴, R⁶, R⁷ and R⁹ are hydrogen, R⁵ is optionally substituted aryl or optionally substituted nonaromatic heterocycle, and R⁸ is haloalkyl. For example, it is a compound described in Table 56.
41) A compound wherein Y is a group of the formula: -NH-SO₂-Y' (wherein Y' is optionally substituted aryl), R¹, R³, R⁴, R⁶, R⁷ and R⁹ are hydrogen, R² is hydrogen, nitro, halogen, cyano, optionally substituted carbamoyl or alkoxy, R⁵ is hydrogen, halogen, haloalkyl, alkoxy or optionally substituted nonaromatic heterocycle, and R⁸ is halogen, haloalkyl or haloalkoxy. For example, it is a compound described in Table 57 or 58.
42) A compound wherein Y is a group of the formula: -NH-SO₂-Y' (wherein Y' is aryl), R¹, R³, R⁴ and R⁹ are hydrogen, R² is hydrogen, nitro or halogen, R⁵, R⁶, R⁷ and R⁸ are hydrogen, halogen or haloalkyl. For example, it is a compound described in Table 59.
43) A compound wherein Y is a group of the formula: -NH-SO₂-Y' (wherein Y' is optionally substituted aryl), R¹, R², R⁴, R⁶, R⁷ and R⁹ are hydrogen, R³ is halogen, cyano, nitro, optionally substituted aryl or nonaromatic heterocycle, R⁵ is hydrogen, optionally substituted amino, alkoxy, haloalkyl or nonaromatic heterocycle, and R⁸ is halogen or haloalkyl. For example, it is a compound described in Table 60.
44) A compound wherein Y is a group of the formula: -NH-SO₂-Y' (wherein Y' is optionally substituted aryl), R¹, R⁴, R⁵ and R⁹ are hydrogen, R² is hydrogen, halogen or a group of the formula: -O-R^{2'} (wherein R² is optionally substituted nonaromatic heterocycle), R³ is hydrogen or halogen, R⁶ is hydrogen or haloalkyl, R⁷ is hydrogen, halogen, nonaromatic heterocycle, and R⁸ is hydrogen or halogen. For example, it is a compound described in Table 61.
45) A compound wherein Y is hydroxy, R¹ is halogen, R² is a group of the formula: -O-R²' (wherein R^{2'} is optionally substituted nonaromatic heterocycle), R³, R⁴, R⁶, R⁷ and R⁹are hydrogen, R⁵ is optionally substituted aryl or optionally substituted nonaromatic heterocycle, and R⁸ is haloalkyl. For example, it is a compound described in Table 61.

Terms used in this description are explained below.

"Aryl" means C6 to C14 monocyclic or condensed aromatic carbocycle. For example, it is phenyl, naphthyl, phenanthryl or the like. Especially, phenyl is preferable.
"Alkyl" means C1 to C8 straight or branched alkyl group. For example, it is methyl, ethyl, n-propyl, isopropyl, n-butyl, isobutyl, sec-buthyl, tert-butyl, n-pentyl, isopentyl, neopentyl, tert-pentyl, n-hexyl, isohexyl or the like. Preferred is C1 to C4 straight or branched alkyl group, and it is methyl, ethyl, n-propyl, isopropyl, n-butyl, isobutyl, sec-buthyl, tert-butyl or the like.
The alkyl part in "alkoxy" means the same group as the above "alkyl". Preferred is C1 to C4 straight or branched alkyloxy group, and it is methoxy, ethoxy, n-propoxy, isopropoxy, n-butoxy, isobutoxy, sec-butoxy or tert-butoxy.
"Halogen" means fluorine, chlorine, bromine or iodine.
"Alkenyl" means C2 to C8 straight or branched alkenyl group which is the above "alkyl" with one or more double bond(s). For example, it is vinyl, 1-propenyl, 2-propenyl, 1-butenyl, 2-butenyl, 3-butenyl or 1,3-butadienyl. Preferred is C2 to C4 straight alkenyl group, and it is vinyl, 1-propenyl, 2-propenyl or the like.
"Alkynyl" means C2 to C8 straight or branched alkenyl group which is the above "alkyl" with one or more triple bond(s). For example, it is ethynyl, 1-propynyl, 2-propynyl, 1-butynyl, 2-butynyl, 3-butynyl or 1-hexenyl. Preferred is C2 to C4 straight alkynyl group, and it is ethynyl, 1-propynyl, 2-propynyl, 1-butynyl, 2-butynyl, 3-butynyl or the like. The above "alkynyl" can include one or more double bond(s) at any position.
"5 or 6-membered ring comprising heteroatom(s) formed with the neighboring carbon atom" means 5 or 6-membered ring fusing with benzene ring substituted at R¹ and R² and optionally containing heteroatom(s). A heteroatom means a nitrogen, sulfur or oxygen atom. Examples of 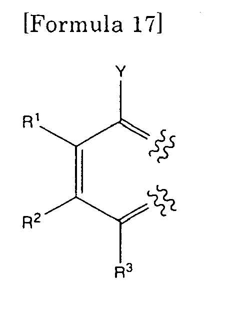 are the followings.

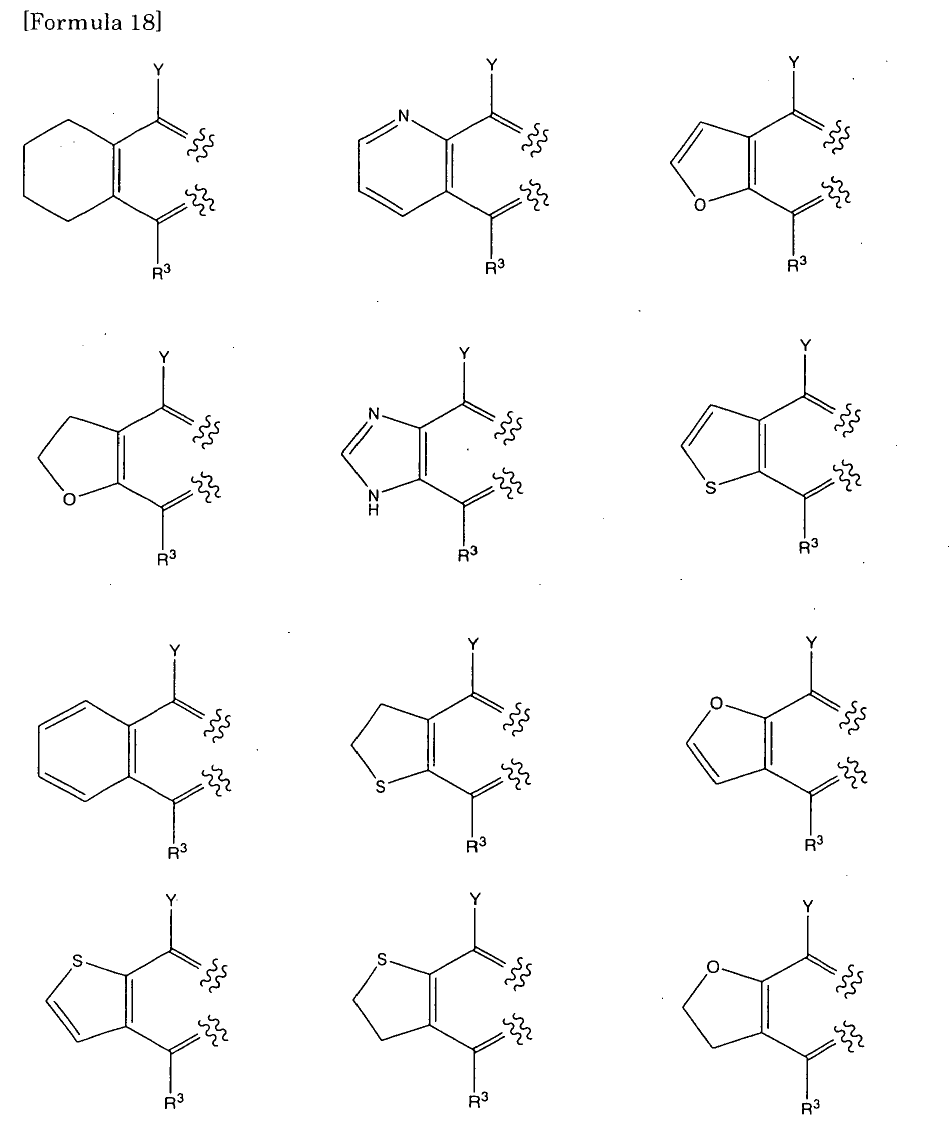

"Heteroaryl" means a 5 to 8-membered aromatic heterocycle containing 1 to 4 oxygen, sulfur and /or nitrogen atom(s) in the ring, or an aromatic heterocycle which is a 5 to 8-membered aromatic heterocycle fused with 1 to 4 of 5 to 8-membered aromatic carboncycle(s) or the other 5 to 8-membered aromatic heterocycle(s). The bonds can be at any substitutable position. The bonds can be at carbon or nitrogen atom in the ring.
For example, it is furyl (e.g., furan-2-yl or furan-3-yl), thienyl (e.g., thiophene-2-yl or thiophene-3-yl), pyrrolyl (e.g., pyrrole-1-yl, pyrrole-2-yl or pyrrole-3-yl), imidazolyl (e.g., imidazole-1-yl, imidazole-2-yl or imidazole-4-yl), pyrazolyl (e.g., pyrazole-1-yl, pyrazole-3-yl or pyrazole-4-yl), triazolyl (e.g., 1H-[1,2,4]triazole-1-yl, 4H-[1,2,4]triazole-4-yl or 1H-[1,2,4]triazole-3-yl), tetrazolyl (e.g., 1H-tetrazole-1-yl, 2H-tetrazole-2-yl, 1H-tetrazole-5-yl or 2H-tetrazole-5-yl), oxazolyl (e.g., oxazole-2-yl, oxazole-4-yl or oxazole-5-yl), isoxazolyl (e.g., isoxazole-3-yl, isoxazole-4-yl or isoxazole-5-yl), thiazolyl (e.g., thiazole-2-yl, thiazole-4-yl or thiazole-5-yl), isothiazolyl (e.g., isothiazole-3-yl, isothiazole-4-yl or isothiazole-5-yl), pyridyl (e.g., pyridine-2-yl, pyridine-3-yl or pyridine-4-yl), pyridazinyl (e.g., pyridazine-3-yl or pyridazine-4-yl), pyrimidinyl (e.g., pyrimidine-2-yl, pyrimidine-4-yl or pyrimidine-5-yl), furazanyl (e.g., furazan-3-yl), pyrazinyl (e.g., pyrazine-2-yl), thiadiazolyl (e.g., [1,3,4] thiadiazole-2-yl), oxadiazolyl (e.g., [1,3,4]-oxadiazole-2-yl), benzofuryl (e.g., benzo[b]furan-2-yl, benzo[b]furan-3-yl, benzo[b]furan-4-yl, benzo[b]furan-5-yl, benzo[b]furan-6-yl or benzo[b]furan-7-yl), benzothienyl (e.g., benzo[b]thiophene-2-yl, benzo[b]thiophene-3-yl, benzo[b]thiophene-4-yl, benzo[b]thiophene-5-yl, benzo[b]thiophene-6-yl or benzo[b]thiophene-7-yl), benzimidazolyl (e.g., benzimidazole-1-yl, benzimidazole-2-yl, benzimidazole-4-yl or benzimidazole-5-yl), benzothiazolyl (e.g., benzothiazole-2-yl, benzothiazole-3-yl, benzothiazole-4-yl, benzothiazole-5-yl, benzothiazole-6-yl or benzothiazole-7-yl), indolyl (e.g., indole-1-yl, indole-2-yl, indole-4-yl, indole-5-yl, indole-6-yl or indole-7-yl), dibenzofuryl, quinolyl (e.g., quinoline-2-yl, quinoline-3-yl, quinoline-4-yl, quinoline-5-yl, quinoline-6-yl, quinoline-7-yl or quinoline-8-yl), isoquinolyl (e.g., isoquinoline-1-yl, isoquinoline-3-yl, isoquinoline-4-yl, isoquinoline-5-yl, isoquinoline-6-yl, isoquinoline-7-yl or isoquinoline-8-yl), cinnolyl (e.g., cinnoline-3-yl, cinnoline-4-yl, cinnoline-5-yl, cinnoline-6-yl, cinnoline-7-yl or cinnoline-8-yl), quinazolyl (e.g., quinazoline-2-yl, quinazoline-4-yl, quinazoline-5-yl, quinazoline-6-yl, quinazoline-7-yl or quinazoline-8-yl), quinoxalyl (e.g., quinoxaline-2-yl, quinoxaline-5-yl or quinoxaline-6-yl), phthalazinyl (e.g., phthalazine-1-yl, phthalazine-5-yl or phthalazine-6-yl), puryl (e.g., purine-2-yl, purine-6-yl, purine-7-yl, purine-8-yl or purine-9-yl), pteridinyl, carbazolyl, phenanthridinyl, acridinyl, phenazinyl, 1,10-phenanthrolinyl, isoindolyl, 1H-indazolyl or indolizinyl (e.g., indolizine-1-yl). Especially preferred is a 5 or 6-membered aromatic heterocycle containing 1 or 2 oxygen, sulfur and/or nitrogen atom(s) in the ring or an aromatic heterocycle which is an aromatic heterocycle fused with a benzene ring. Especially preferred is furyl (e.g., furan-2-yl or furan-3-yl), thienyl (e.g., thiophene-2-yl or thlophene-3-yl), pyrrolyl (e.g., pyrrole-1-yl, pyrrole-2-yl or pyrrole-3-yl), pyridyl (e.g., pyridine-2-yl, pyridine-3-yl or pyridine-4-yl), pyrimidinyl (e.g., pyrimidine-2-yl, pyrimidine-4-yl or pyrimidine-5-yl), benzofuryl (e.g., benzo[b]furan-2-yl, benzo[b]furan-3-yl, benzo[b]furan-4-yl, benzo[b]furan-5-yl, benzo[b]furan-6-yl or benzo[b]furan-7-yl) or benzothienyl (e.g., benzo[b]thiophene-2-yl, benzo[b]thiophene-3-yl, benzo[b]thiophene-4-yl, benzo[b]thiophene-5-yl, benzo[b]thiophene-6-yl or benzo[b]thiophene-7-yl).

"Nonaromatic heterocycle" means a 5 to 8-membered nonaromatic heterocycle containing 1 to 4 oxygen, sulfur and /or nitrogen atom(s) in the ring or an nonaromatic heterocycle which is a 5 to 8-membered nonaromatic heterocycle fused with 1 to 4 of 5 to 8-membered carboncycle(s) or the other 5 to 8-membered heterocycle(s). The bonds can be at any substitutable position. The bonds can be at carbon or nitrogen atom in the ring. "Nonaromatic heterocycle" can be saturated or unsaturated, if it is nonaromatic. For example, it is perhydroazepino, 2-perhydroazepinyl, 3-perhydroazepinyl, 4-perhydroazepinyl, perhydroazocino, 2-perhydroazocinyl, 3-perhydroazocinyl, 4-perhydroazocinyl, 5-perhydroazocinyl, 1,3-dioxolane-2-yl, 1,3-dioxolane-4-yl, perhydro-1,2-thiazine-2-yl, perhydro-1,4-thiazine-4-yl, 1-pyrrolinyl, 2-pyrrolinyl, 3-pyrrolinyl, pyrrolidino, 2-pyrrolidinyl, 3-pyrrolidinyl, 1-imidazolinyl, 2-imidazolinyl, 4-imidazolinyl, 1-imidazolidinyl, 2-imidazolidinyl, 4-imidazolidinyl, 1-pyrazolinyl, 3-pyrazolinyl, 4-pyrazolinyl, 1-pyrazolidinyl, 3-pyrazolidinyl, 4-pyrazolidinyl, piperidino, 2-piperidyl, 3-piperidyl, 4-piperidyl, piperazino, 2-piperazinyl, 2-morpholinyl, 3-morpholinyl, morpholino, tetrahydropyranyl, aziridinyl (e.g., aziridine-1-yl or aziridine-2-yl), piperidino, piperidyl (e.g., 2-piperidyl, 3-piperidyl or 4-piperidyl), morpholino, morpholinyl (e.g., 2-morpholinyl or 3-morpholinyl), pyrrolinyl (e.g., 1-pyrrolinyl, 2-pyrrolinyl, 3-pyrrolinyl, 4-pyrrolinyl or 5-pyrrolinyl), pyrrolidinyl (e.g., 1-pyrrolidinyl, 2-pyrrolidinyl or 3-pyrrolidinyl), imidazolinyl (e.g.,1-imidazolinyl, 2-imidazolinyl or 4-imidazolinyl), piperazino, piperazinyl (e.g., 2-piperazinyl), thiolanyl (e.g., thiolane-2-yl or thiolane-3-yl), tetrahydrofuranyl (e.g., tetrahydrofuran-2-yl or tetrahydrofuran-3-yl), dioxanyl (e.g., 1,4-dioxane-2-yl), oxathianyl (e.g., 1,4-oxathiane-2-yl or 1,4-oxathiane-3-yl) or tetrahydropyranyl (e.g., tetrahydropyran-2-yl, tetrahydropyran-3-yl or tetrahydropyran-4-yl). Especially preferred is perhydroazepino, perhydroazocino, 1,3-dioxolane-2-yl, perhydro-1,2-thiazine-2-yl, perhydro-1,4-thiazine-4-yl, pyrrolidino, piperidino, 2-piperidyl, 3-piperidyl, 4-piperidyl, piperazino, 2-piperazinyl, 2-morpholinyl, 3-morpholinyl or morpholino. Preferred is a 5 or 6-membered nitrogen-containing nonaromatic heterocycle. For example, it is piperidino, piperidyl (e.g., 2-piperidyl, 3-piperidyl or 4-piperidyl), morpholino, morpholinyl (e.g., 2-morpholinyl or 3-morpholinyl), piperidino, piperidyl (e.g., 2-piperidyl, 3-piperidyl or 4-piperidyl), morpholino, morpholinyl (e.g., 2-morpholinyl or 3-morpholinyl), pyrrolinyl (e.g., 1-pyrrolinyl, 2-pyrrolinyl, 3-pyrrolinyl, 4-pyrrolinyl or 5-pyrrolinyl), pyrrolidinyl (e.g., 1-pyrrolidinyl, 2-pyrrolidinyl or 3-pyrrolidinyl), imidazolinyl (e.g., 1-imidazolinyl, 2-imidazolinyl or 4-imidazolinyl), piperazino or piperazinyl (e.g., 2-piperazinyl). Nonaromatic heterocycle can also have bonds at carbon or nitrogen atom as well as the above heteroaryl.
The alkyl part of "alkylthio" means the same group as the above "alkyl". Preferred is C1 to C4 straight or branched alkylthio, and it is methylthio, ethylthio, n-propylthio, isopropylthio, n-butylthio, isobutylthio, sec-butylthio or tert-butylthio.
The alkyl part of "alkylsulfinyl" means the same group as the above "alkyl". Preferred is C1 to C4 straight or branched alkylsulfinyl, and it is methylsulfinyl, ethylsulfinyl, n-propylsulfinyl, isopropylsulfinyl, n-butylsulfinyl, isobutylsulfinyl, sec-butylsulfinyl or tert-butylsulfinyl.
The alkyl part of "alkylsulfonyl" means the same group as the above "alkyl". Preferred is C1 to C4 straight or branched alkylsulfonyl, and it is methylsulfonyl, ethylsulfonyl, n-propylsulfonyl, isopropylsulfonyl, n-butylsulfonyl, isobutylsulfonyl, sec-butylsulfonyl or tert-butylsulfonyl.
"Haloalkyl" means a group which is the above "alkyl" whose hydrogen atom(s) is(are) substituted with 1 to 6 halogen. For example, it is trifluoromethyl, difluoromethyl, 2,2,2-trifluoroethyl, 1,1-difluoroethyl, 3,3,3-trifluoro-n-propyl, trichloromethyl, dichloromethyl, 2,2,2-trichloroethyl, 1,1-dichloroethyl or 3,3,3-trichloro-n-propyl. Preferred is trifluoromethyl, trichloromethyl or 2,2,2-trichloroethyl.
"Haloalkoxy" means a group, which is the above "alkoxy" whose hydrogen atom(s) is(are) substituted with 1 to 6 halogen. For example, it is trifluoromethoxy, difluoromethoxy, 2,2,2-trifluoroethoxy, 1,1-difluoroethoxy, 3,3,3-trifluoro-n-propoxy, trichloromethoxy, dichloromethoxy, 2,2,2-trichloroethoxy, 1,1-dichloroethoxy or 3,3,3-trichloro-n-propoxy. Preferred is trifluoromethoxy, trichloromethoxy or 2,2,2-trichloroethoxy.
"Alkylene" means C1 to C8 straight or branched alkylene. For example, it is methylene, ethylene, trimethylene, tetramethylene, ethylethylene, propylene, pentamethylene, hexamethylene or octamethylene. Preferred is C1 to C4 straight or branched alkylene. It is methylene, ethylene, trimethylene, tetramethylene, propylene or the like.
The alkyl part of "alkoxycarbonylamino" means the same group as the above "alkyl". Preferred is carbonylamino substituted with C1 to C4 straight or branched alkoxy.

A substituent of "optionally substituted aryl" is hydroxy, carboxy, halogen, optionally substituted alkyl, optionally substituted alkenyl, optionally substituted alkynyl, optionally substituted alkoxy, cycloalkyl, cycloalkynyl, alkoxycarbonyl, nitro, nitroso, amino, optionally substituted amino, azide, amidino, guanidino, optionally substituted aryl, optionally substituted heteroaryl, optionally substituted alkylthio, cyano, isocyano, mercapto, optionally substituted carbamoyl, optionally substituted alkylsulfonyl, optionally substituted arylsulfonyl, optionally substituted sulfamoyl, sulfoamino, formyl, alkylcarbonyl, optionally substituted arylcarbonyl, alkylcarbonyloxy, hydrazino, optionally substituted nonaromatic heterocycle, optionally substituted alkylenedioxy, alkylene optionally intervened with heteroatom(s) or the like. Preferred is halogen, cyano, optionally substituted carbamoyl, optionally substituted alkoxy, optionally substituted alkyl, optionally substituted aryl, optionally substituted alkylenedioxy, optionally substituted heteroaryl, optionally substituted nonaromatic heterocycle, optionally substituted amino, hydroxy, formyl, optionally substituted alkenyl, alkylthio, alkylene optionally intervened with heteroatom(s), alkoxycarbonyl, alkylsulfonyl or the like. Optionally substituted alkylenedioxy and alkylene optionally intervened with heteroatom(s) are preferably substituted at the neighboring positions on the aryl.

As a substituent of "optionally substituted aryl", especially preferred is halogen, cyano, carbamoyl, optionally substituted alkoxy (e.g., haloalkoxy), optionally substituted alkyl (e.g., haloalkyl or hydroxyalkyl), alkylenedioxy, heteroaryl, hydroxy, formyl, optionally substituted alkenyl (e.g., alkoxycarbonylalkenyl), alkylthio or alkoxycarbonyl.

As a substituent of "optionally substituted aryl" for R², especially preferred is optionally substituted alkoxy (e.g., haloalkoxy).
As a substituent of "optionally substituted aryl" for R³, especially preferred is optionally substituted alkoxy (e.g., haloalkoxy), halogen, optionally substituted alkyl (e.g., haloalkyl), cyano, heteroaryl, alkylthio or hydroxy.
As a substituent of "optionally substituted aryl" for R^{3'}, especially preferred is halogen, alkyl, alkoxy, alkylenedioxy or cyano. The aryl can be optionally monosubstituted or disubstituted by these substituents.
As a substituent of "optionally substituted aryl" for R⁵, especially preferred is halogen, optionally substituted alkoxy (e.g., haloalkoxy), heteroaryl, alkylthio, optionally substituted alkyl (e.g., haloalkyl or hydroxyalkyl), formyl, optionally substituted alkenyl (e.g., alkoxycarbonylalkenyl), cyano or carbamoyl.
As a substituent of "optionally substituted aryl" for R^{5'}, especially preferred is optionally substituted alkyl (e.g., haloalkyl), halogen, optionally substituted alkoxy (e.g., haloalkoxy or alkoxyalkoxy), alkylthio or alkoxycarbonyl.

"Cycloalkyl" is C3 to C8 cyclic alkyl. For example, it is cyclopropyl, cyclobutyl, cyclopentyl, cyclohexyl, cycloheptyl or cyclooctyl. Preferred is C3 to C6 cyclic alkyl. It is cyclopropyl, cyclobutyl, cyclopentyl or cyclohexyl.
"Cycloalkenyl" is C3 to 8 cyclic alkenyl which is the above "cycloalkyl" with 1 or more double bond(s). For example, it is 1-cyclopropene-1-yl, 2-cyclopropene-1-yl, 1-cyclobutene-1-yl, 2-cyclobutene-1-yl, 1-cyclopentene-1-yl, 2-cyclopentene-1-yl, 3-cyclopentene-1-yl, 1-cyclohexene-1-yl, 2-cyclohexene-1-yl, 3-cyclohexene-1-yl, 1-cycloheptene-1-yl, 2-cycloheptene-1-yl, 3-cycloheptene-1-yl or 4-cycloheptene-1-yl.

"Hydroxyalkyl" means a group which is the above "alkyl" whose hydrogen atom(s) is(are) substituted with 1 to 6 hydroxy. For example, it is hydroxymethyl, dihydroxymethyl, 2-hydroxyethyl, 1-hydroxyethyl, 3-hydroxy-n-propyl, 2-hydroxy-n-propyl, 1-hydroxy-n-propyl or 1-hydroxy-1-methylethyl. Preferred is hydroxymethyl, dihydroxymethyl, 2-hydroxyethyl, 1-hydroxyethyl, 3-hydroxy-n-propyl, 2-hydroxy-n-propyl, 1-hydroxy-n-propyl or 1-hydroxy-1-methylethyl.
A substituent of "optionally substituted alkyl" is hydroxy, carboxy, halogen, optionally substituted alkoxy, cycloalkyl, cycloalkynyl, alkoxycarbonyl, nitro, nitroso, amino, optionally substituted amino, azide, amidino, guanidino, optionally substituted aryl, optionally substituted heteroaryl, optionally substituted alkylthio, cyano, isocyano, mercapto, optionally substituted carbamoyl, optionally substituted alkylsulfonyl, optionally substituted arylsulfonyl, optionally substituted sulfamoyl, sulfoamino, formyl, alkylcarbonyl, optionally substituted arylcarbonyl, alkylcarbonyloxy, hydrazino, optionally substituted nonaromatic heterocycle, optionally substituted alkylenedioxy, alkylene optionally intervened with heteroatom(s), -C(=O)-nonaromatic heterocycle or the like. Preferred is halogen, optionally substituted amino, hydroxy, alkoxy, alkoxycarbonyl, carboxy, cyano or the like.
A substituent of "optionally substituted amino" is alkylsulfonyl, optionally substituted alkyl (e.g., alkoxyalkyl), optionally substituted aryl, alkylcarbonyl, alkoxycarbonyl, alkylene optionally intervened with heteroatom(s) or the like. A substituent on amino (e.g., alkylene optionally intervened with -O- or -S-) can be taken together with the neighboring nitrogen atom to form a ring.
A substituent of "optionally substituted alkoxy" is the same as the substituent of the above "optionally substituted alkyl". Especially preferred is halogen, alkoxy, optionally substituted amino, hydroxy, cyano or the like.
A substituent of "optionally substituted alkenyl" is the same as the substituent of the above "optionally substituted alkyl". Especially preferred is alkoxycarbonyl, carboxy, halogen, optionally substituted amino, hydroxy, alkoxy, cyano or the like.
A substituent of "optionally substituted alkynyl" is the same as the substituent of the above "optionally substituted alkyl". Especially preferred is hydroxy, cyano or the like.
A substituent of "optionally substituted carbamoyl" is the same as the substituent of the above "optionally substituted amino". Especially preferred is optionally substituted alkyl, alkylene optionally intervened with heteroatom(s) or the like. A substituent on amino of carbamoyl (e.g., alkylene optionally intervened with -O- or -S-) can be taken together with the neighboring nitrogen atom to form a ring.

A substituent of "optionally substituted 5 or 6-membered ring optionally containing heteroatom(s) formed by taking together R¹ and R² with the neighboring carbon" is the same as the substituent of the above "optionally substituted aryl". Especially preferred is alkyl, halogen, cyano or the like.
A substituent of "optionally substituted sulfamoyl" is the same as the substituent of the above "optionally substituted amino". Especially preferred is optionally substituted alkyl or the like.

A substituent of "optionally substituted heteroaryl" is the same as the substituent of the above "optionally substituted aryl". Especially preferred is halogen, cyano, carbamoyl, optionally substituted alkoxy (e.g., haloalkoxy), optionally substituted alkyl (e.g., haloalkyl, hydroxyalkyl or arylalkyl), alkylenedioxy, heteroaryl, hydroxy, formyl, optionally substituted alkenyl (e.g., alkoxycarbonylalkenyl), alkylthio or alkoxycarbonyl.

A substituent of "optionally substituted nonaromatic heterocycle" is the substituent of the above "optionally substituted aryl" and oxo. Especially preferred is optionally substituted aryl, heteroaryl, oxo, alkylsulfonyl or carbamoyl.
As a substituent of "optionally substituted nonaromatic heterocycle" for R^{2'}, especially preferred is oxo or alkylsulfonyl.
As a substituent of "optionally substituted nonaromatic heterocycle" for R⁵, especially preferred is optionally substituted aryl, heteroaryl, oxo, alkylsulfonyl or carbamoyl.

A substituent of "optionally substituted alkylthio" is the same as the substituent of the above "optionally substituted alkyl". Especially, it is halogen, alkoxy or the like.

"Alkylenedioxy" means C1 to C6 alkylenedioxy. Preferred is C1 to C3 alkylenedioxy. For example, it is methylenedioxy, ethylenedioxy or propylenedioxy.
A substituent of "optionally substituted alkylenedioxy" is alkoxy or the like.
"Alkylene optionally intervened with heteroatom(s)" means C1 to C6 alkylene which is the above "alkylene" optionally intervened with heteroatom(s) (-NH-, -O-, -S-). For example, it is -CH₂-CH₂-CH₂-CH₂-, - CH₂- CH₂-O- CH₂- CH₂- or - CH₂- CH₂- CH ₂-NH-.
Examples of aryl substituted with "alkylene optionally intervened with heteroatom(s)" are the followings.

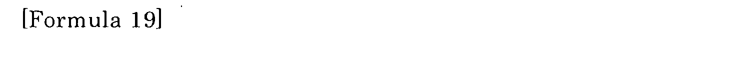

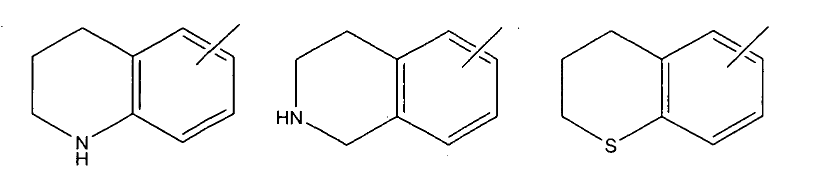

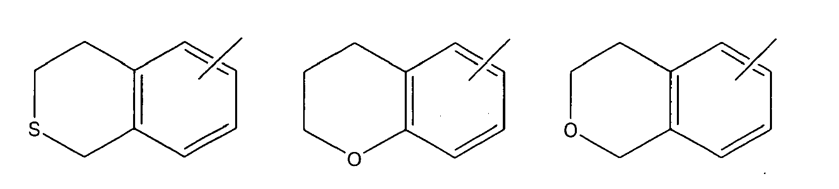

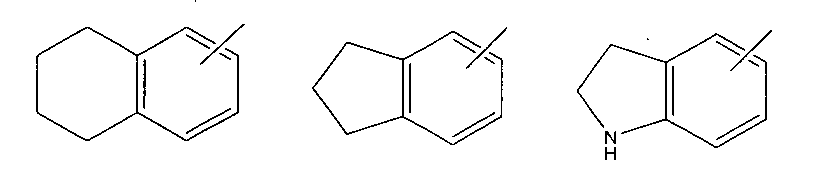

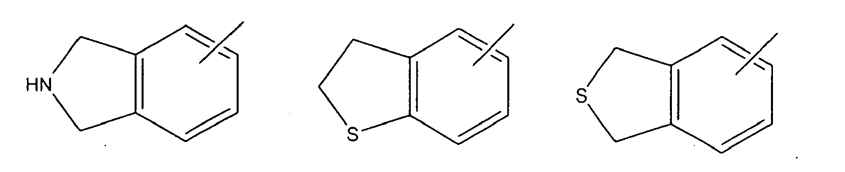

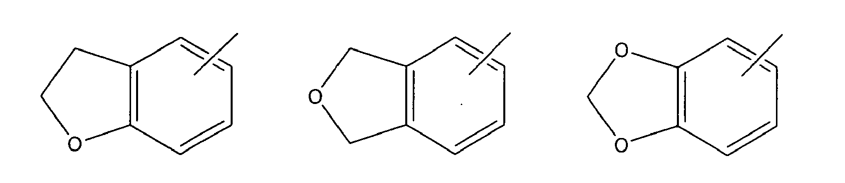

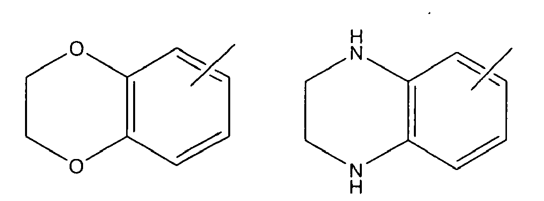

The alkyl part of "alkylcarbonyl" or "alkylcarbonyloxy" is the same as the above "alkyl". The alkoxy part of "alkoxycarbonyl" or "alkoxycarbonylalkenyl" is the same as the above "alkoxy". The alkenyl part of "alkoxycarbonylalkenyl" is the same as the above "alkenyl". The aryl part of "arylsulfonyl" is the same as the above "aryl".

A method for producing a compound of the present invention is explained below.
A method for synthesizing salicylic acid anilides, followed by the introduction of acetylene derivatives by Sonogashira reaction, can be performed as below. 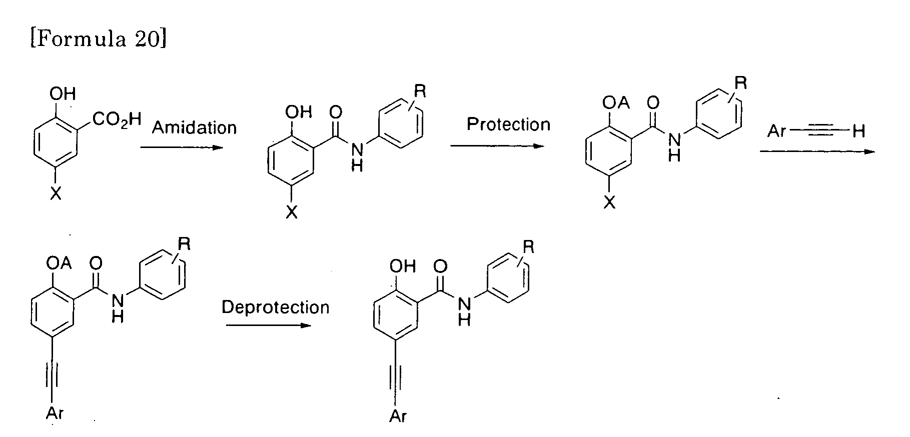 (R is any substituent, X is halogen or the like, A is protection group (e.g., mesyl) and Ar is aromatic ring. The aromatic ring is optionally substituted.)
Amidation can be performed under conventional reaction conditions. For example, amide derivatives can be obtained by dissolving salicylic acid derivatives and aniline derivatives in a solvent such as cholorobenzene, xylene or 1,4-dioxane, and then reacting with PCl₃ or the like. The reaction can be performed at about 50 to 200 °C, for example, at about 150 °C.
Amide derivative having phenolic hydroxyl group can be converted to mesylate derivatives by reacting with methanesulfonyl chloride. As a solvent, tetrahydrofuran, methylene chloride, pyridine or the like can be used. The reaction can be performed at about 0 to 100 °C, for example, at room temperature. This reaction can be performed under the presence of base such as triethylamine or N, N-diisopropylethylamine.
Next, Sonogashira reaction of mesylate derivatives with acetylene derivatives followed by the deprotection, if necessary, gives the desired product. Sonogashira reaction can be performed with a solvent such as dimethylformamide, toluene or 1,2-dimethoxyethane at 0 to 100 °C, for example, about 50 °C. This reaction can be performed under the presence of the catalytic amount of Pd(PPh₃)Cl₂, the catalytic amount of Cul, about 2 equivalents of triethylamine, N,N-diisopropylethylamine, potassium carbonate or the like. Acetylene derivative can be used at about 1.1 to 1.8 equivalents, for example, at about 1.5 equivalents. Deprotection can be performed under basic conditions, for example, by adding sodium hydroxide solution, potassium hydroxide solution or potassium carbonate solution in alcohol solvent (e.g., methanol or ethanol).

A method for the reaction of aromatic boronic acids with salicylanilides under Suzuki reaction conditions is explained below. 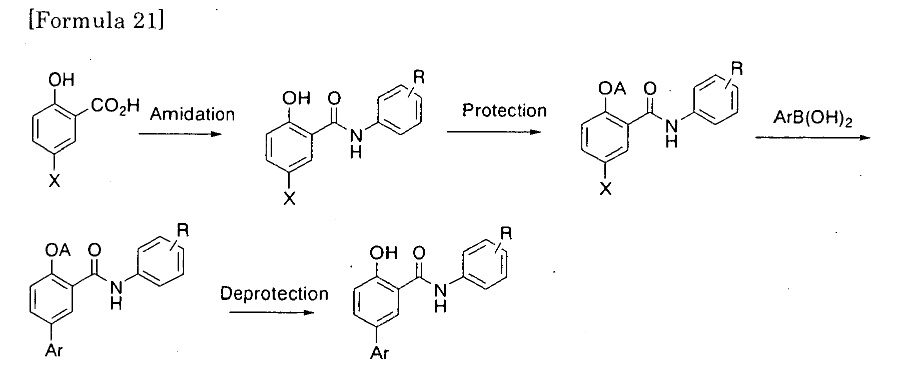 (X is halogen, A is protection group (e.g., mesyl), R is any substituent and Ar is aromatic ring. The aromatic ring is optionally substituted.)

Amidation, protection and deprotection process can be performed as above.
Suzuki reaction can be used for obtaining biphenyl amide derivatives from amide derivatives. More specifically, biphenyl amide derivative can be obtained by protecting hydroxy group, and then reacting with boronic acid derivatives in the presence of PdCl₂ (dppf) and potassium carbonate. Dimethylformamide, toluene or 1,2-dimethoxyethane can be used as a solvent. The reaction can be performed at room temperature to 150 °C, for example, at 120 °C.

A method for introducing aromatic ring on aniline side by Suzuki reaction after synthesizing salicylamide derivative is explained below. 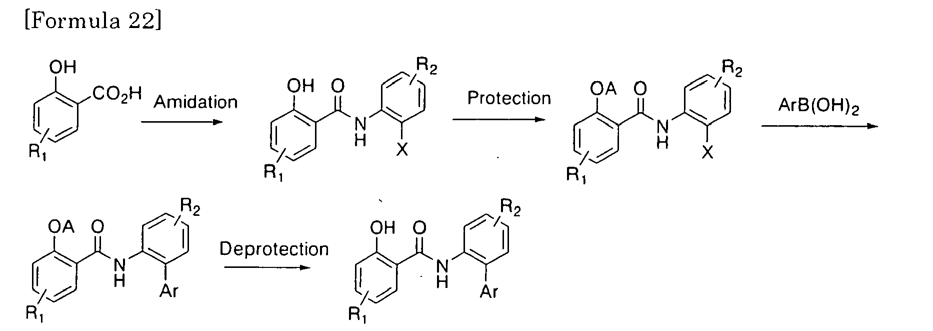 (R₁ or R₂ is any substituent, X is halogen or the like, A is protection group (e.g., mesyl) and Ar is aromatic ring. The aromatic ring is optionally substituted.)

Amidation process or Suzuki reaction can be performed as above.
Protection process can be performed with methyl iodide in the presence of base. Potassium carbonate, sodium hydride, sodium hydroxide or the like can be used as base. The reaction can be performed at 0 to 100 °C, for example, at room temperature.
Deprotection of methyl ether derivative can be performed with BBr₃, Me₃SiI or the like. The reaction can be performed at 0 to 100 °C, for example, at room temperature with chloroform, methylene chloride or the like as a solvent.
Although biaryl part is synthesized after the salicylanilide formation in the above reaction scheme, it is possible that biaryl amine part is synthesized before salicylanilide formation.

Carboxylic acid with a substituent at the 3-position of salicylic acids which are not commercially available can be synthesized by the carbonylation of the corresponding phenol derivative as below. 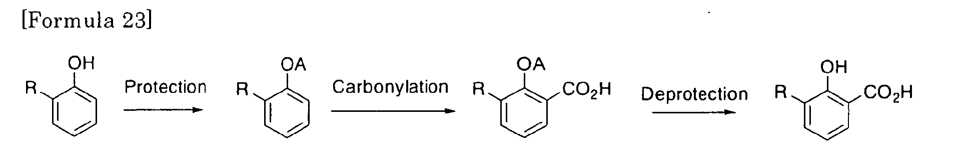 (R is any substituent and A is protection group.)
Protection, carbonylation and deprotection process can be performed under the conventional conditions.
Nitrile derivative can be synthesized by the reaction of metal cyanide such as CuCN and the corresponding bromine derivatives or triflate derivatives.

A method for synthesizing ethers by the reaction of alkoxide and phenol-protected salicylanilide is explained below. 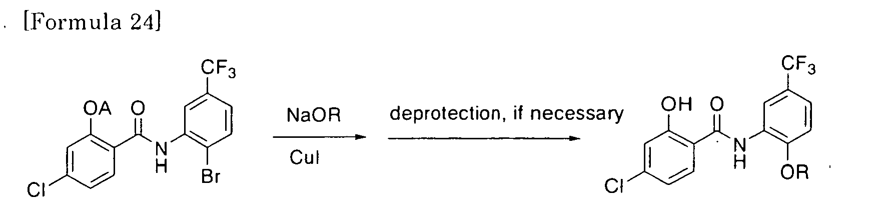 (A is protection group and R is any substituent (e.g., alkyl).)

Methyl ether derivatives can be obtained by adding bromine derivative to metal alcoholate in alcohol solution in the presence of catalytic amount of Cul. Sodium methoxide, sodium ethoxide, phenoxide or the like can be used as metal alcoholate. Dimethylformamide, alcohol, tetrahydrofuran, dimethylsulfoxide, 1,4-dioxane or the like can be used as a solvent. The reaction can be performed at about 0 to 150 °C, for example, at about 90 °C. It is preferable that phenolic hydroxyl group of anilide salicylate is protected before this reaction. If necessary, it can be deprotected afterward.

A synthetic method of ketones by the reaction of boronic esters, in which phenols of salicylanilides are protected, with acid chloride followed by removing protecting group of phenol in the final step is explained below. 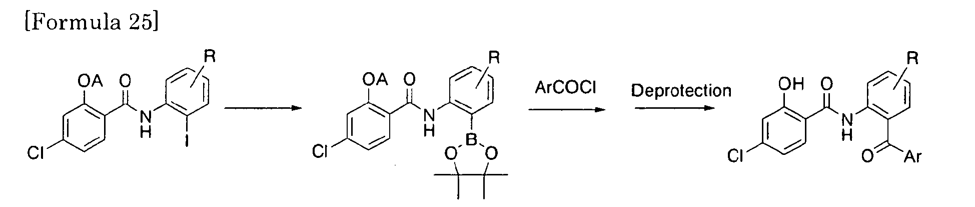 (A is protection group, R is any substituent and Ar is aromatic ring. The aromatic ring is optionally substituted.)

Boronic ester derivative can be obtained by the reaction of bis(pinacolate)diboron with iodide derivatives in the presence of PdCl₂(dppf) and potassium acetate. The reaction can be performed at about 0 to 150 °C, for example, at about 80 °C in dimethylsulfoxide or the like.
The desired ketone derivatives can be obtained by the reaction of boronic ester derivatives obtained with acid chloride derivatives in the presence of PdCl₂(dppf) and potassium carbonate followed by the deprotection, if necessary. The reaction can be performed at about 0 to 150 °C, for example, at about 100 °C in acetone, toluene or the like.

A method for obtaining the desired compounds, by appropriately reducing the salicylanilide derivatives, in which phenolic hydroxyl group is protected and nitro group is present at the aniline part, to give amine, followed by the condensation of resulting amine with aromatic carboxylic acid to give amide derivative and, if necessary, removing the protection group afterwards, is explained. 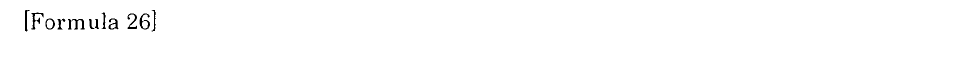 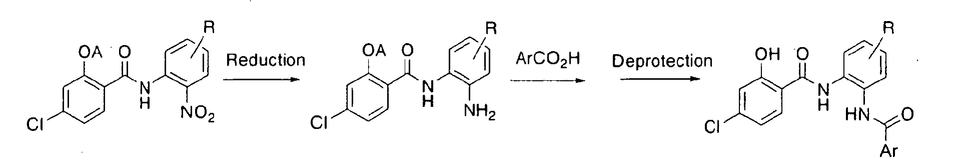 (A is protection group, R is any substituent and Ar is aromatic ring. The aromatic ring is optionally substituted.)

Catalytic reduction or the like can be used as a reduction method. For example, the reduction can be performed in a solvent such as alcohol, tetrahydrofuran or ethyl acetate using a catalyst such as 5% palladium carbon, Raney nickel or platinum oxide under hydrogen atmosphere.
Reduction step can be performed with a reducing agent such as SnCl₂, Fe or Zn. In this case, the reaction is carried out in a solvent such as alcohol at 0 to 100 °C, for example, at 70 °C.
Aniline derivatives thus obtained are subjected to the reaction with carboxylic acid derivative followed by the deprotection, if necessary, to give the desired products. For example, the reaction can be performed with acid chloride as carboxylic acid derivative in the presence of base such as triethylamine, N,N-diisopropylethylamine or pyridine. Tetrahydrofuran, methylene chloride, chloroform or the like can be used as a solvent.

A method for synthesizing compounds having a reversed amide bond in aniline part of salicylanilide, compared to the amide bond shown in the above method, is explained below. The corresponding sulfonamide derivative can be synthesized using sulfonic acids as starting material a instead of carboxylic acids. 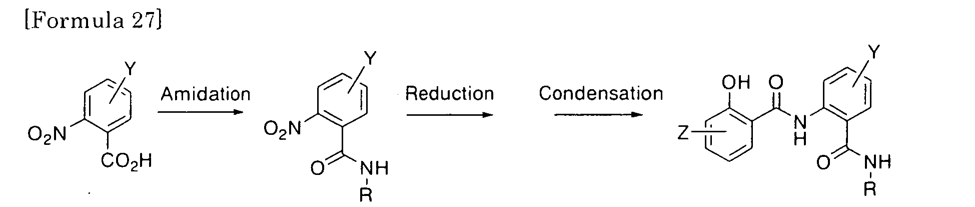 (Y, R and Z are each independently any substituent.)

Amidation can be performed by reacting salicylic acid derivative with a halogenatation reagents to give acid chloride, and then reacting with aniline derivative in the presence of base such as triethylamine. In the acid chloride preparation step, methylene chloride, toluene, tetrahydrofuran or the like can be used as a solvent. The reaction between phenylamine derivative and acid chloride can be performed in tetrahydrofuran, methylene chloride, pyridine or the like. Additionally, it can be also performed in organic solvent and water bilayer conditions. In that case, sodium hydrogen carbonate, potassium carbonate, sodium carbonate or the like can be used as base.
Reduction and condensation steps can be performed as above.

A method for synthesizing sulfonamides by using anthranylanilide derived from o-nitro benzoic acid derivative, and appropriate sulfonyl chlorides is explained below. 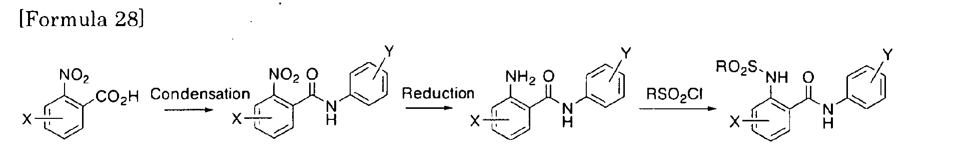 (X, Y or R is any substituent.)

Reduction and condensation process can be performed as above.
Sulfonamide derivatives can be obtained by reacting benzenesulphonyl chloride with amine derivative in a solvent such as pyridine. The reaction can be performed at 0 to 100 °C, for example, at room temperature.

Compounds of the present invention include producable and pharmaceutically acceptable salts of the compounds of the present invention. "A pharmaceutically acceptable salt" includes, for example, salts of inorganic acid such as hydrochloric acid, sulfuric acid, nitric acid or phosphoric acid; salts of organic acid such as para-toluenesulfonic acid, methanesulfonic acid, oxalic acid or citric acid; salts of organic base such as ammonium, trimethylammonium or triethylammonium; salts of alkali metal such as sodium or potassium; salts of alkaline-earth metal such as calcium or magnesium.

Compounds of the present invention include a solvate thereof and can be coordinate with any number of solvent molecules to compound (I) or (II). Preferred is hydrate.

When a compound of the present invention (I) or (II) has an asymmetric carbon atom, it contains racemic body and all stereoisomers (diastereoisomer, antipode or the like). When a compound of the present invention (I) or (II) has a double bond and there is geometrical isomer at a substituent position of double bond, it includes both types of the isomers.

A compound of the present invention can be used for therapy or prevention of CTGF related diseases, for example, a disease caused by CTGF production. Especially preferred is to use for therapy or prevention of diseases caused by CTGF overproduction. For example, it can be used for excessive cicatrization occurred from acute or recurrent injury by surgery or radiotherapy; fibrosing diseases of organ such as kidney, lung, liver, oculus, heart or skin comprising scleroderma, keloid or hypertrophic scar.
Abnormal expression of CTGF is shown with popular tissue cicatrization, tumor-like growth of skin or vascular continuous cicatrization and induces circulatory deterioration, hypertension, hypertrophy or the like. Furthermore, CTGF relates to various diseases caused by endothelial cell growth or migration, for example, cancers including skin fibroma, symptoms related to abnormal expression of endothelial cells, breast cancer desmoplastic fibroma (desmosplasis), hemangiolipoma or angioleiomyoma. The other related symptoms include atherosclerosis, systemic sclerosis (atherosclerotic lesion, inflammatory intestinal disease, Crohn disease, the other proliferative process which plays a central role in angiogenesis, arterial sclerosis or the like), arthritis, cancer, the other symptoms, angiogenesis which relates to glaucoma, inflammation because of disease or injury (joint fluid or the like), tumor growth and metastasis, interstitial diseases, skin diseases, arthritis (chronic rheumatoid arthritis or the like), arteriosclerosis, diabetic neuropathy, diabetic nephropathy, hypertension, the other nephropathy or fibrosing diseases caused by chemotherapy, radiation therapy, dialysis, homoplastic transplantation or graft rejection.

A cell breeding disorder also includes fibroplastic disorder and relates to, for example, overproduction of extracellular matrix. Such symptoms includes hepatic fibrosis, renal fibrosis, atherosclerosis, cardial fibrosis, adhesion or operation scar, although they are not restricted.

When a compound of the present invention is administered as a pharmaceutical composition, it can be orally or parenterally administered. Oral administration may be prepared and administered in the usual form such as tablets, granules, powders, capsules, pills, solutions, syrups, buccal tablets or sublingual tablets according to a well-known method. Parenteral administration can be preferably administered in any form which is usually used, for example, injection such as intramuscular or intravenous administration, suppository, percutaneous absorption agent or inhalation. Especially preferred is oral administration.

A pharmaceutical composition can be manufactured by mixing an effective amount of a compound of the present invention with various pharmaceutical additives suitable for the administered form, such as excipients, binders, moistening agents, disintegrators, lubricants or diluents as occasion demands. When the composition is an injection, a compound of the present invention with a suitable carrier can be sterilized to give a pharmaceutical composition.

Examples of the excipients include lactose, saccharose, glucose, starch, calcium carbonate and crystalline cellulose. Examples of the binders include methylcellulose, carboxymethylcellulose, hydroxypropylcellulose, gelatin and polyvinylpyrrolidone. Examples of the disintegrators include carboxymethylcellulose, sodium carboxymethylcellulose, starch, sodium alginate, agar and sodium lauryl sulfate. Examples of the lubricants include talc, magnesium stearate and macrogol. Cacao oil, macrogol, methylcellulose or the like can be used as a base material of suppositories. When the composition is manufactured as solutions, emulsified injections or suspended injections, dissolving accelerators, suspending agents, emulsifiers, stabilizers, preservatives, isotonic agents or the like which is usually used can be added. For oral administration, sweetening agents, flavors or the like can be added.

Although the dosage of a compound of the present invention as a pharmaceutical composition should be determined in consideration of age and body weight of the patient, the type and severity of the disease, the administration route or the like, a usual oral dosage for an adult is 0.05 to 100 mg/kg/day and preferably 0.1 to 10 mg/kg/day. Although the dosage for parenteral administration highly varies with administration routes, a usual dosage is 0.005 to 10 mg/kg/day and preferably 0.01 to 1 mg/kg/day. The dosage can be administered in one to several divisions per day.

This invention is further explained by the following Examples, Experimental Examples and Formulation Examples, which are not intended to limit the scope of the present invention. Synthesized compounds were confirmed by NMR spectrum, mass spectrum or the like. Data measured by mass spectrum are described in Tables.

### Example 1

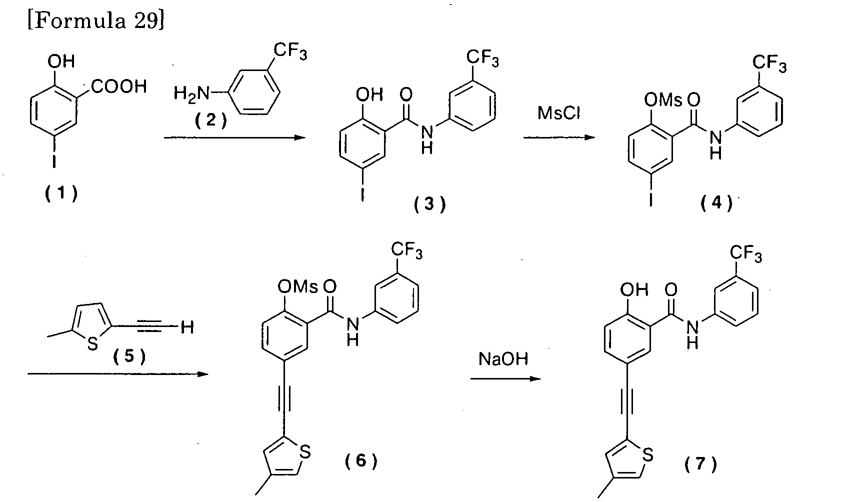

### Salicylanilide (3):

5-Iodosalicylic acid (1) (2.43 g, 8.215 mmol) and m-trifluoromethylaniline (2) (1.49 g, 8.215 mmol) were added to chlorobenzene (50 ml). PCl₃ (0.4 ml, 0.5eq) was added, and the mixture was heated at 150 °C for 2 hours. Chlorobenzene was evaporated under reduced pressure and the resulting crystals deposited from diethyl ether were collected by filtration to give 3.06g (82%) of a desired compound (3).
NMR(DMSO)δppm:6.85 (1H, d_{AB}, J=6Hz, Ar-H), 7.51 (1H, d_{AB}, J=6Hz, Ar-H), 7.62 (1H, d_{AB}, J=6Hz, Ar-H), 7.94 (1H, d_{AB}, J=6Hz, Ar-H), 8.18 (1H, d_{AB}, J=6Hz, Ar-H), 10.61 (1H, s, NH), 11.8 (1H, s, OH).

### Mesylate (4):

The above amide derivative (3) (0.5 g, 1.23 mmol) was dissolved in tetrahydrofuran (10 ml). Triethylamine (0.24 ml, 1.4 eq) and methanesulfonyl chloride (0.13 ml, 1.4 eq) were added, and the mixture was reacted at room temperature for 30 minutes. The solution was added to ice water (70 ml), extracted twice with acetic acid ethyl ester (100 ml), washed with water and dried (anhydrous sodium sulfate). The residue (4) 0.57 g (100%) obtained by condensing the solvent under reduced pressure was used directly in the next step.

### Acetylene derivative (6):

The above amide derivative (4) (0.53 g, 1.09 mmol) was dissolved in dimethylformamide (10 ml). Acetylene derivative (5) (334 mg, 1.5 eq), Pd(PPh₃)₂Cl₂(19 mg, 0.025 eq), CuI(0.05 eq) and triethylamine(0.30 ml, 2 eq) were added, and the mixture was reacted at 50 °C for 30 minutes under N₂ atmosphere. The reaction solution was added to ice water (50 ml), extracted twice with acetic acid ethyl ester (50 ml), washed three times with water (50 ml) and dried (anhydrous sodium sulfate). The residue obtained by evaporating the solvent under reduced pressure was purified by silica gel column chromatography (toluene- acetic acid ethyl ester =20-1) and recrystallized from n-hexane to give a desired compound (6) 0.456g (87%).
NMR(CDCl₃)δppm: 2.50 (3H, s, CH₃), 3.26 (3H, s, OMs), 6.68 (1H, s, Ar-H), 7.13 (1H, s, Ar-H), 7.26 to 7.46 (5H, m, Ar-H), 7.62 (1H, d_{AB}, J=6Hz, Ar-H), 7.96 (1H, d_{AB}, J=6Hz, Ar-H), 7.97 (1H, s, Ar-H), 8.02 (1H, s, Ar-H), 8.5 (1H, s, NH).
MS: 480(M+H)⁺

### Compound (7)

The above acetylene derivative (6) (399 mg, 0.825 mmol) was dissolved in ethanol (7 ml). 2N-sodium hydroxide solution (2.1 ml, 3 eq) was added, and the mixture was reacted at 80 °C for 30 minutes. The reaction solution is condensed under reduced pressure. To the residue, were added water (20 ml) and acetic acid ethyl ester (50 ml). 2 N-hydrochloric acid solution (2 ml) was added thereto under ice-cooling, extracted twice with acetic acid ethyl ester (50 ml), washed three times with water 50 ml and dried (anhydrous sodium sulfate). The residue obtained by evaporating the solvent under reduced pressure was purified by silica gel column chromatography (toluene-acetic acid ethyl ester =20-1) and recrystallized from n-hexane to give a desired compound (7) 280 mg (84%).
The melting point: 221-222 °C
NMR(CDCl₃+ CD₃OD)δppm:2.49 (3H, s, CH₃), 6.67 (1H, d_{AB}, J=2Hz, thiophene-H), 6.98 (1H, d_{AB}, J=6Hz Ar-H), 7.08 (1H, d_{AB}, J=2Hz, thiophen-H), 7.37 to 7.56 (5H, m, Ar-H), 7.89 (1H, d_{AB}, J=6Hz, Ar-H), 8.00 (1H, s, Ar-H), 10.05 (1H, s, NH)
IRν(KBr):3153, 1639, 1614, 1569, 1504, 1492, 1168, 1128cm⁻¹.
MS:400(M-H) 402(M+H)⁺

Compounds (8 to 41) in Table 1 to 6 were synthesized in a similar way as above.

**[Table 1]**

| | | |
|---|---|---|
| 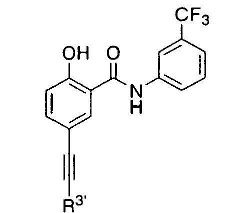 | | |

| Compound | R^{3'} | MS |
|---|---|---|
| 8 | 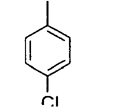 | 414(M-H)⁻ 416(M+H)⁺ |
| 9 | 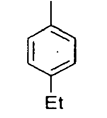 | 408(M-H)⁻ 410(M+H)⁺ |
| 10 | 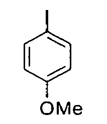 | 410(M-H)⁻ 412(M+H)⁺ |
| 11 | 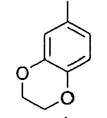 | 438(M-H)⁻ 440(M+H)⁺ |
| 12 | 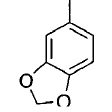 | 424(M-H)⁻ 426(M+H)⁺ |
| 13 | 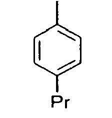 | 422(M-H)⁻ 424(M+H)⁺ |
| 14 | 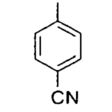 | 405(M-H)⁻ |
| 15 | 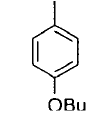 | 452(M-H)⁻ 454(M+H)⁺ |
| 16 | 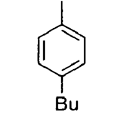 | 438(M+H)⁺ |

**[Table 2]**

| | | |
|---|---|---|
| 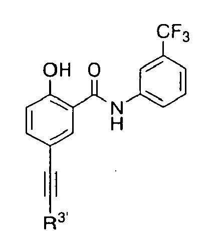 | | |

| Compound | R^{3'} | MS |
|---|---|---|
| 17 | 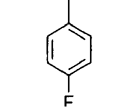 | 398(M-H)⁻ 400(M+H)⁺ |
| 18 | 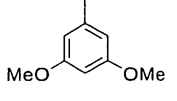 | 440(M-H)⁻ 442(M+H)⁺ |

**[Table 3]**

| | | |
|---|---|---|
| 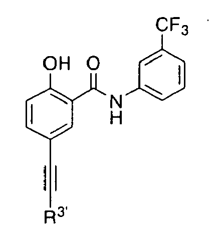 | | |

| Compound | R^{3'} | MS |
|---|---|---|
| 19 | 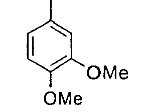 | 440(M-H)⁻ 442(M+H)⁺ |
| 20 | 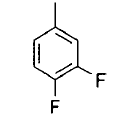 | 416(M-H)⁻ |
| 21 | 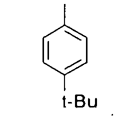 | 436(M-H)⁻ 438(M+H)⁺ |
| 22 | 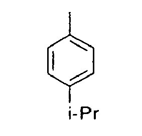 | 422(M-H)⁻ 424(M+H)⁺ |

**[Table 4]**

| | | | |
|---|---|---|---|
| 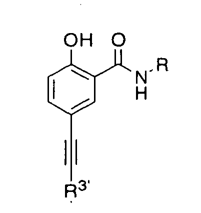 | | | |

| Compound | R^{3'} | R | MS |
|---|---|---|---|
| 23 | 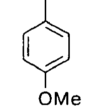 | 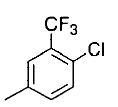 | 444(M-H)⁻ 446(M+H)⁺ |
| 24 | 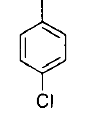 | 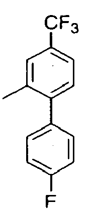 | 508(M-H)⁻ 510(M+H)⁺ |
| 25 | 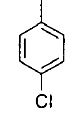 | 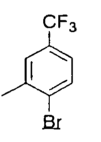 | 492(M-H)⁻ |

**[Table 5]**

| | | | |
|---|---|---|---|
| 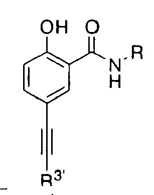 | | | |

| Compound | R^{3'} | R | MS |
|---|---|---|---|
| 26 | 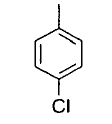 | 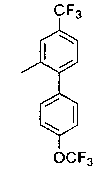 | 575(M-H)⁻ |
| 27 | 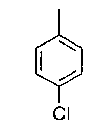 | 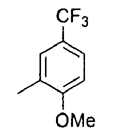 | 444(M-H)⁻ 446(M+H)⁺ |
| 28 | 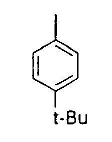 | 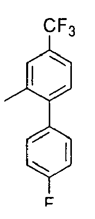 | 530(M-H)⁻ 532(M+H)⁺ |
| 29 | 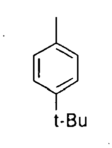 | 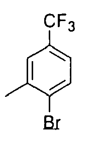 | 514(M-H)⁻ 516(M+H)⁺ |
| 30 | 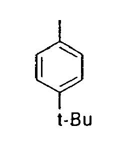 | 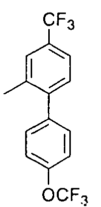 | 596(M-H)⁻ 598(M+H)⁺ |
| 31 | 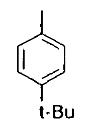 | 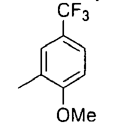 | 466(M-H)⁻ 468(M+H)⁺ |
| 32 | 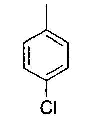 | 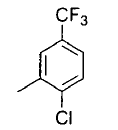 | 448(M-H)⁻ |

**[Table 6]**

| | | |
|---|---|---|
| 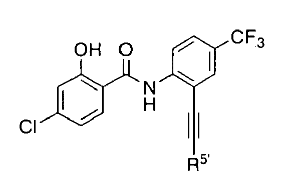 | | |

| Compound | R^{5'} | MS |
|---|---|---|
| 33 | 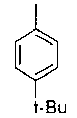 | 470(M-H)⁻ |
| 34 | t-Bu | 396(M+H)⁺ |
| 35 | 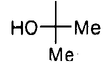 | 396(M-H)⁻ 398(M+H)⁺ |
| 36 | 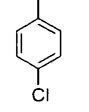 | 450(M+H)⁺ |
| 37 | 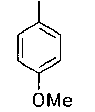 | 445(M)⁺ |
| 38 | 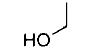 | 368(M-H)⁻ 370(M+H)⁺ |
| 39 | 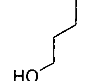 | 398(M+H)⁺ |
| 40 | 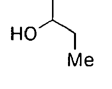 | 396(M⁻+H)⁻ ⁻ 398(M+H)⁺ |
| 41 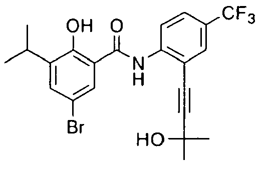 | | 482(M-H)⁻ 484 (M+H)⁺ |

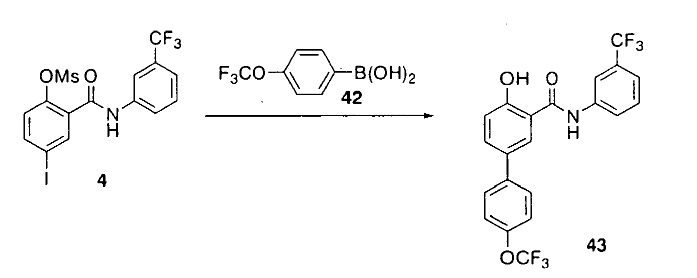

### Biphenyl derivative (43):

The above iodide derivative (4) (250 mg, 0.515 mmol) was dissolved in dimethylformamide (5 ml). Boronic acid (42) (159 mg, 1.5 eq), PdCl₂ (dppf) (159 mg, 0.15 eq) and potassium carbonate (214 mg, 3 eq) were added, and the mixture was reacted at 80 °C for 1 hour under N₂ atmosphere. The reaction solution was added to ice water (50 ml), extracted twice with acetic acid ethyl ester (50ml), washed three times with water (50 ml) and dried over anhydrous sodium sulfate. The residue obtained by evaporating the solvent under reduced pressure was purified by silica gel column chromatography (toluene-acetic acid ethyl ester =20-1) and the following recrystallization from n-hexane to give a desired product (43) 106 mg (50 %).
The melting point: 200-202 °C
NMR (CDCl₃ )δppm:17.08(1H, d_{AB}, J=6Hz Ar-H), 7.28 to 7.66 (8H, m, Ar-H), 7.91 (1H, d_{AB}, J=6Hz, Ar-H), 8.00 (1H, s, Ar-H), 8.23 (1H, s, NH), 10.25 (1H, s, OH).
IRνₘₐₓ (KBr):3156, 1637, 1614, 1570, 1496, 1334, 1290, 1137cm⁻¹
MS:440(M-H)⁻, 442(M+H)⁺

Compounds (44 to 58) in Table 7and 8 were synthesized in a similar way as above.

**[Table 7]**

| | | |
|---|---|---|
| 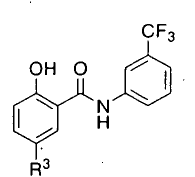 | | |

| Compound | R³ | MS |
|---|---|---|
| 44 | 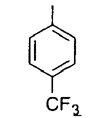 | 424(M-H)⁻ |
| 45 | 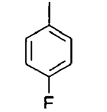 | 376(M+H)⁺ |
| 46 | 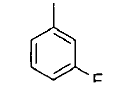 | 374(M-H)⁻ 376(M+H)⁺ |
| 47 | 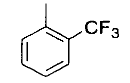 | 424(M-H)⁻ 426(M+H)⁺ |
| 48 | 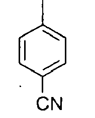 | 381(M-H)⁻ |
| 49 | 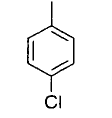 | 390(M-H)⁻ 392(M+H)⁺ |
| 50 | 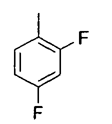 | 392(M-H)⁻ 394(M+H)⁺ |
| 51 | 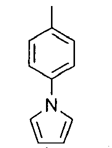 | 421(M-H)⁻ 423(M+H)⁺ |
| 52 | 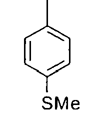 | 402(M-H)⁻ 404(M+H)⁺ |

**[Table 8]**

| | | | |
|---|---|---|---|
| 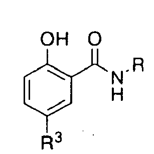 | | | |

| Compound | R³ | R | MS |
|---|---|---|---|
| 53 | 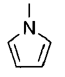 | 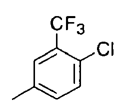 | 379(M-H)⁻ 381(M+H)⁺ |
| 54 | 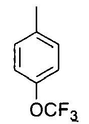 | 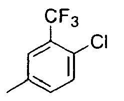 | 474(M-H)⁻ 476(M+H)⁺ |
| 55 | 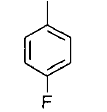 | 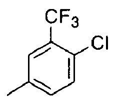 | 408(M-H)⁻ 410(M+H)⁺ |
| 56 | 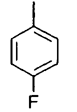 | 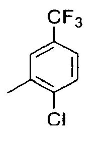 | 408(M-H)⁺ 410(M+H)⁺ |
| 57 | 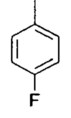 | 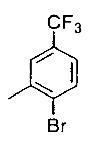 | 452(M-H)⁻ 454(M+H)⁺ |
| 58 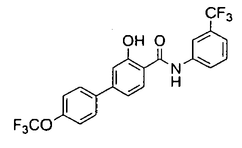 | | | 440(M-H)⁻ 442(M+H)⁺ |

### Example 3

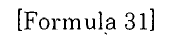

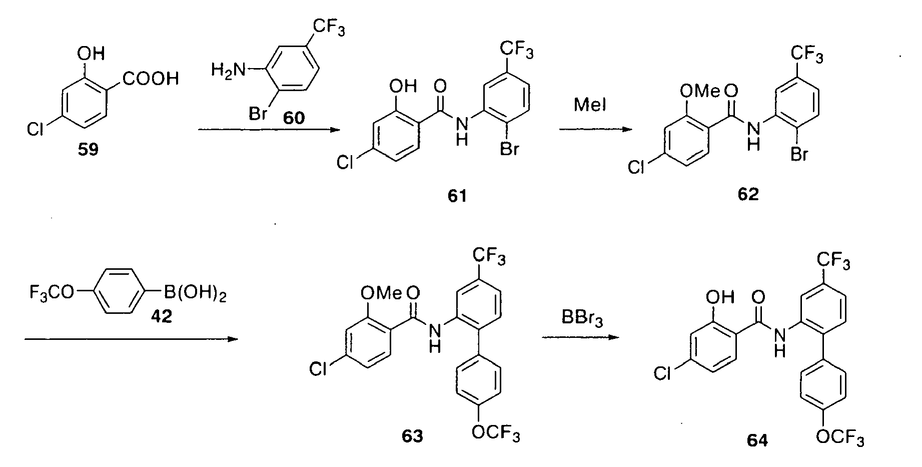

### Salicylamide derivative (61):

4-chlorosalicylate (59) (1.08 g, 6.249 mmol) and 2-bromo-5-trifluoromethyl-aniline (60) (1.5 g, 6.249mmol) were added to chlorobenzene (15 ml). PCl₃ (0.27 ml, 0.5eq) was added, and the mixture was heated at 150 °C for 2 hours. The residue obtained by evaporating the solvent under reduced pressure was purified by silica gel column chromatography (toluene-acetic acid ethyl ester =9-1) and the following recrystallization from n-hexane to give a desired compound (61) (2.02 g, 82%).
The melting point: 154-155 °C
NMR (CDCl₃) δppm: 6.96 (1H, d_{AB}, J=6Hz, Ar-H), 7.09 (1H, s, Ar-H), 7.33 (1H, d_{AB}, J=6Hz, Ar-H), 7.51 (1H, d_{AB}, J=6Hz, Ar-H), 8.59 (1H, s, Ar-H), 8.77 (1H, s, NH), 11.80 (1H, s, OH).
MS: 392(M-H), 394(M+H)⁺.

### Methyl ether derivative (62):

Amide derivative (61) (577 mg, 1.46 mmol) was dissolved in dimethylformamide (6 ml). Potassium carbonate (0.40 g, 2 eq) and methyl iodide (0.18ml, 2 eq) were added, and the mixture was reacted at room temperature for 1 hour. The mixture was added to ice water (30 ml), extracted twice in acetic acid ethyl ester (30 ml), washed three times with water (30 ml) and dried over anhydrous sodium sulfate. The residue obtained by evaporating the solvent under reduced pressure was purified by silica gel column chromatography (toluene) and the following recrystallization from n-hexane to give a desired product (62) (498 mg, 83%).
mp: 144-145 °C
NMR (CDCl₃)δppm:4.12 (3H,s, OMe), 7.07 (1H, S, Ar-H), 7.14 (1H, d_{AB}, J=6Hz, Ar-H), 7.26 (1H, d_{AB}, J=6Hz, Ar-H), 8.25 (1H, d_{AB}, J=6Hz, Ar-H), 9.03 (1H, s, NH), 10.51 (1H, s, OH).
MS: 406(M-H)-, 408(M+H)⁺.

### Biphenyl derivative (63):

Amide (13) (300 mg, 0.734 mmol) was dissolved in dimethylformamide (6 ml). Boronic acid (42) (227 mg, 1.5 eq), PdCl₂ (dppf) (90 mg, 0.15 eq) and potassium carbonate (304mg, 3eq) were added, and the mixture was reacted at 80 °C for 2 hour under N₂ atmosphere. The reaction solution was added to ice water (30 ml), extracted twice with acetic acid ethyl ester (30 ml), washed three times with water (30 ml) and dried over anhydrous sodium sulfate. The residue obtained by evaporating the solvent under reduced pressure was purified by silica gel column chromatography (toluene-acetic acid ethyl ester =9-1) and the following recrystallization from n-hexane to give a desired product (63) (332 mg, 90%).
mp: 181-182 °C
NMR (CDCl3) δppm : 3.34 (3H, s, OMe), 6.86 (1H, S, Ar-H), 7.10 (1H, d_{AB}, J=6Hz, Ar-H), 7.31 to 7.51 (6H, m, Ar-H), 8.24 (1H, d_{AB}, J=6Hz, Ar-H), 9.0 (1H, s, Ar-H), 9.80 (1H, s, NH).
MS: 488(M-H)⁻, 490(M+H)⁺.

### Compound (64):

Methyl ether derivative (63) (259 mg, 0.529 mmol) was dissolved in CH₂Cl₂ (10 ml). 1M/L BBr₃/ CH₂Cl₂ solution (0.8 ml, 1.5 eq) was added, and the mixture was stirred at room temperature for 30 minutes. To the reaction solution, were added ice water (20 ml) and saturated sodium bicarbonate water (2 ml), and washed. After washing with water, the solution was dried over anhydrous sodium sulfate. The residue obtained by evaporating the solvent under reduced pressure was purified by silica gel column chromatography (toluene-acetic acid ethyl ester =9-1) and the following recrystallization from n-hexane to give a desired product (64) (213 mg, 85 %).
mp: 136-137°C
NMR (CDCl3) δppm: 6.75 (1H, s, Ar-H), 7.03 (1H, s, Ar-H), 7.41 to 7.53 (6H, m, Ar-H), 7.88 (1H, s, Ar-H), 8.67(1H, s, NH), 11.92 (1H, s, OH).
IRvₘₐₓ (KBr):3290, 3098, 1630, 1600, 1580, 1553, 1430, 1331, 1248, 1168, 1129cm⁻¹.
MS:474(M-H) 476(M+H)⁺.

Compounds (65 to 151 and 3-0 to 3-48) in Table 9 to 37 were synthesized in a similar way as above.

**[Table 9]**

| | | |
|---|---|---|
| 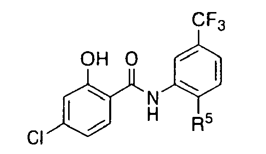 | | |

| Compound | R⁵ | MS |
|---|---|---|
| 65 | 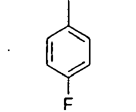 | 408(M-H)⁻ 410(M+H)⁺ |
| 66 | 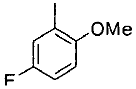 | 438(M-H)⁻ 440(M-H)⁺ |
| 67 | 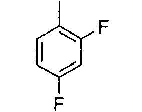 | 427(M-H)⁻ |
| 68 | 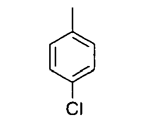 | 455(M-H)⁻ 457(M+H)⁺ |
| 69 | 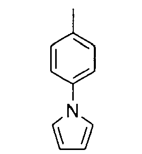 | 436(M-H)⁻ 438(M+H)⁺ |
| 70 | 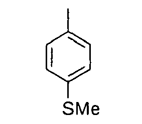 | 436(M-H)⁻ 438(M+H)⁺ |
| 71 | 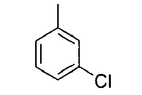 | 424(M-H)⁻ 426(M+H)⁺ |
| 72 | 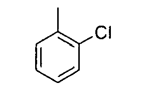 | 424(M-H)⁻ 426(M+H)⁺ |
| 73 | 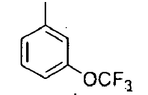 | 474(M-H)⁻ 476(M+H)⁺ |
| 74 | 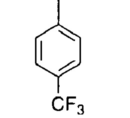 | 458(M-H)⁻ 460(M+H)⁺ |
| 75 | 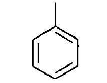 | 392(M+H)⁺ |

**[Table 10]**

| | | |
|---|---|---|
| 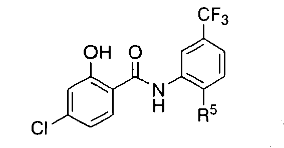 | | |

| Compound | R⁵ | MS |
|---|---|---|
| 76 | 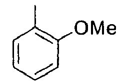 | 444(M+Na)⁺ |
| 77 | 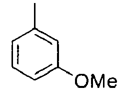 | 422(M+H)⁺ |
| 78 | 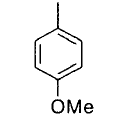 | 422(M+H)⁺ |
| 79 | 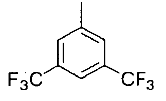 | 528(M+H)⁺ |

**[Table 11]**

| | | |
|---|---|---|
| 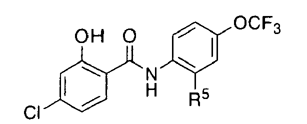 | | |

| Compound | R⁵ | MS |
|---|---|---|
| 80 | 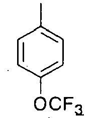 | 492(M+H)⁺ |
| 81 | 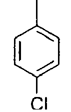 | 442(M+H)⁺ |
| 82 | 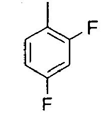 | 444(M+H)⁺ |
| 83 | 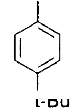 | 464(M+H)⁺ |
| 84 | 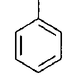 | 408(M+H)⁺ |
| 85 | 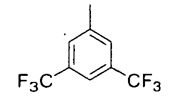 | 544(M+H)⁺ |
| 86 | 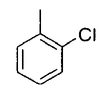 | 442(M+H)⁺ |

**[Table 12]**

| | | |
|---|---|---|
| 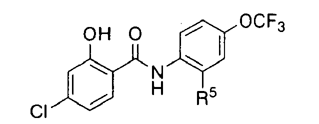 | | |

| Compound | R⁵ | MS |
|---|---|---|
| 87 | 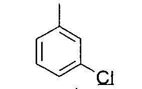 | 442(M+H)⁺ |
| 88 | 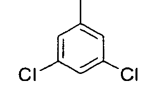 | 477(M+H)⁺ |
| 89 | 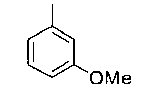 | 438(M+H)⁺ |
| 90 | 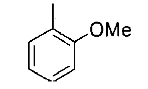 | 438(M+H)⁺ |
| 91 | 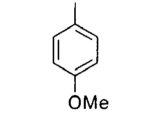 | 438(M+H)⁺ |
| 92 | 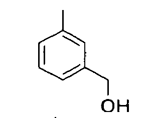 | 460(M+Na)⁺ |
| 93 | 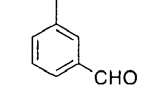 | 458(M+Na)⁺ |
| 94 | 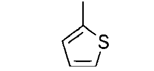 | 414(M+H)⁺ |
| 95 | 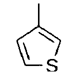 | 414(M+H)⁺ |
| 96 | 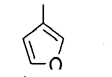 | 398(M+H)⁺ |
| 97 | 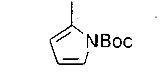 | 519 (M+Na)⁺ |
| 98 | 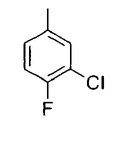 | 460(M+H)⁺ |

**[Table 13]**

| | | |
|---|---|---|
| 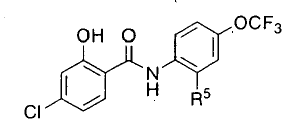 | | |

| Compound | R⁵ | MS |
|---|---|---|
| 99 | 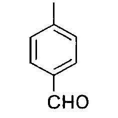 | 436(M+H)⁺ |
| 100 | 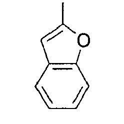 | 448(M+H)⁺ |

**[Table 14]**

| | | |
|---|---|---|
| 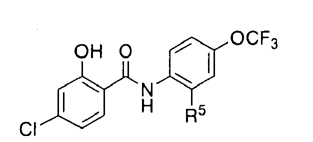 | | |

| Compound | R⁵ | MS |
|---|---|---|
| 101 | 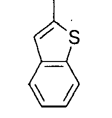 | 486(M+Na)⁺ |
| 102 | 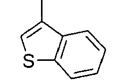 | 464(M+H)⁺ |
| 103 | 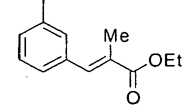 | 520(M+H)⁺ 542(M+Na)⁺ |
| 104 | 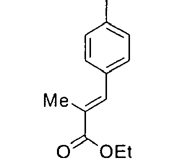 | 520(M+H)⁺ 542(M+Na)⁺ |

**[Table 15]**

| | | |
|---|---|---|
| 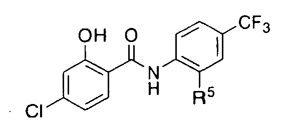 | | |

| Compound | R5 | MS |
|---|---|---|
| 105 | 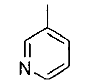 | 393(M+H)⁺ |
| 106 | 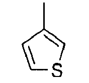 | 398(M+H)⁺ |
| 107 | 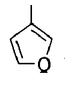 | 382(M+H)⁺ |

**[Table 16]**

| | | | |
|---|---|---|---|
| 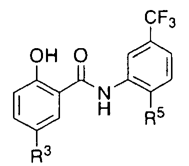 | | | |

| Compound | R³ | R⁵ | MS |
|---|---|---|---|
| 108 | Cl | 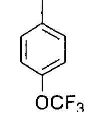 | 474(M-H)⁻ 476(M+H)⁺ |
| 109 | 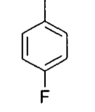 | 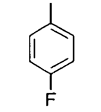 | 468(M-H)⁻ 470(M+H)⁺ |

**[Table 17]**

| | | | |
|---|---|---|---|
| 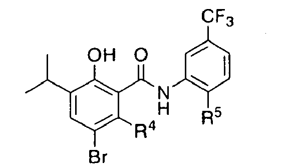 | | | |

| Compound | R⁴ | R⁵ | MS |
|---|---|---|---|
| 110 | H | 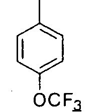 | 562(M+H)⁺ |
| 111 | Me | 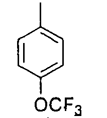 | 576(M+H)⁺ |
| 112 | H | 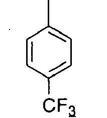 | 544(M-H)⁻ 546(M+H)⁺ |
| 113 | H | 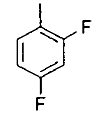 | 512(M-H)⁻ |

**[Table 18]**

| | | |
|---|---|---|
| 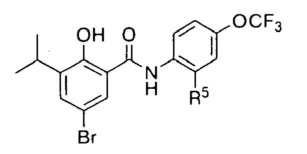 | | |

| Compound | R⁵ | MS |
|---|---|---|
| 114 | 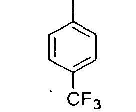 | 562(M+H)⁺ |
| 115 | 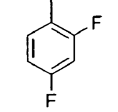 | 530(M+H)⁺ |
| 116 | 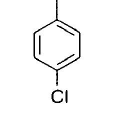 | 528(M+H)⁺ |

**[Table 19]**

| | | |
|---|---|---|
| 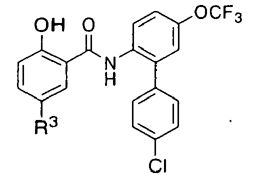 | | |

| Compound | R³ | MS |
|---|---|---|
| 117 | I | 534(M+H)⁺ |
| 118 | 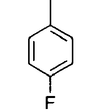 | 502(M+H)⁺ |
| 119 | 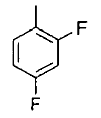 | 520(M+H)⁺ |
| 120 | 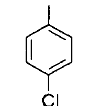 | 518(M+H)⁺ |
| 121 | 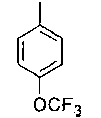 | 568(M+H)⁺ |
| 122 | 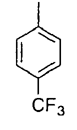 | 552(M+H)⁺ |

**[Table 20]**

| | | |
|---|---|---|
| 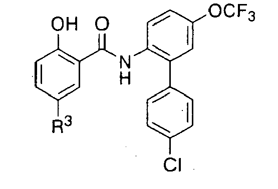 | | |

| Compound | R³ | MS |
|---|---|---|
| 123 | 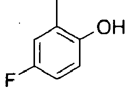 | 518(M+H)⁺ |
| 124 | 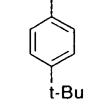 | 540(M+H)⁺ |
| 125 | 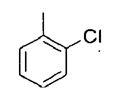 | 516(M-H)⁻ 518(M+H)⁺ |
| 126 | 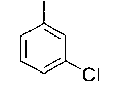 | 516(M-H)⁻ 518(M+H)⁺ |

**[Table 21]**

| | | | |
|---|---|---|---|
| 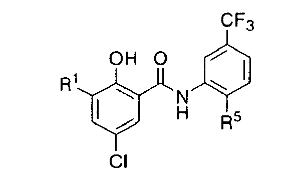 | | | |

| Compound | R¹ | R⁵ | MS |
|---|---|---|---|
| 127 | i-Pr | 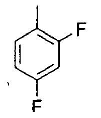 | 468(M-H)⁻ 470(M+H)⁺ |
| 128 | i-Pr | 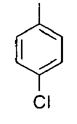 | 468(M+H)⁺ |
| 129 | i-Pr | 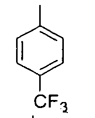 | 502(M+H)⁺ |
| 130 | i-Pr | 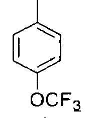 | 518(M+H)⁺ |
| 131 | i-Pr | 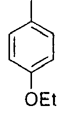 | 478(M+H)⁺ |

**[Table 22]**

| | | | |
|---|---|---|---|
| 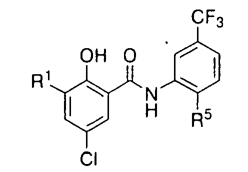 | | | |

| Compound | R¹ | R⁵ | MS |
|---|---|---|---|
| 132 | Me | 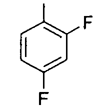 | 442(M+H)⁺ |
| 133 | Cl | 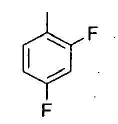 | 462(M+H)⁺ |
| 134 | OMe | 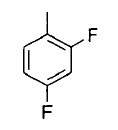 | 458(M+H)⁺ |
| 135 | Et | 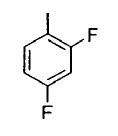 | 456(M+H)⁺ |
| 136 | Et | 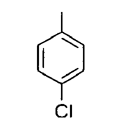 | 454(M+H)⁺ |
| 137 | NO₂ | 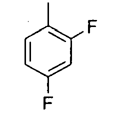 | 471(M-H)⁻ 473(M+H)⁺ |
| 138 | NO₂ | 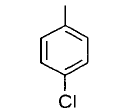 | 469(M-H)⁻ 471(M+H)⁺ |
| 139 | Pr | 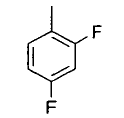 | 468(M-H)⁻ 470(M+H)⁺ |
| 140 | Pr | 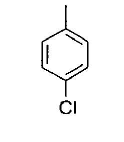 | 466(M-H)⁻ 468(M+H)⁺ |

**[Table 23]**

| | | | |
|---|---|---|---|
| 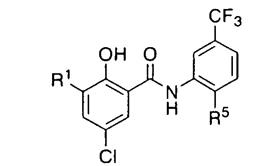 | | | |

| Compound | R¹ | R⁵ | MS |
|---|---|---|---|
| 141 | H | 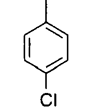 | 426(M+H)⁺ |
| 142 | Me | 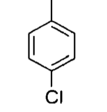 | 440(M+H)⁺ |

**[Table 24]**

| | | | |
|---|---|---|---|
| 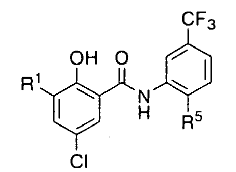 | | | |

| Compound | R¹ | R⁵ | MS |
|---|---|---|---|
| 143 | H | 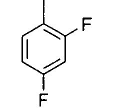 | 428(M+H)⁺ |
| 144 | F | 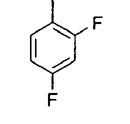 | 446(M+H)⁺ |
| 145 | F | 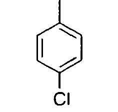 | 444(M+H)⁺ |

**[Table 25]**

| | | |
|---|---|---|
| 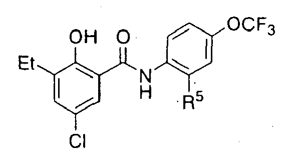 | | |

| Compound | R⁵ | MS |
|---|---|---|
| 146 | 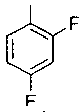 | 472(M+H)⁺ |
| 147 | 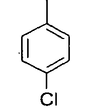 | 470(M+H)⁺ |

**[Table 26]**

| | | |
|---|---|---|
| 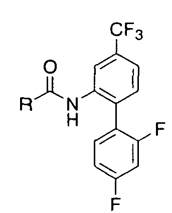 | | |

| Compound | R | MS |
|---|---|---|
| 148 | 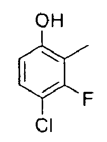 | 446(M)⁺ |
| 149 | 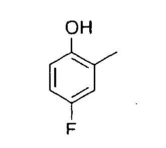 | 412(M+H)⁺ |
| 150 | 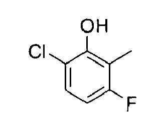 | 446(M+H)⁺ |
| 151 | 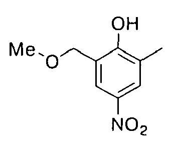 | 481(M+H)⁺ |

**[Table 27]**

| | | | |
|---|---|---|---|
| 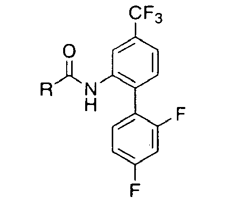 | | | |

| Compound | R | MS | Melting point |
|---|---|---|---|
| 3-0 | 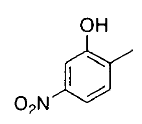 | 439(M+H)⁺ | |
| 3-1 | 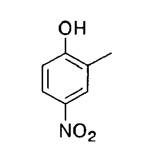 | 439(M+H)⁺ | |
| 3-2 | 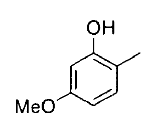 | | 156.0 |
| 3-3 | 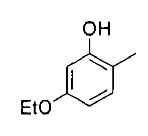 | | 165.0 |
| 3-4 | 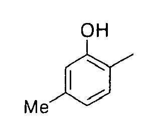 | | 188.2 |
| 3-5 | 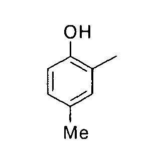 | | 188.0 |
| 3-6 | 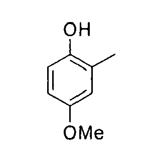 | | 175 |

**[Table 28]**

| | | | |
|---|---|---|---|
| 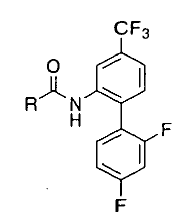 | | | |

| Compound | R | MS | Melting point |
|---|---|---|---|
| 3-7 | 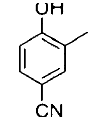 | 419(M+H)⁺ | 211.3 |
| 3-8 | 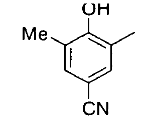 | 433(M+H)⁺ | |
| 3-9 | 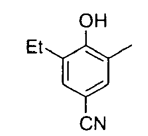 | 477(M+H)⁺ | |
| 3-10 | 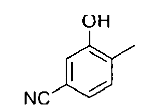 | 419(M+H)⁺ | 224.6 |
| 3-11 | 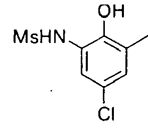 | | NMR(CDCl₃): 3.03 (3H, s), 12.34 (1H, s) |
| 3-12 | 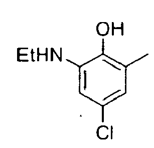 | 470(M+H)⁺ | |
| 3-13 | 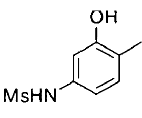 | | NMR(DMSO-d₆): 3.05 (3H, s), 11.33 (1H, s) |
| 3-14 | 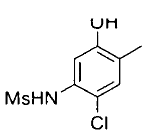 | 512(M+H)⁺ | |
| 3-15 | 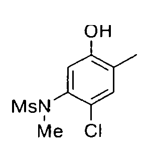 | 535(M+H)⁺ | |

**[Table 29]**

| | | |
|---|---|---|
| 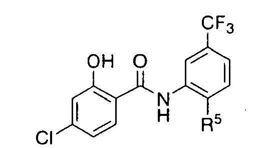 | | |

| Compound | R⁵ | MS |
|---|---|---|
| 3-16 | 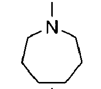 | 413(M+H)⁺ |
| 3-17 | 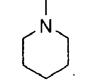 | 399(M+H)⁺ |
| 3-18 | 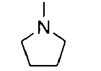 | 385(M+H)⁺ |
| 3-19 | 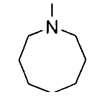 | 427(M+H)⁺ |
| 3-20 | 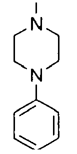 | 476(M+H)⁺ |
| 3-21 | 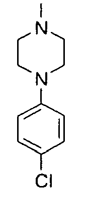 | 511(M+H)⁺ |
| 3-22 | 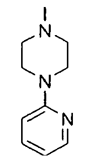 | 477(M+H)⁺ |
| 3-23 | 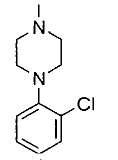 | 511(M+H)⁺ |
| 3-24 | 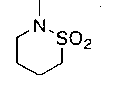 | 449(M+H)⁺ |

**[Table 30]**

| | | |
|---|---|---|
| 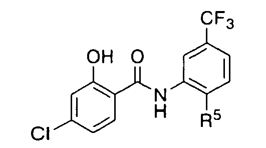 | | |

| Compound | R⁵ | MS |
|---|---|---|
| 3-25 | 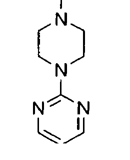 | 478(M+H)⁺ |

**[Table 31]**

| | | |
|---|---|---|
| 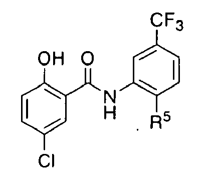 | | |

| Compound | R⁵ | MS |
|---|---|---|
| 3-26 | 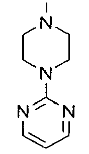 | 478(M+H)⁺ |
| 3-27 | 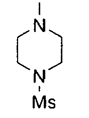 | 478(M+H)⁺ |
| 3-28 | 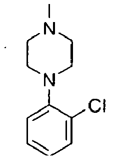 | 511 (M+H)⁺ |

**[Table 32]**

| | | |
|---|---|---|
| 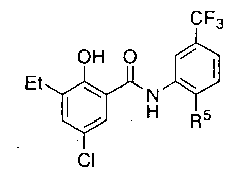 | | |

| Compound | R⁵ | MS |
|---|---|---|
| 3-29 | 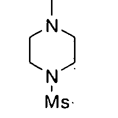 | 506(M+H)⁺ |
| 3-30 | 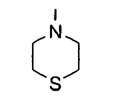 | 445(M+H)⁺ |
| 3-31 | 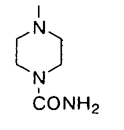 | 470(M+H)⁺ |
| 3-32 | 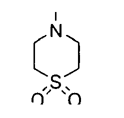 | 477(M+H)⁺ |

**[Table 33]**

| | | |
|---|---|---|
| 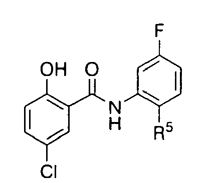 | | |

| Compound | R⁵ | MS |
|---|---|---|
| 3-33 | 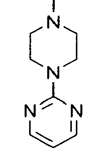 | 428(M+H)⁺ |
| 3-34 | 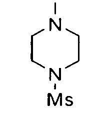 | 496(M+H)⁺ |

**[Table 34]**

| | | |
|---|---|---|
| 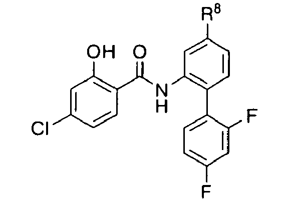 | | |

| Compound | R⁸ | MS |
|---|---|---|
| 3-35 | F | 378(M+H)⁺ |
| 3-36 | Me | 374(M+H)⁺ |
| 3-37 | OMe | 390(M+H)⁺ |
| 3-38 | Cl | 394(M+H)⁺ |
| 3-39 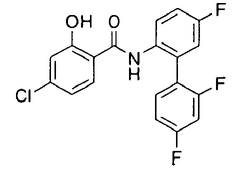 | | 378(M+H)⁺ |

**[Table 35]**

| | | |
|---|---|---|
| 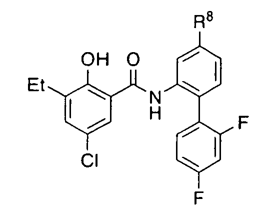 | | |

| Compound | R⁸ | MS |
|---|---|---|
| 3-41 | NO₂ | 433(M+H)⁺ |
| 3-42 | F | 406(M+H)⁺ |

**[Table 36]**

| | | |
|---|---|---|
| 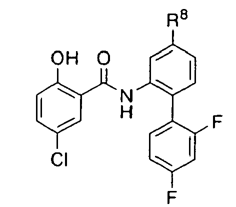 | | |

| Compound | R⁸ | MS |
|---|---|---|
| 3-43 | NO₂ | 405(M+H)⁺ |
| 3-44 | F | 378(M+H)⁺ |

**[Table 37]**

| | | | |
|---|---|---|---|
| 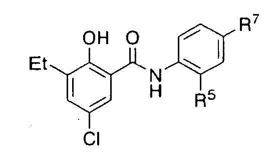 | | | |

| Compound | R⁵ | R⁷ | MS |
|---|---|---|---|
| 3-45 | 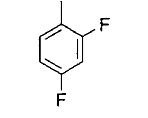 | F | 406(M+H)⁺ |
| 3-46 | 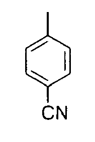 | CF₃ | 445(M+H)⁺ |
| 3-47 | 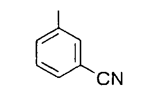 | CF₃ | 445(M+H)⁺ |
| 3-48 | 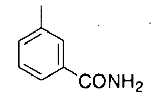 | CF₃ | 463(M+H)⁺ |

### Example 4

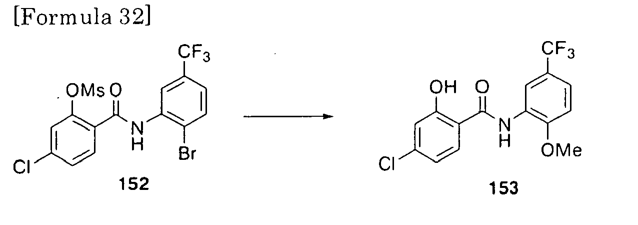

### Methyl ether derivative (153):

Bromide (152) (200 mg, 0.423 mmol) was dissolved in dimethylformamide (4 ml). CuI (8.1 mg, 0.1 eq) and NaOMe in methanol (5.2 M, 6eq) were added. After being reacted at 90 °C for 1 hour, the reaction mixture was added to ice water (30 ml) and 2 N hydrochloric acid (1.2 ml), extracted twice with acetic acid ethyl ester (30 ml), washed three times with concentrated brine (30 ml) and dried over anhydrous sodium sulfate. The residue obtained by evaporating the solvent under reduced pressure was purified by silica gel column chromatography (n-hexane-acetic acid ethyl ester =2-1) and the following recrystallization from n-hexane to give a desired product (112 mg, 77 %). NMR (CDCl3) δppm: 4.03 (3H, s, OMe), 6.91 (1H, d_{AB}, J=6Hz, Ar-H), 7.02 (2H, m, Ar-H), 7.42 (2H, t, J=6Hz, Ar-H), 8.63 (1H, s, Ar-H), 8.72 (1H, s, NH), 12.10 (1H, s, OH).
IRνₘₐₓ (KBr):3422, 1646, 1596, 1552, 1496, 1447, 1265, 1121cm⁻¹.
MS: 344(M-H)⁻ 346(M+H)⁺.

As shown in Table 38 and 39, ether derivatives (154 to 158), thioether, sulfoxide, which is prepared by the oxidation of corresponding thioethers, sulfone derivatives (159 to 165), and an amine derivative (166) were synthesized in a similar way as above.

**[Table 38]**

| | | | | |
|---|---|---|---|---|
| 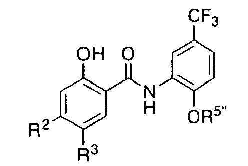 | | | | |

| Compound | R² | R³ | R^{5"} | MS |
|---|---|---|---|---|
| 154 | Cl | H | t-Bu | 386(M-H)⁻ |
| 155 | H | Cl | Me | 344(M-H)⁻ 346(M+H)⁺ |
| 156 | Cl | H | 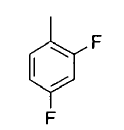 | 444(M+H)⁺ |
| 157 | Cl | H | 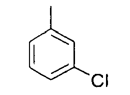 | 442(M+H)⁺ |
| 158 | Cl | H | Ph | 408(M+H)⁺ |

**[Table 39]**

| | | | |
|---|---|---|---|
| 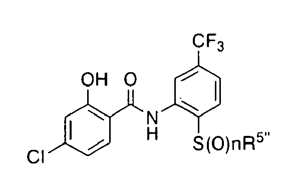 | | | |

| Compound | R^{5''} | n | MS |
|---|---|---|---|
| 159 | 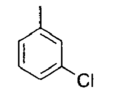 | 0 | 458(M+H)⁺ |
| 160 | 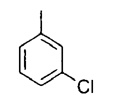 | 1 | 496(M+Na)⁺ |
| 161 | Me | 0 | 362(M+H)⁺ |
| 162 | 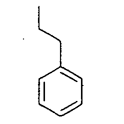 | 0 | 438(M+Na)⁺ |
| 163 | 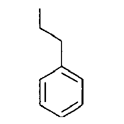 | 1 | 490(M+Na)⁺ |
| 164 | 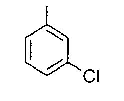 | 2 | 512(M+Na)⁺ |
| 165 | 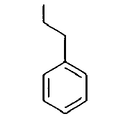 | 2 | 506(M+Na)⁺ |
| 166 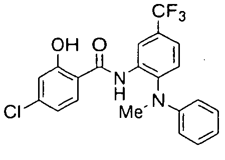 | | | 421(M+H)⁺ |

### Example 5

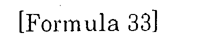

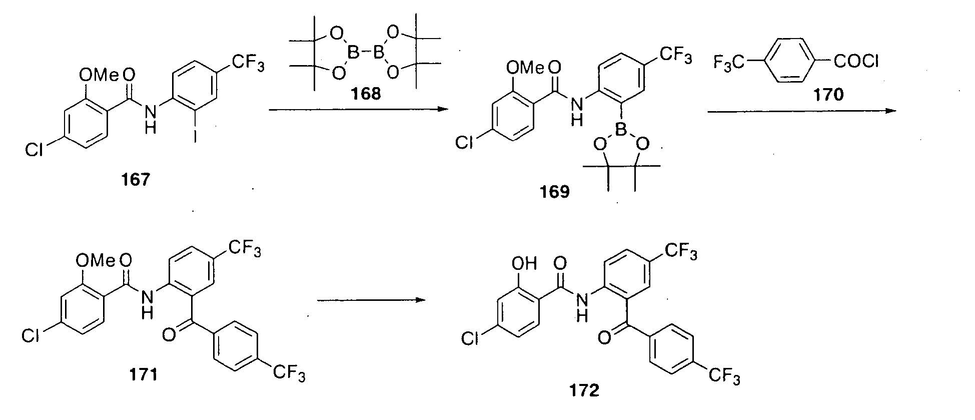

### Boronic ester (169):

Iodide (167) (1.61 g, 3.534 mmol) was dissolved in dimethylsulfoxide (32 ml). Bis(pinacolato)diboron (168) (0.99 g, 1.1 eq), PdCl₂(dppf) (0.29 g, 0.1 eq) and potassium acetate (1.04 g, 3 eq) were added, and the mixture was reacted at 80 °C for 3 hours under N₂ atmosphere. The reaction solution was added in ice water (130 ml), extracted twice with acetic acid ethyl ester (130 ml), washed three times with water (130 ml) and dried over anhydrous sodium sulfate. The crystal obtained by evaporating the solvent under reduced pressure was washed with acetic acid ethyl ester to give a desired product (169) (1.07 g, 67 %). The residue obtained by evaporating the washing solution under reduced pressure was purified by silica gel column chromatography (n-hexane-acetic acid ethyl ester =1-1) and following recrystallization from n-hexane to give a desired product (169) (305 mg, 18 %) (Total is 85 %).
NMR(CDCl₃)δppm:1.35 (12H, s, CH₃), 4.04 (3H,s, OMe), 7.05 (1H, s, Ar-H), 7.1 to 7.26 (3H, m, Ar-H), 7.58 (1H, s, Ar-H), 7.78 (1H, d_{AB}, J=6Hz Ar-H), 8.09 (1H, d_{AB}, J=6Hz Ar-H), 10.40 (1H, s, NH).

### Ketone derivative (171):

Boronic ester (169) (447 mg, 1.097 mmol) was dissolved in acetone (10 ml) and toluene (10 ml). Acid chloride (170) (0.326 ml, 2 eq), PdCl₂ (dppf) (90 mg, 0.leq) and potassium carbonate (454 mg, 3 eq) were added, and the mixture was reacted at 100 °C for 4 hours under N₂ atmosphere. The reaction solution was added to ice water (100 ml), extracted twice with acetic acid ethyl ester (100 ml), washed twice with water (100 ml) and dried over anhydrous sodium sulfate. The residue obtained by evaporating the solvent under reduced pressure was purified by silica gel column chromatography (toluene) and the following recrystallization from n-hexane to give a desired product (171) (360 mg, 65%).
mp: 126 to 127°C
NMR( CDCl₃)δppm: 4.18 (3H, s, OMe), 7.04 (1H, s, Ar-H), 7.10 (1H, d_{AB}, J=6Hz Ar-H), 7.70 (1H, s, Ar-H), 7.81 to 7.91 (5H, m, Ar-H), 8.20 (1H, d_{AB}, J=6Hz Ar-H), 8.98 (1H, d_{AB}, J=6HzAr-H), 11.98 (1H, s, NH).
MS: 502 (M+H)⁺

### Compound (172):

The above ketone derivative (171) (301 mg, 0.6 mmol) was dissolved in methylene chloride (10ml). 1M/L BBr₃ in methylene chloride (1M solution 0.9ml, 1.5eq) was added, and the mixture was stirred at room temperature for 30 minutes. To the reaction solution, were added ice water (20 ml) and saturated sodium bicarbonate water (15m), and washed. After washing with water, the solution was dried over anhydrous sodium sulfate. The residue obtained by evaporating the solvent under reduced pressure was purified by silica gel column chromatography (toluene) and the following recrystallization from n-hexane to give a desired product (172) (231 mg, 80%).
mp: 189 to 190 °C.
NMR(CDCl₃)δppm : 6.99 (1H, d_{AB}, J=6Hz Ar-H), 7.07 (1H,s, Ar-H), 7.57 (1H, d_{AB}, J=6Hz Ar-H), 7.87 (1H, s, Ar-H), 7.96 (1H, d_{AB}, J=6Hz Ar-H), 8.97 (1H, d_{AB}, J=6Hz Ar-H), 12.08 (1H, s, NH), 12.25 (1H, s, OH).
MS: 488 (M+H)⁺.

In a similar way as above, compounds (173 to 189 and 5-1) in Table 40 to 42 were synthesized. Furthermore, alcohol derivatives (5-2 and 5-3) were synthesized by NaBH₄ reduction.

**[Table 40]**

| | | |
|---|---|---|
| 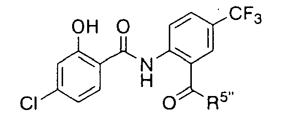 | | |

| Compound | R^{5"} | MS |
|---|---|---|
| 173 | 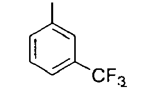 | 486(M-H)⁻ 488(M+H)⁺ |
| 174 | 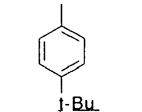 | 474(M-H)⁻ 476(M+H)⁺ |
| 175 | 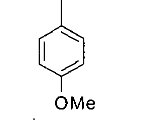 | 450(M+H)⁺ |
| 176 | 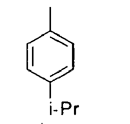 | 462(M+H)⁺ |
| 177 | 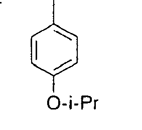 | 478(M+H)⁺ |
| 178 | 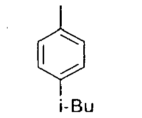 | 476(M+H)⁺ |
| 179 | 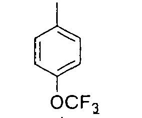 | 504(M+H)⁺ |
| 180 | 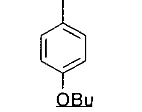 | 492(M+H)⁺ |
| 5-1 | 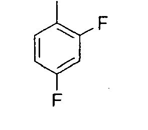 | 456(M+H)⁺ |

**[Table 41]**

| | | |
|---|---|---|
| 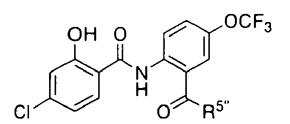 | | |

| Compound | R^{5"} | MS |
|---|---|---|
| 181 | 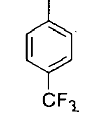 | 504(M+H)⁺ |
| 182 | 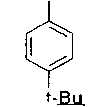 | 492(M+H)⁺ |
| 183 | 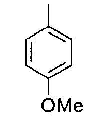 | 466(M+H)⁺ |
| 184 | 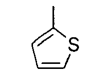 | 440(M-H)⁻ 442(M+H)⁺ |
| 185 | 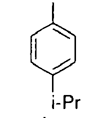 | 478(M+H)⁺ |
| 186 | 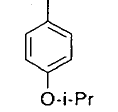 | 494(M+H)⁺ |
| 187 | 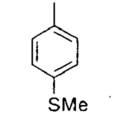 | 482(M+H)⁺ |
| 188 | 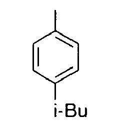 | 492(M+H)⁺ |
| 189 | 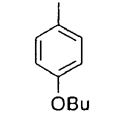 | 508(M+H)⁺ |

**[Table 42]**

| | | |
|---|---|---|
| 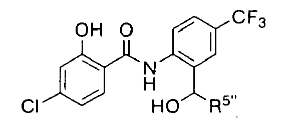 | | |

| Compound | R^{5"} | MS |
|---|---|---|
| 5-2 | 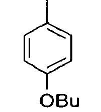 | 493(M)⁺ |
| 5-3 | 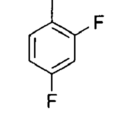 | 458(M+H)⁺ |

### Example 6

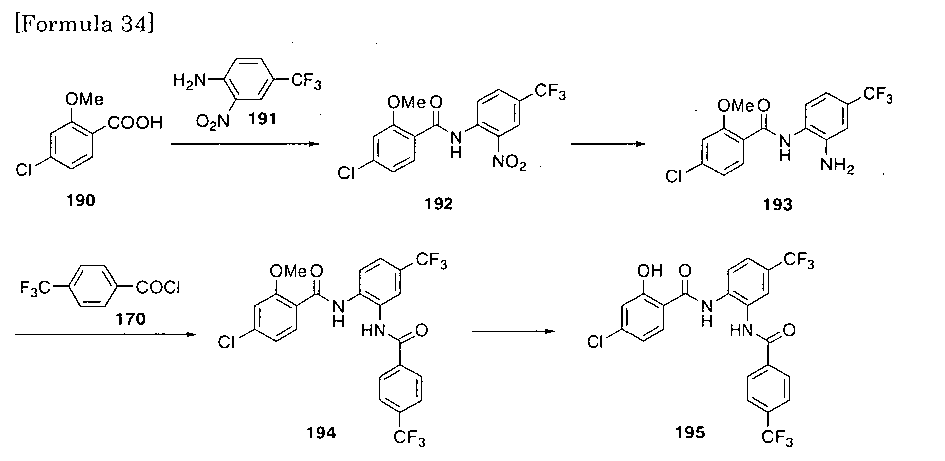

### Amide derivative (192):

3-methoxysalicylic acid (190) (4.4 g, 23.6 mmol) and 4-amino-3-nitrobenzotrifluoride (191) (4.86 g, 23.6 mmol) were added to chlorobenzene (44 ml). PCl₃(1.03 ml, 0.5eq) was added thereto and the mixture was heated at 150 °C for 1 hour. The residue obtained by evaporating the solvent under reduced pressure was purified by silica gel column chromatography (toluene) and the following recrystallization from n-hexane to give a desired product (192) (7.47 g, 85 %).
NMR(CDCl₃)δppm:4.16(3H, s, OMe), 7.07 (1H, s, Ar-H), 7.13 (1H, d_{AB}, J=6Hz Ar-H), 7.89 (1H, d_{AB}, J=6Hz Ar-H), 8.21 (1H, d_{AB}, J=6Hz Ar-H), 8.50 (1H, s, Ar-H), 9.21 (1H, d_{AB}, J=6Hz Ar-H), 12.27 (1H, s, NH).

### Amine (193):

The above nitro derivative (192) (7.46 g, 19.9 mmol) was added to MeOH (150 ml). 5% Pd-C (1.5 g) was added, and hydrogen gas (1.58 L, 3eq) was introduced to the suspension. After catalyst was removed by filtration, the residue obtained by evaporating the solvent under reduced pressure was purified by silica gel column chromatography (acetic acid ethyl ester) and the following recrystallization from n-hexane to give a desired product (193) (1.6 g, 30 %).
NMR (CDC13) δppm: 1.80 (2H, broad, NH₂), 4.07 (3H, s, OMe), 7.05 - 87.15 (4H, m, Ar-H), 7.64 (1H, d_{AB}, J=6Hz Ar-H), 8.23 (1H, d_{AB}, J=6Hz Ar-H), 9.60 (1H, s, NH).
MS: 345(M+H)⁺.

### Amide derivative (194):

The above amine (193) (300 mg, 0.87 mmol) was dissolved in tetrahydrofuran (8 ml). After adding triethylamine (0.15 ml, 1.2 eq) at room temperature, acid chloride (170) (0.142 ml, 1.1 eq) was added, and the mixture was reacted at room temperature for 30 minutes. The reaction solution was added in ice water (40 ml), extracted twice with acetic acid ethyl ester (40 ml), washed twice with water (40 ml) and dried over anhydrous sodium sulfate. The residue obtained by evaporating the solvent under reduced pressure (194) (1 g) was used in the next step directly.

### Compound (195):

The above amide derivative (194) (1 g, 0.87 mmol) was dissolved in methylene chloride (10 ml). BBr₃/CH₂Cl₂ solution (1M solution 1.31 ml, 1.5eq) was added, and the mixture was stirred at room temperature for 30 minutes. To the reaction solution, were added ice water (20 ml) and saturated sodium bicarbonate (20 ml). After being washing with water, the organic layer was dried over anhydrous sodium sulfate. The residue obtained by evaporating the solvent under reduced pressure was purified with silica gel column chromatography (n-hexane-acetic acid ethyl ester =2-1) and the following recrystallization from n-hexane to give a desired product (195) (407 mg, 93 %).
NMR (CDCl₃+ CD₃OD) δppm: 6.95 (1H, d_{AB}, J=6Hz Ar-H), 6.99 (1H, s, Ar-H), 7.33 (1H, s, Ar-H), 7.57 (1H, d_{AB}, J=6Hz Ar-H), 7.72 to 7.9 (5H, m, Ar-H), 8.09 (1H, d_{AB}, J=6Hz Ar-H).
MS: 503 (M+H)⁺.

Compounds (196 to 204) in Table 43 were synthesized in a similar way as above.

**[Table 43]**

| | | |
|---|---|---|
| 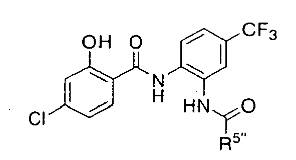 | | |

| Compound | R^{5"} | MS |
|---|---|---|
| 196 | 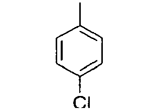 | 469(M+H)⁺ |
| 197 | 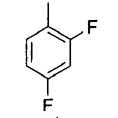 | 471(M+H)⁺ |
| 198 | 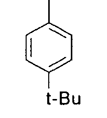 | 489(M-H)⁻ 491(M+H)⁺ |
| 199 | 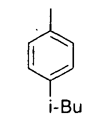 | 489(M-H)⁻ 491(M+H)⁺ |
| 200 | 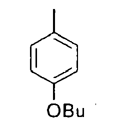 | 505(M-H)⁻ 507(M+H)⁺ |
| 201 | 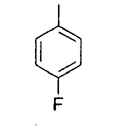 | 453(M+H)⁺ |
| 202 | 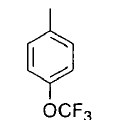 | 519(M+H)⁺ |
| 203 | 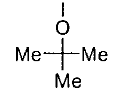 | 453 (M+Na)⁺ |
| 204 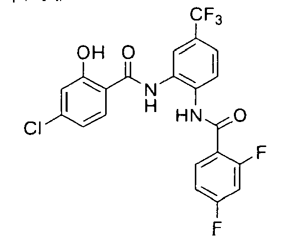 | | 493 (M+Na)⁺ |

### Example 7

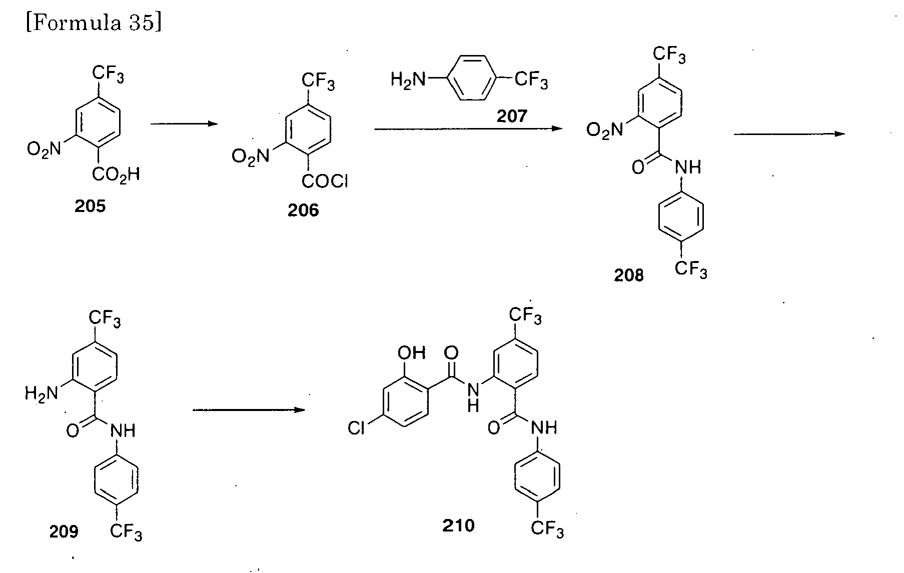

### Nitro compound (208):

To carboxylic acid (205) (500 mg, 2.13 mmol), were added toluene (10 ml) and oxalyl chloride (0.37 ml, 2 eq). The mixture was reacted at 110°C for 0.5 hour. The residue (206) obtained by evaporating the solvent under reduced pressure was dissolved in tetrahydrofuran (10 ml). Aniline derivative (207) (274 mg, 0.8eq) and triethylamine (0.36 ml, 1.2eq) were added, and the mixture was reacted at room temperature for 1 hour. The reaction solution was added to ice water (30 ml), extracted twice with acetic acid ethyl ester (30 ml), washed twice with water (30 ml) and dried over anhydrous sodium sulfate. The residue obtained by evaporating the solvent under reduced pressure (208) (560 mg) was used in the next step without purification.

### Aniline (209):

The above nitro derivative (208) (554 mg, 1.70 mmol) was added to MeOH (10 ml) and dissolved. 5% Pd-C (0.4 g) was added, and hydrogen gas (104 ml) was introduced. After the catalyst was removed by filtration, the residue obtained by evaporating the solvent under reduced pressure was purified with silica gel column chromatography (n-hexane-acetic acid ethyl ester =9-1) and recrystallized from n-hexane to give a desired compound (209) 288 mg (49 %).
NMR (CDCl₃) δppm: 5.30 (2H, broad, NH₂), 6.98 (2H, d_{AB}, J=3Hz Ar-H), 7.28 (1H, s, Ar-H), 7.58 - 7.75 (5H, m, Ar-H), 7.88 (1H, s, Ar-H).

### Compound (210):

4-chlorosalicylate (59) (130 mg, 0.684 mmol) and aniline (209) (238 mg, 0.684 mmol) were added to cholorobenzene (5 ml). PCl₃ (0.03 ml, 0.5 eq) was added thereto and the mixture was heated at 150 °C for 2 hour. The residue obtained by evaporating the solvent under reduced pressure was purified with silica gel column chromatography (n-hexane-acetic acid ethyl ester =4-1) and recrystallized from n-hexane to give a desired compound (210) (227 mg, 50%).
mp: 235 to 236 °C.
NMR(CDCl₃)δppm: 6.96 (1H, d_{AB}, J=6Hz Ar-H), 7.04 (1H, s, Ar-H), 7.48 (1H, d_{AB}, J=6Hz, Ar-H), 7.64 - 7.77 (5H, m, J=6Hz Ar-H), 8.18 (1H, s, Ar-H), 9.04 (1H, s, Ar-H), 12.00 (1H, s, NH), 12.14 (1H, s, OH).
MS: 503 (M+H)⁺.

Amide, sulfonamide and sulfonate (211 to 221) in Table 44 and 45 were synthesized in a similar way as above.

**[Table 44]**

| | | |
|---|---|---|
| 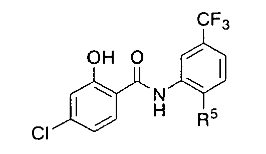 | | |

| Compound | R⁵ | MS |
|---|---|---|
| 211 | 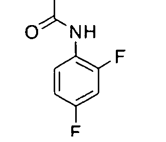 | 471(M+H)⁺ |
| 212 | 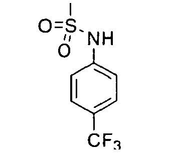 | 537(M-H)⁻ 539(M+H)⁺ |
| 213 | 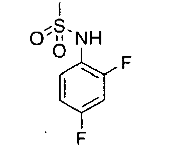 | 505(M-H)⁻ 507(M+H)⁺ |
| 214 | 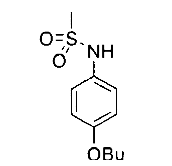 | 541(M-H)⁻ 542(M+H)⁺ |
| 215 | 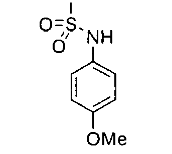 | 499(M-H)⁻ 500(M)⁺ 501(M+H)⁺ |
| 216 | 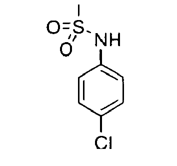 | 503(M-H)⁻ 505(M+H)⁺ |

**[Table 45]**

| | | |
|---|---|---|
| 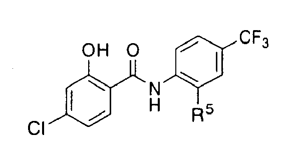 | | |

| Compound | R⁵ | MS |
|---|---|---|
| 217 | 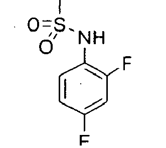 | 507(M+H)⁺ |
| 218 | 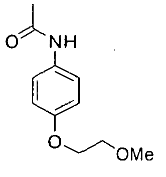 | 494(M+H)⁺ |
| 219 | 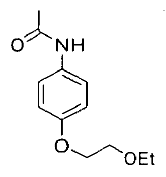 | 508M+H)⁺ |
| 220 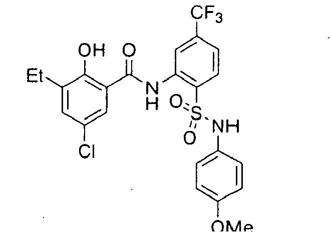 | | 527(M-H)⁻ 428(M)⁺ |
| 221 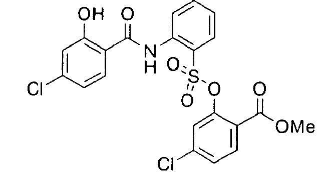 | | 518(M+Na)⁺ |

### Example 8

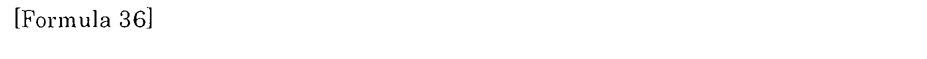

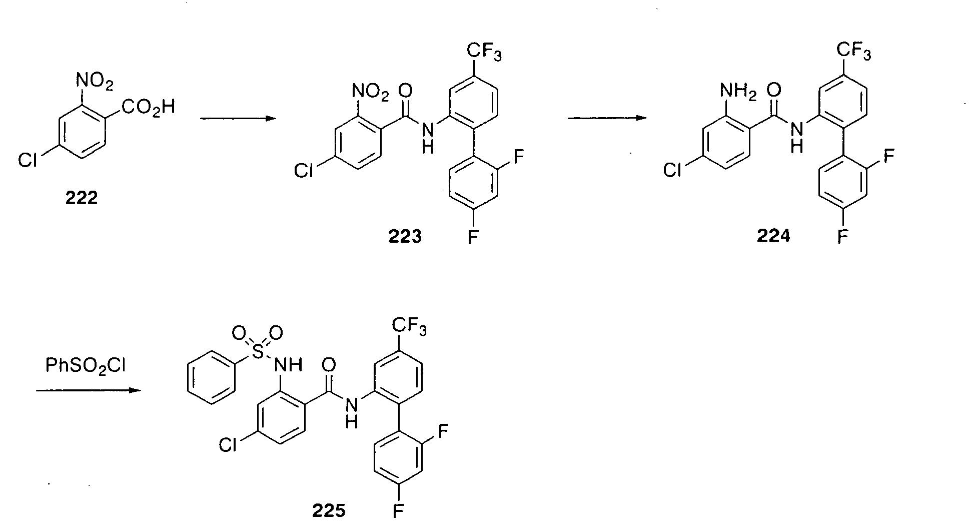

### Nitro derivative (223):

To a solution of carboxylic acid (222)(440 mg, 2.19 mmol) in methylene chloride (5 ml), were added successively oxalyl chloride (0.287 ml, 3.29 mmol) and N,N-dimethylformamide (0.01 ml, 0.13 mmol) at room temperature. The mixture was stirred for 3 hours, and then concentrated under reduced pressure. The residue was dissolved in ethyl acetate (5 ml) and the solution was added dropwise to a mixture of the above amine in ethyl acetate (5 ml) and saturated sodium hydrogen carbonate solution with ice-cooling. After stirring the reaction solution for 2 hours with ice-cooling and for another 2 hours at room temperature, ethyl acetate was added. The organic layer was washed successively with water and brine and dried over anhydrous sodium sulfate. The residue obtained by evaporating the solvent under reduced pressure was recrystallized from diisopropylethyl ether /ethyl acetate to give the above compound (223) 682 mg (The yield is 82%) as a white powder.
¹H NMR (CDCl₃) δppm: 6.96- 7.10 (2H, m), 7.23 (1H, br s), 7.30-7.46 (3H, m), 7.57 (1H, d, J = 7.8 Hz), 7.64-7.70 (1H, m), 8.07 (1H, d, J = 2.1 Hz), 8.58 (1H, s).
IR (KBr)νₘₐₓ: 3215, 1647, 1541, 1334, 1136, 1102 cm⁻¹.
MS: m/z 457 7 (MH⁺).

### Amine (224):

To a solution of the nitro intermediate prepared above (223) (500 mg, 1.10 mmol) in ethanol, was added SnCl₂ -2H₂O (1.48 g, 6.56 mmol), and the mixture was stirred at 70 °C for 5 hours. The reaction mixture was cooled to room temperature, and 2 N sodium hydroxide solution (6.6 ml) was added. The mixture was diluted with water and extracted with ethyl acetate. The extract was washed successively with saturated sodium hydrogen carbonate solution and brine, and dried over anhydrous sodium sulfate. To the residue obtained by evaporating under reduced pressure, was added n-hexane, and the mixture was suspended. The crystal was collected by filtration to give amine (224) (209 mg, 46% yield) as a white powder.
¹H NMR (CDCl₃)δppm : 5.60 (2H, br), 6.57 (1H, dd, J = 2.1, 8.7 Hz), 6.68 (1H, d, J = 1.8 Hz), 6.96 (1H, d, J = 8.7 Hz), 7.00- 7.11 (2H, m), 7.30-7.44 (2H, m), 7.47-7.54 (1H, m), 7.62 (1H, br s), 8.59 (1H, s).

### Sulfonamide (225):

To a solution of the above amine intermediate (224) (75 mg, 0.176 mmol) in pyridine (1 ml), was added benzenesulfonyl chloride (0.037 ml, 0.290 mmol) with ice-cooling, and the mixture was stirred overnight at room temperature. After being diluted with ethyl acetate, the solution was washed successively with 2 N hydrochloric acid, saturated sodium hydrogen carbonate solution and brine, and dried over anhydrous sodium sulphate. To the residue obtained by evaporating the solvent under reduced pressure, was added diisopropylethyl ether, and the mixture was suspended. The crystal was collected by filtration to give sulfonamide (225) (59 mg, 59% yield) as a white powder.
mp: 200-201 °C
¹H NMR (CDCl₃) δppm: 6.94-7.11 (4H, m), 7.26-7.36 (1H, m), 7.38- 7.4 7 (1H, m), 7.74 (1H, br s), 7.82-7.88 (2H, m), 8.36 (1H, br s), 10.53 (1H, s).
IR (KBr)νₘₐₓ: 3246, 1624, 1492, 1330, 1172 cm⁻¹.
MS: m/z 567 (MH+).

Compounds (226 to 243) in Table 46 to 49 were synthesized in a similar way as above.

**[Table 46]**

| | | | |
|---|---|---|---|
| 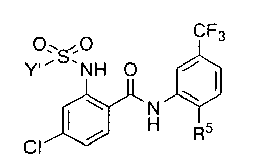 | | | |

| Compound | Y' | R⁵ | MS |
|---|---|---|---|
| 226 | Me | 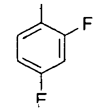 | 505(M+H)⁺ |
| 227 | Ph | 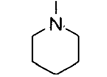 | 538(M+H)⁺ |
| 228 | Me | 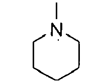 | 476(M+H)⁺ |
| 229 | Et | 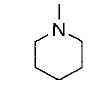 | 490(M+H)⁺ |

**[Table 47]**

| | | | |
|---|---|---|---|
| 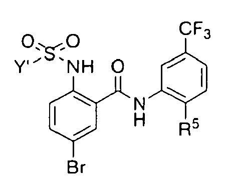 | | | |

| Compound | Y' | R⁵ | MS |
|---|---|---|---|
| 230 | Me | 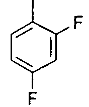 | 549(M+H)⁺ |
| 231 | Ph | H | 499(M+H)⁺ |
| 232 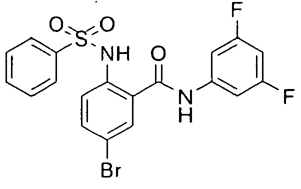 | | | 476(M+H)⁺ |

**[Table 48]**

| | | |
|---|---|---|
| 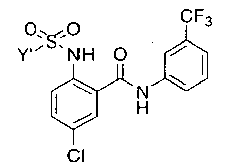 | | |

| Compound | Y' | MS |
|---|---|---|
| 233 | 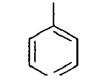 | 455(M+H)⁺ |
| 234 | 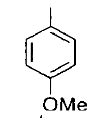 | 485(M+H)⁺ |
| 235 | 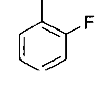 | 473M+H)⁺ |

**[Table 49]**

| | | |
|---|---|---|
| 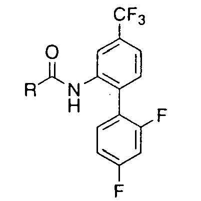 | | |

| Compound | R | MS |
|---|---|---|
| 236 | 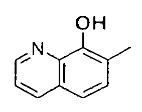 | 445(M+H)⁺ |
| 237 | 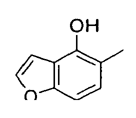 | 434(M+H)⁺ |
| 238 | 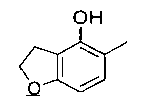 | 436 (M+H)⁺ |
| 239 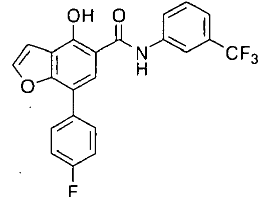 | | 416(M+H)⁺ |
| 240 | 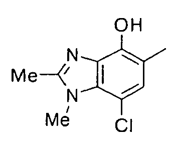 | 496(M+H)⁺ |
| 241 | 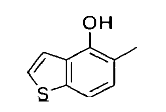 | 450(M+H)⁺ |
| 242 | 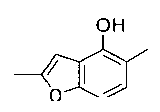 | 448(M+H)⁺ |
| 243 | 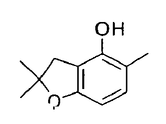 | 464(R4+H)⁺ |

This invention also includes the following compounds (244 to 400) synthesized as above.

**[Table 50]**

| | | |
|---|---|---|
| 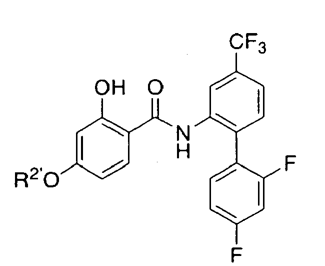 | | |

| Compound | R^{2'} | MS: m/z |
|---|---|---|
| 244 | 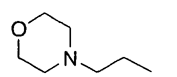 | 523 (M+H)⁺ |
| 245 | 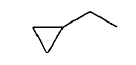 | 464 (M+H)⁺ |
| 246 | 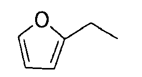 | 490 (M+H)⁺ |
| 247 | 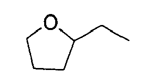 | 494 (M+H)⁺ |
| 248 | 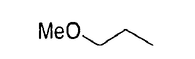 | 468 (M+H)⁺ |
| 249 | 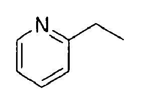 | 501 (M+H)⁺ |
| 250 | 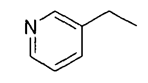 | 501 (M+H)⁺ |
| 251 | 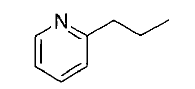 | 515 (M+H)⁺ |
| 252 | 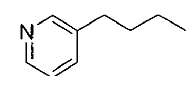 | 529 (M+H)⁺ |
| 253 | 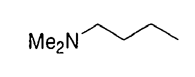 | 495 (M+H)⁺ |
| 254 | 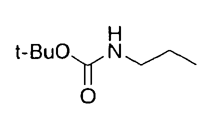 | 551(M+H) |

**[Table 51]**

| | | |
|---|---|---|
| 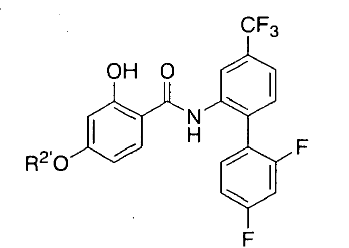 | | |

| Compound. | R^{2'} | MS: m/z |
|---|---|---|
| 255 | 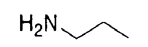 | 453 (M+H)⁻ |
| 256 | 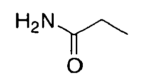 | 467 (M+H)⁺ |
| 257 | 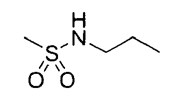 | 531 (M+H)⁺ |
| 258 | i-Pr | 452 (M+H)⁺ |
| 259 | 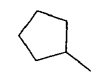 | 476 (M+H)⁻ |
| 260 | 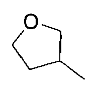 | 480 (M+H)⁺ |
| 261 | 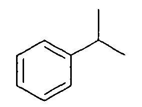 | 514 (M+H)⁺ |
| 262 | 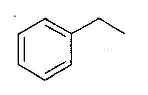 | 500 (M+H)⁺ |
| 263 | 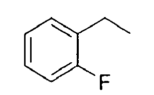 | 518 (M+H)⁺ |
| 264 | 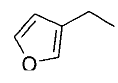 | 488 (M+H)⁻ |

**[Table 52]**

| | | |
|---|---|---|
| 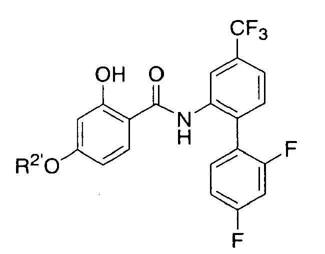 | | |

| Compound | R^{2'} | MS: m/z |
|---|---|---|
| 265 | 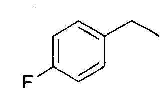 | 516 (M+H)⁻ |
| 266 | 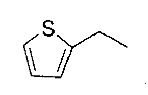 | 504 (M+H)⁻ |
| 267 | 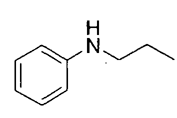 | 529 (M+H)⁺ |
| 268 | 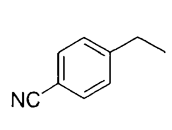 | 523 (M+H)⁻ |
| 269 | 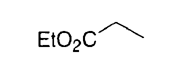 | 496 (M+H)⁺ |
| 270 | 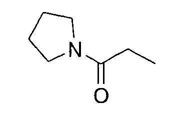 | 521 (M+H)⁺ |
| 271 | 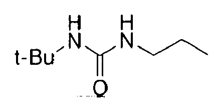 | 552 (M+H)⁺ |
| 272 | 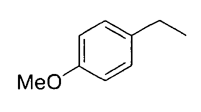 | 528 (M+H)⁻ |
| 273 | 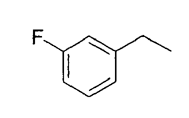 | 516 (M+H)⁻ |

**[Table 53]**

| | | |
|---|---|---|
| 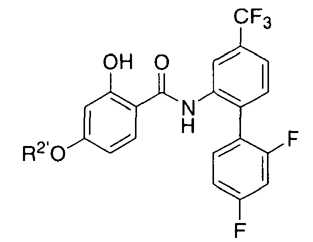 | | |

| Compound | R^{2'} | MS: m/z |
|---|---|---|
| 274 | 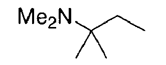 | 509 (M+H)⁺ |
| 275 | 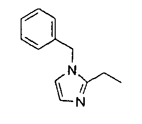 | 580 (M+H)⁺ |
| 276 | 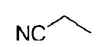 | 447 (M+H)⁻ |
| 277 | 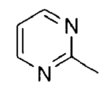 | 486 (M+H)⁻ |
| 278 | MeO₂S- | 488 (M+H)⁺ |
| 279 | 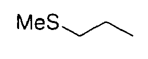 | 482 (M+H)⁻ |
| 280 | 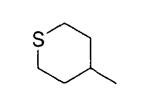 | 508 (M+H)⁻ |
| 281 | 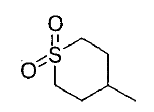 | 540 (M+H)⁻ |
| 282 | 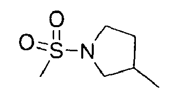 | 555 (M+H)⁻ |
| 283 | 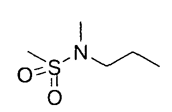 | 545 (M+H)⁺ |

**[Table 54]**

| | | |
|---|---|---|
| 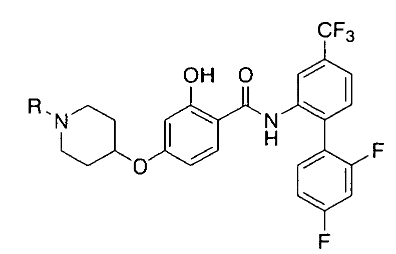 | | |

| Compound | R | MS: m/z |
|---|---|---|
| 284 | 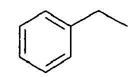 | 583 (M+H)⁺ |
| 285 | 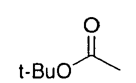 | 591 (M+H)⁻ |
| 286 | H | 493 (M+H)⁺ |
| 287 | 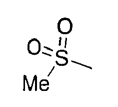 | 569 (M+H)⁻ |
| 288 | 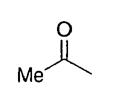 | 533 (M+H)⁻ |

**[Table 55]**

| | | |
|---|---|---|
| 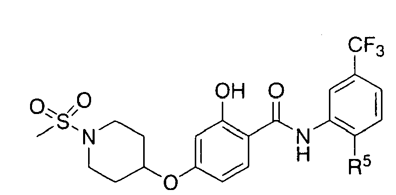 | | |

| Compound | R⁵ | MS: m/z |
|---|---|---|
| 289 | 4-Cl-phenyl | 567 (M+H)⁻ |
| 290 | 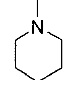 | 540 (M+H)⁻ |
| 291 | 4-F-phenyl | 551 (M+H)⁻ |
| 292 | 3-F-phenyl | 551 (M+H)⁻ |
| 293 | 4-MeO-phenyl | 563 (M+H)⁻ |
| 294 | 2-thienyl | 539 (M+H)⁻ |
| 295 | 3-Cl-phenyl | 567 (M+H)⁻ |
| 296 | 3,4-di-F-phenyl | 569 (M+H)⁻ |
| 297 | phenyl | 532 (M+H)⁻ |
| 298 | 3-MeO-phenyl | 563 (M+H)⁻ |
| 299 | 3-Me-phenyl | 547 (M+H)⁻ |
| 300 | 3-CN-phenyl | 558 (M+H)⁻ |
| 301 | 5-MeO-pyridin-3-yl | 564 (M+H)⁻ |
| 302 | 2-furyl | 523 (M+H)⁻ |
| 303 | 4-Me-phenyl | 547 (M+H)⁻ |
| 304 | 2-F-phenyl | 551 (M+H)⁻ |
| 305 | 4-CN-phenyl | 558 (M+H)⁻ |
| 306 | Br | 538 (M+H)⁺ |
| 307 | 2-Me-4-F-phenyl | 565 (M+H)⁻ |
| 308 | 2-Cl-phenyl | 567 (M+H)⁻ |
| 309 | 2-Me-phenyl | 546 (M+H)⁻ |
| 310 | 2-MeO-phyenyl | 563 (M+H)⁻ |
| 311 | 2-F-5-Cl-phenyl | 585 (M+H)⁻ |
| 312 | 2-F-5-Me-phenyl | 565 (M+H)⁻ |
| 313 | 4-F-5-Cl-phenyl | 585 (M+H)⁻ |
| 314 | 4-MeO-5-Cl-phenyl | 597 (M+H)⁻ |
| 315 | 4-MeO-5-F-phenyl | 581 (M+H)⁻ |

**[Table 56]**

| | | | | |
|---|---|---|---|---|
| 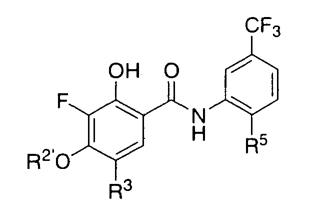 | | | | |

| Compound | R^{2'} | R³ | R⁵ | MS: m/z |
|---|---|---|---|---|
| 316 | Me | H | 2,4-di-F-phenyl | 442(M+H)⁺ |
| 317 | Me | Cl | 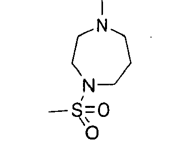 | 540(M+H)⁺ |
| 318 | Me | H | 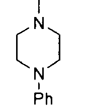 | 490 (M+H)⁺ |
| 319 | Me | Br | 2,4-di-F-phenyl | 520 (M+H)⁺ |
| 320 | Et | H | 2, 4-di-F-phenyl | 456 (M+H)⁺ |
| 321 | Me | Cl | 2,4-di-F-phenyl | 476 (M+H)⁺ |
| 322 | Me | H | 4-Cl-phenyl | 440 (M+H)⁺ |
| 323 | Et | H | 4-Cl-phenyl | 454 (M+H)⁺ |
| 324 | Me | Cl | 4-Cl-phenyl | 475 (M+H)⁺ |
| 325 | Et | Br | 2,4-di-F-phenyl | 534 (M+H)⁺ |
| 326 | Me | Br | 4-Cl-phenyl | 518 (M+H)⁺ |
| 327 | Et | Br | 4-Cl-phenyl | 532 (M+H)⁺ |
| 328 | Et | Cl | 2,4-di-F-phenyl | 490 (M+H)⁺ |
| 329 | Et | Cl | 4-Cl-phenyl | 489 (M+H)⁺ |
| 330 | Et | H | 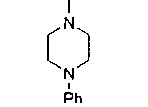 | 504 (M+H)⁺ |
| 331 | Et | H | 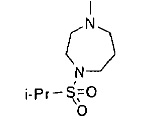 | 548 (M+H)⁺ |
| 332 | PhCH₂ | H | 4-Cl-phenyl | 516 (M+H)⁺ |
| 333 | i-Pr | H | 4-Cl-phenyl | 468 (M+H)⁺ |
| 334 | 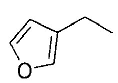 | H | 4-Cl-phenyl | 506 (M+H)⁺ |
| 335 | 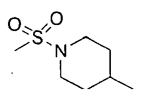 | H | 4-Cl-phenyl | 587 (M+H)⁺ |

**[Table 57]**

| | | | | | |
|---|---|---|---|---|---|
| 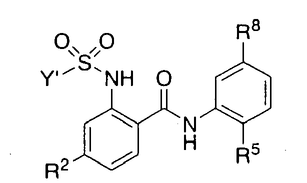 | | | | | |

| Compound | Y' | R² | R⁵ | R⁸ | MS: m/z |
|---|---|---|---|---|---|
| 336 | Ph | NO₂ | H | CF₃ | 466 (M+H)⁺ |
| 337 | Ph | Cl | H | CF₃ | 455(M+H)⁺ |
| 338 | Ph | Cl | CF₃ | CF₃ | 523 (M+H)⁺ |
| 339 | Ph | Cl | H | F | 405 (M+H)⁺ |
| 340 | Ph | Cl | F | F | 423 (M+H)⁺ |
| 341 | Ph | Br | H | CF₃ | 499 (M+H)⁺ |
| 342 | Ph | CN | H | CF₃ | 446 (M+H)⁺ |
| 343 | Ph | NO₂ | H | F | 416 (M+H)⁺ |
| 344 | Ph | NO₂ | CF₃ | CF₃ | 534 (M+H)⁺ |
| 345 | Ph | NO₂ | 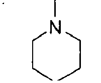 | CF₃ | 549 (M+H)⁺ |
| 346 | Ph | NO₂ | 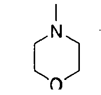 | CF₃ | 551 (M+H)⁺ |
| 347 | Ph | NO₂ | 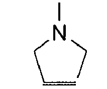 | CF₃ | 535 (M+H)⁺ |
| 348 | Ph | NO₂ | H | Cl | 432 (M+H)⁺ |
| 349 | Ph | NO₂ | H | OCF₃ | 482 (M+H)⁺ |
| 350 | Ph | NO₂ | OMe | CF₃ | 496 (M+H)⁺ |
| 351 | Ph | H | H | CF₃ | 421 (M+H)⁺ |
| 352 | Ph | H | CF₃ | CF₃ | 489 (M+H)⁺ |
| 353 | 4-Cl-phenyl | NO₂ | H | CF₃ | 500 (M+H)⁺ |
| 354 | 4-F-phenyl | NO₂ | H | CF₃ | 484 (M+H)⁺ |
| 355 | Ph | 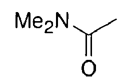 | H | CF₃ | 492 (M+H)⁺ |
| 356 | Ph | 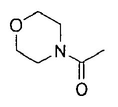 | H | CF₃ | 534 (M+H)⁺ |
| 357 | Ph | 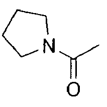 | H | CF₃ | 518 (M+H)⁺ |

**[Table 58]**

| | | | | | |
|---|---|---|---|---|---|
| 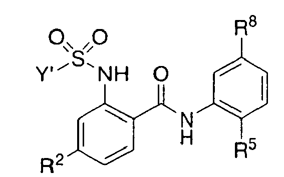 | | | | | |

| Compound | Y' | R² | R⁵ | R⁸ | MS: m/z |
|---|---|---|---|---|---|
| 358 | Ph | OMe | H | CF₃ | 451 (M+H)⁺ |
| 359 | Ph | OMe | CF₃ | CF₃ | 519 (M+H)⁺ |
| 360 | Ph | OMe | 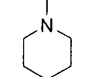 | CF₃ | 534 (M+H)⁺ |
| 361 | 2-CN-phenyl | NO₂ | H | CF₃ | 491 (M+H)⁺ |
| 362 | Ph | NO₂ | 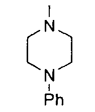 | CF₃ | 626 (M+H)⁺ |

**[Table 59]**

| Compound | | MS: m/z |
|---|---|---|
| 363 | 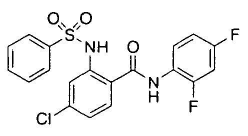 | 423 (M+H)⁺ |
| 364 | 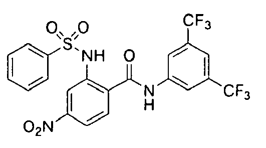 | 534 (M+H)⁺ |
| 365 | 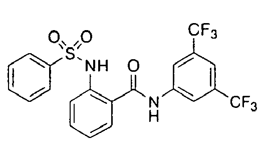 | 489 (M+H)⁺ |
| 366 | 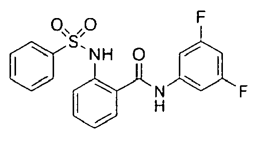 | 389 (M+H)⁺ |

**[Table 60]**

| | | | | | |
|---|---|---|---|---|---|
| 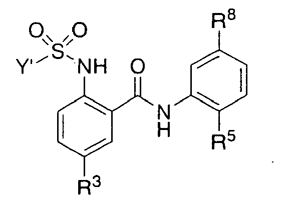 | | | | | |

| Compound | Y' | R³ | R⁵ | R⁸ | MS: m/z |
|---|---|---|---|---|---|
| 367 | Ph | Br | H | F | 450 (M+H)⁺ |
| 368 | 4-MeO-phenyl | Cl | H | CF₃ | 485 (M+H)⁺ |
| 369 | 2-F-phenyl | Cl | H | CF₃ | 473 (M+H)⁺ |
| 370 | 4-CN-phenyl | Cl | H | CF₃ | 480 (M+H)⁺ |
| 371 | Ph | 4-F-pheny | H | CF₃ | 515 (M+H)⁺ |
| 372 | Ph | 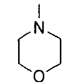 | H | CF₃ | 542 (M+H)⁺ |
| 373 | Ph | Br | CF₃ | CF₃ | 567 (M+H)⁺ |
| 374 | Ph | Br | 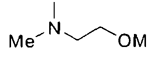 | CF₃ | 586 (M+H)⁺ |
| 375 | 3-F-phenyl | Cl | H | CF₃ | 473 (M+H)⁺ |
| 376 | 4-F-phenyl | Cl | H | CF₃ | 473 (M+H)⁺ |
| 377 | Ph | CN | H | CF₃ | 446 (M+H)⁺ |
| 378 | Ph | NO₂ | H | CF₃ | 466 (M+H)⁺ |
| 379 | Ph | Br | 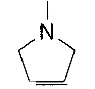 | CF₃ | 568 (M+H)⁺ |
| 380 | Ph | Br | H | Cl | 465 (M+H)⁺ |
| 381 | Ph | CN | CF₃ | CF₃ | 514 (M+H)⁺ |
| 382 | Ph | CN | 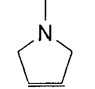 | CF₃ | 515 (M+H)⁺ |
| 383 | Ph | CN | H | Cl | 412 (M+H)⁺ |
| 384 | Ph | Br | OMe | CF₃ | 529 (M+H)⁺ |
| 385 | Ph | Br | 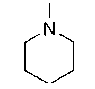 | CF₃ | 582 (M+H)⁺ |
| 386 | Ph | CN | OMe | CF₃ | 476 (M+H)⁺ |
| 387 | Ph | CN | 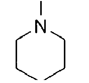 | CF₃ | 529 (M+H)⁺ |
| 388 | Ph | CN | H | F | 396 (M+H)⁺ |
| 389 | 4-Cl-phenyl | Br | H | CF₃ | 533 (M+H)⁺ |
| 390 | 4-F-phenyl | Br | H | CF₃ | 517 (M+H)⁺ |
| 391 | 4-Cl-phenyl | CN | H | CF₃ | 480 (M+H)⁺ |
| 392 | 4-F-phenyl | CN | H | CF₃ | 464 (M+H)⁺ |

**[Table 61]**

| Compound | | MS: m/z |
|---|---|---|
| 393 | 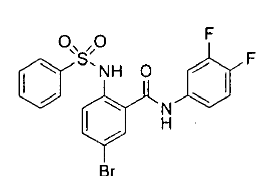 | 468(M+H)⁺ |
| 394 | 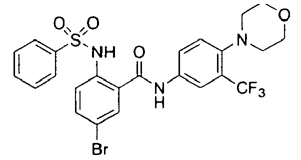 | 585 (M+H)⁺ |
| 395 | 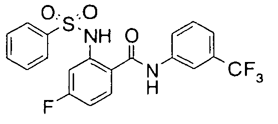 | 437(M - H)⁻ |
| 396 | 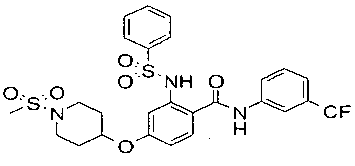 | 596(M - H)⁻ |
| 397 | 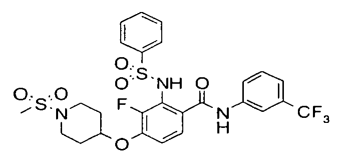 | |
| 398 | 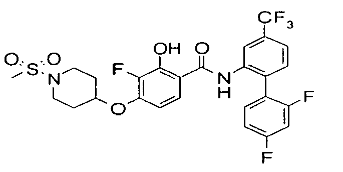 | 587 (M-H)⁻ |
| 399 | 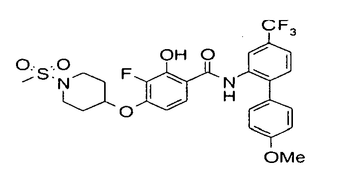 | 581 (M-H)⁻ |
| 400 | 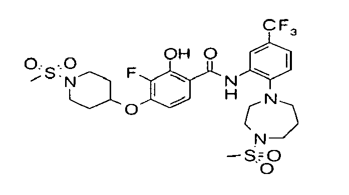 | 651 (M-H)⁻ |

### Experimental Example 1

### Establishment of a cell line and luciferase assay

Several kidneys of 6 to 8-week-old male Wistar rats were perfused with sterilized phosphate buffered saline (PBS) and excised. Glomeruli were isolated from the cortex by a sieving method (with 180, 125 or 63µm mesh). Isolated glomeruli were cultured in RPMI1640 medium containing 20% bovine serum, 1% penicillin-streptomycin and 1% Hepes buffer at 37 °C under 5% carbon dioxide (CO₂) atmosphere. After 3 weeks, the medium was changed to the new one. After 4 weeks, mesangial cells which proliferated and became confluent were diluted 2 to 5 times and subcultured. Subcultures were repeated over 20 times, and mesangial cells cultured in usual RPMI1640 medium containing 10% bovine serum, 1% penicillin-streptomycin and 1% Hepes buffer were cloned by a limiting dilution method to establish cell line Ms0-2.

Next, a chimeric gene that human CTGF promoter region containing TGF-β responsive element was connected to luciferase expression region (pGVB) was constructed and cotransfected with G418 resistant gene pWLneo into MsO-2 cells. Cells were selected in the medium containing G418 (400µg/ml) and a cell line (Ms0-2-3) obtained by isolating the colonies was used for luciferase assay.

Luciferase assay was performed with Ms0-2-3 cells which were cultured with serum-free medium for 48 hours. The cells were stimulated with TGF-6 (2 ng/ml) for 2 hours after addition of a compound. After 24 hours, cell lysis solution was added and the cells were dissolved. 20µl of cell lysate was transferred to a plate suitable for the assay of luciferase activity. Substrate was added thereto and luciferase activity of each well was measured by luminometer. Enhancement of luciferase activity by TGF-β stimulation (without a compound) was determined as 100% and the inhibitory rate of activity by addition of the compound was calculated. The value obtained from the inhibitory curve was judged as the inhibitory rate against CTGF promoter activity and determined as IC50 value. The following Table 62 shows IC50 values of compounds of the present invention. IC50 values of the other compounds of the present invention are 0.002 to 3.74 µM.

**[Table 62]**

| Compound | IC₅₀ (µM) | Compound | IC₅₀ (µM) | Compound | IC₅₀ (µM) | Compound | IC₅₀ (µM) |
|---|---|---|---|---|---|---|---|
| 7 | 0.224 | 56 | 0.049 | 109 | 0.064 | 3-5 | 0.157 |
| 25 | 0.081 | 79 | 0.004 | 127 | 0.166 | 3-23 | 0.025 |
| 43 | 0.102 | 96 | 0.302 | 144 | 0.035 | 3-40 | 0.468 |
| 157 | 0.002 | 217 | 0.278 | 177 | 0.400 | 235 | 0.033 |

### Experimental Example 2

### Inhibitory activity on CTGF expression in cultured cells

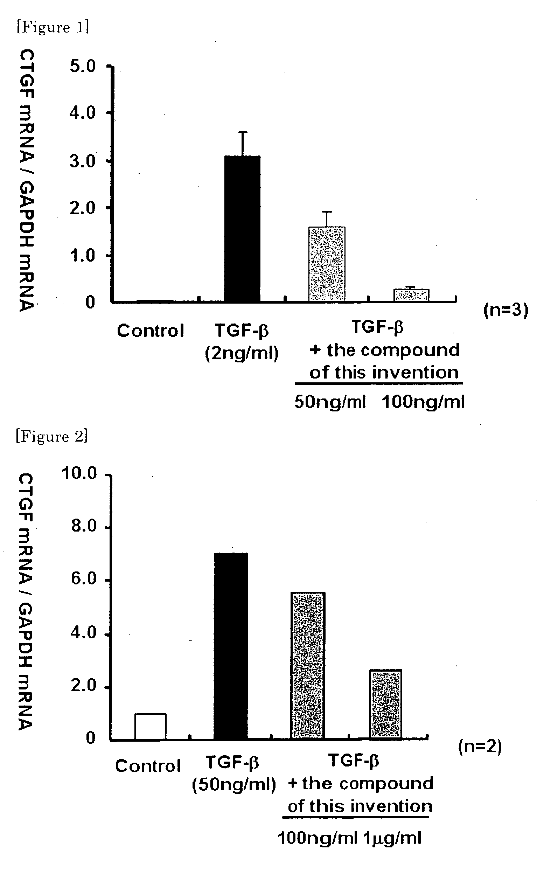

The following experiment was performed to confirm that compounds found out by the luciferase assay with Ms0-2-3 cells have real inhibitory activity on CTGF expression in cultured cells.
Ms0-2 cells were used in the experiment. After they were cultured with serum-free medium for 48 hours, a compound was added and the cells were stimulated by TGF-β(2ng/ml) after another 2 hours. After 16 hours, the cells were washed with PBS. The solubilizing agent (ISOGEN) was added and total RNA was extracted by the well-known method. Obtained RNA was reverse transcribed by the well-known method and quantitative PCR was performed with primers and probes which were engineered and synthesized to calculate the amount of CTGF mRNA. By correcting the amount of CTGF mRNA with the amount of GAPDH mRNA measured at the same time, CTGF/GAPDH ratio was calculated. Inhibitory activity on CTGF expression of a compound of the present invention in cultured cells was confirmed. The results were shown in Figure 1.

### Experimental Example 3

### Inhibitory activity on CTGF expression in cultured glomeruli

According to Example 1 described in JP2005-229834, glomeruli were isolated. The isolated glomeruli were plated on serum-free RPMI1640 medium containing insulin, transferrin and selenium at the rate of 1.5-2.0 x 10⁴ /1ml/well and cultured at 37 °C under 5% CO₂ atmosphere. They were stimulated by TGF-β (50 ng/ml) for 2 hours after addition of a compound. After 24 hours, glomeruli were collected and washed with PBS. Total RNA extraction, reverse transcription and quantization of the amount of CTGF mRNA were performed as the method described in Experimental Example 2. Inhibitory activity on CTGF expression of a compound of the present invention in cultured glomeruli was confirmed. The results were shown in Figure 2.

### Experimental Example 4

### In vivo CTGF inhibitory activity

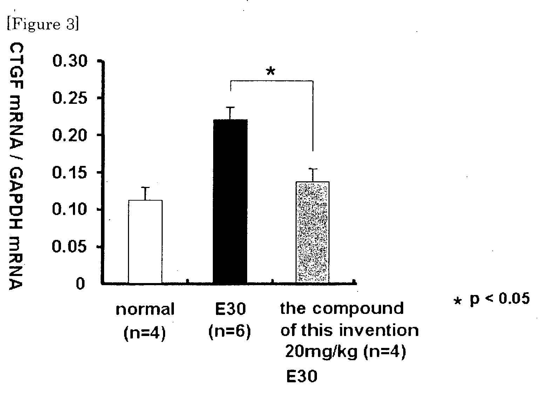

*In vivo* inhibitory activity on CTGF expression of a compound was evaluated by producing kidney disorder models and referring to the previous information that the amount of CTGF expression in the kidney cortex is enhanced when the disease develops.
8 to 9-week-old male Wistar rats were used in the experiment. E-30 monoclonal antibody produced according to a method described in [Exp Nephrol, 10:245-258(2002)] was diluted with physiologic saline (Otsuka Normal Saline, Otsuka Pharmaceutical Co., Ltd.) to became 100µg/0.4ml/rat and administered from rat tail vein under anesthesia of ether to develop the disease. A compound suspended in 0.5% methylcellulose solution was administered singly and orally 2 days after administration of antibody. Kidneys were perfused and extracted under pentobarbital anesthesia on the next day of the administration. ISOGEN was added to the collected kidney cortex, which was used for measurement of CTGF mRNA. A compound of the present invention significantly inhibited enhancement of CTGF expression level in kidney cortex (Figure 3).

### Formulation Example

The following formulation examples 1 to 8 are provided to further illustrate the present invention and are not intended to limit the scope of the present invention. The term of "active ingredient" means a compound of the present invention, a tautomer, a prodrug, a pharmaceutical acceptable salt, or a hydrate thereof.

### (Formulation Example 1)

Hard gelatin capsules are prepared with the following ingredients:

| | Dose (mg/capsule) |
|---|---|
| Active ingredient | 250 |
| Starch (dried) | 200 |
| Magnesium stearate | 10 |
| Total | 460 mg |

### (Formulation Example 2)

Tablets are prepared with the following ingredients:

| | Dose (mg/tablet) |
|---|---|
| Active ingredient | 250 |
| Cellulose (microcrystal) | 400 |
| Silicon dioxide, fumed | 10 |
| Stearic acid | 5 |
| Total | 665 mg |

The ingredients are blended and compressed to form tablets each weighing 665 mg.

### (Formulation Example 3)

An aerosol solution is prepared containing the following ingredients :

| | Weight |
|---|---|
| Active ingredient | 0.25 |
| Ethanol | 25.75 |
| Propellant 22 (chlorodifluoromethane) | 74.00 |
| Total | 100.00 |

The active ingredient is mixed with ethanol and the admixture is added to a portion of the propellant 22, cooled to -30 °C and transferred to a filling device. Then the required amount is provided in a stainless steel container and diluted with the reminder of the propellant. The valve units are then attached to the container.

### (Formulation Example 4)

Tablets, each containing 60 mg of active ingredient, are made as follows.

| | |
|---|---|
| Active ingredient | 60 mg |
| Starch | 45 mg |
| Microcrystals cellulose | 35 mg |
| Polyvinylpyrrolidone (as 10% solution in water) | 4 mg |
| Sodium carboxymethyl starch | 4.5 mg |
| Magnesium stearate | 0.5 mg |
| Talc | 1 mg |
| Total | 150 mg |

The active ingredient, starch, and cellulose are passed through a No. 45 mesh U.S. sieve, and the mixed thoroughly. The aqueous solution containing polyvinylpyrrolidone is mixed with the obtained powder, and then the admixture is passed through a No. 14 mesh U.S. sieve. The granules so produced are dried at 50 °C and passed through a No. 18 mesh U.S. sieve. The sodium carboxymethyl starch, magnesium stearate, and talc, previously passed through No. 60 mesh U.S. sieve, are added to the granules, mixed, and then compressed on a tablet machine to yield tablets each weighing 150 mg.

### (Formulation Example 5)

Capsules, each containing 80 mg of active ingredient, are made as follows:

| | |
|---|---|
| Active ingredient | 80 mg |
| Starch | 59 mg |
| Microcrystals cellulose | 59 mg |
| Magnesium stearate | 2 mg |
| Total | 200 mg |

The active ingredient, cellulose, starch, and magnesium stearate are blended, passed through a No. 45 mesh U.S. sieve, and filled into hard gelatin capsules in 200 mg quantities.

### (Formulation Example 6)

Suppositories, each containing 225 mg of active ingredient, are made as follows:

| | |
|---|---|
| Active ingredient | 225 mg |
| Saturated fatty acid glycerides | 2000 mg |
| Total | 2225 mg |

The active ingredient is passed through a No. 60 mesh U.S. sieve and suspended in the saturated fatty acid glycerides previously melted using the minimum heat necessary. The mixture is then poured into a suppository mold of nominal 2g capacity and allowed to cool.

### (Formulation Example 7)

Suspensions, each containing 50 mg of active ingredient, are made as follows:

| | |
|---|---|
| Active ingredient | 50 mg |
| Sodium carboxymethyl cellulose | 50 mg |
| Syrup | 1.25 mL |
| Benzoic acid solution | 0.10 mL |
| Flavor | q.v. |
| Color | q.v. |
| Purified water to total | 5 mL |

The active ingredient is passed through a No. 45 U.S. sieve, and mixed with the sodium carboxymethyl cellulose and syrup to form a smooth paste. The benzoic acid solution and flavor are diluted with a portion of the water, added and stirred. Then sufficient water is added to produce the required volume.

### (Formulation Example 8)

An intravenous formulation may be prepared as follows:

| | |
|---|---|
| Active ingredient | 100 mg |
| Isotonic saline | 1000 mL |

The solution of the above ingredients is generally administered intravenously to a patient at a rate of 1 mL per minute.

### Industrial Applicability

Compounds of the present invention have inhibitory activity on CTGF expression. Therefore, a pharmaceutical composition comprising a compound of the present invention is useful for therapy of a disease caused by overexpression of CTGF.

### Brief Description of the Drawings

- [Figure 1]: Inhibitory activity on CTGF expression in cultured cells
- [Figure 2]: Inhibitory activity on CTGF expression in cultured glomeruli
- [Figure 3]: *In vivo* CTGF inhibitory activity

## Claims

1. A CTGF expression inhibitor comprising a compound of the formula I: 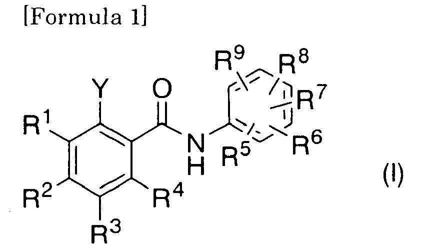 a pharmaceutically acceptable salt or solvate thereof as an active ingredient,
(wherein Y is hydroxy or a group of the formula: -NH-SO₂-Y' (wherein Y' is optionally substituted aryl or optionally substituted alkyl),
R¹ is hydrogen, optionally substituted alkyl, optionally substituted amino, nitro, optionally substituted alkoxy, halogen, optionally substituted alkenyl or optionally substituted alkynyl,
R² is hydrogen, halogen, nitro, optionally substituted amino, cyano, optionally substituted alkyl, optionally substituted carbamoyl, hydroxy, optionally substituted alkenyl, optionally substituted alkynyl, optionally substituted aryl or
a group of the formula: -O-R² (wherein R²' is optionally substituted alkyl, alkylsulfonyl, cycloalkyl, optionally substituted nonaromatic heterocycle or heteroaryl), or
R¹ and R² can be taken together with the neighboring carbon atom to form an optionally substituted 5 or 6-membered ring optionally containing heteroatom(s),
R³ is hydrogen, halogen, cyano, optionally substituted sulfamoyl, optionally substituted carbamoyl, optionally substituted amino, nitro, optionally substituted alkyl, optionally substituted alkoxy, optionally substituted alkenyl, optionally substituted aryl, optionally substituted heteroaryl, optionally substituted nonaromatic heterocycle or
a group of the formula: -C≡C-R^{3'} (wherein R^{3'} is hydrogen, optionally substituted aryl, optionally substituted heteroaryl, hydroxy or optionally substituted alkyl),
R⁴ is hydrogen, halogen, optionally substituted alkyl, optionally substituted alkoxy, optionally substituted alkenyl, optionally substituted alkynyl or optionally substituted alkylthio,
R⁵ is hydrogen, carbamoyl, cyano, nitro, halogen, optionally substituted alkyl, alkenyl, alkoxycarbonyl amino, alkoxy, haloalkoxy, alkylthio, alkylsulfinyl, alkylsulfonyl, optionally substituted amino, optionally substituted aryl, optionally substituted heteroaryl, optionally substituted nonaromatic heterocycle,
a group of the formula: -X'-R^{5'}(wherein X' is -C≡C-, and R^{5'} is optionally substituted aryl, optionally substituted heteroaryl, optionally substituted nonaromatic heterocycle, optionally substituted alkyl, alkoxy, hydroxy or hydrogen) or
a group of the formula: -X"-R^{5"} (wherein X" is -O-Z-, -S-Z-, -C(=O)-, -SO-Z-, -SO₂-Z-, -NRSO₂-, -NRC(=O)-, -SO₂NR-, -C(=O)NR-, -CR(OH)-, -SO₂O- or -NR-, R^{5"} is optionally substituted aryl, optionally substituted heteroaryl or optionally substituted nonaromatic heterocycle, R is hydrogen or alkyl, and Z is a bond or alkylene), and
R⁶, R⁷, R⁸ and R⁹ are each independently hydrogen, optionally substituted alkyl, optionally substituted alkenyl, optionally substituted alkynyl, halogen, optionally substituted alkoxy, cyano, nitro, optionally substituted amino, optionally substituted aryl or nonaromatic heterocycle).

2. The CTGF expression inhibitor of claim 1, wherein a group of the formula: 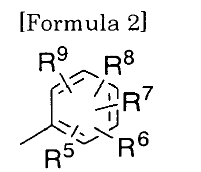 is a group of the formula:

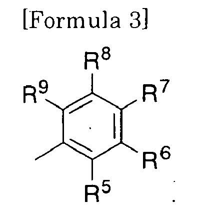

3. The CTGF expression inhibitor of claim 2, wherein
R⁶ is hydrogen, optionally substituted alkyl or halogen,
R⁷ is hydrogen, optionally substituted alkoxy, halogen, cyano, nitro, optionally substituted alkyl, optionally substituted alkenyl, optionally substituted alkynyl or nonaromatic heterocycle,
R⁸ is hydrogen, nitro, optionally substituted amino, halogen, optionally substituted alkyl, optionally substituted alkoxy, optionally substituted alkenyl, optionally substituted alkynyl, cyano or haloalkoxy, and
R⁹ is hydrogen, alkyl, halogen or optionally substituted aryl.

4. The CTGF expression inhibitor of claim 3, wherein
R⁵ is hydrogen, halogen, optionally substituted alkyl, alkoxycarbonylamino, alkoxy, haloalkoxy, alkylthio, alkylsulfinyl, alkylsulfonyl, optionally substituted amino, optionally substituted aryl, optionally substituted heteroaryl, optionally substituted nonaromatic heterocycle,
a group of the formula: -X'-R^{5'}(wherein X' is -C≡C-, and R^{5'} is optionally substituted aryl, optionally substituted heteroaryl, optionally substituted nonaromatic heterocycle, optionally substituted alkyl, alkoxy, hydroxy or hydrogen) or
a group of the formula: -X"-R^{5"} (wherein X" is -O-Z-, -S-Z-, -C(=O)-, -SO-Z-, -SO₂-Z-, -NRSO₂- -NRC(=O)-, -SO₂NR-, -C(=O)NR-, -CR(OH)-, -SO₂O- or -NR-, R^{5"} is optionally substituted aryl, optionally substituted heteroaryl or optionally substituted nonaromatic heterocycle, R is hydrogen or alkyl and Z is a bond or alkylene).

5. The CTGF expression inhibitor of claim 3, wherein
R³ is hydrogen, halogen, optionally substituted aryl, optionally substituted heteroaryl, optionally substituted nonaromatic heterocycle or
a group of the formula: -C≡C-R^{3'} (wherein R^{3'} is hydrogen, optionally substituted aryl, optionally substituted heteroaryl, hydroxy or optionally substituted alkyl).

6. A compound of the formula II: 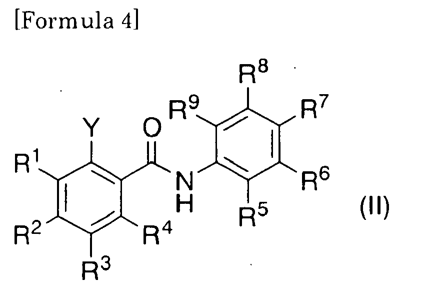 a pharmaceutically acceptable salt or solvate thereof,
(wherein Y is hydroxy or a group of the formula: -NH-SO₂-Y' (wherein Y' is optionally substituted aryl or optionally substituted alkyl),
R¹ is hydrogen, optionally substituted alkyl, optionally substituted amino, nitro, optionally substituted alkoxy, halogen, optionally substituted alkenyl or optionally substituted alkynyl,
R² is hydrogen, halogen, nitro, optionally substituted amino, cyano, optionally substituted alkyl, optionally substituted carbamoyl, optionally substituted alkoxy, hydroxy, optionally substituted alkenyl, optionally substituted alkynyl or optionally substituted aryl, or
R¹ and R² can be taken together with the neighboring carbon atom to form an optionally substituted 5 or 6-membered ring optionally containing heteroatom(s),
R³ is a group of the formula: C≡CR³ (wherein R³ is hydrogen, optionally substituted aryl, optionally substituted heteroaryl, hydroxy or optionally substituted alkyl),
R⁴ is hydrogen, halogen, optionally substituted alkyl, optionally substituted alkoxy, optionally substituted alkenyl, optionally substituted alkynyl or optionally substituted alkylthio,
R⁵ is hydrogen, carbamoyl, cyano, nitro, halogen, alkyl, alkenyl, alkoxycarbonylamino, alkoxy, haloalkoxy, alkylthio, alkylsulfinyl, alkylsulfonyl, optionally substituted aryl, optionally substituted heteroaryl, optionally substituted nonaromatic heterocycle,
a group of the formula: -X'-R⁵' (wherein X' is -C≡C-, and R^{5'} is optionally substituted aryl, optionally substituted heteroaryl, optionally substituted nonaromatic heterocycle, optionally substituted alkyl, alkoxy, hydroxy or hydrogen) or
a group of the formula: -X"-R^{5"} (wherein X" is -O-Z-, -S-Z-, -C(=O)-, -SO-Z-, -SO₂-Z-, -NRSO₂-, -NRC(=O)-, -SO₂NR-, -C(=O)NR-, -CR(OH)-, -SO₂O- or -NR-, R^{5"} is optionally substituted aryl, optionally substituted heteroaryl or optionally substituted nonaromatic heterocycle, R is hydrogen or alkyl, and Z is a bond or alkylene),
R⁶ is hydrogen, alkyl or halogen,
R⁷ is hydrogen, optionally substituted alkoxy, halogen, cyano, nitro, optionally substituted alkyl, optionally substituted alkenyl or optionally substituted alkynyl,
R⁸ is hydrogen, nitro, optionally substituted amino, halogen, optionally substituted alkyl, alkoxy, optionally substituted alkenyl, optionally substituted alkynyl, cyano or haloalkoxy, and
R⁹1s hydrogen, alkyl, halogen or optionally substituted aryl).

7. The compound of claim 6 wherein R⁸ is haloalkyl, a pharmaceutically acceptable salt or solvate thereof.

8. The compound of claim 7 wherein R⁵ is substituted aryl, a pharmaceutically acceptable salt or solvate thereof.

9. A compound of the formula II: 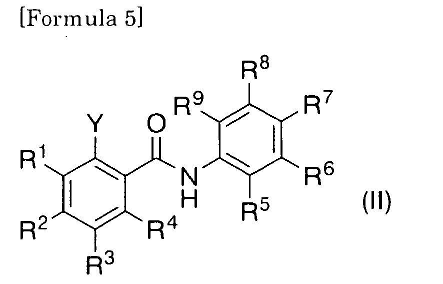 a pharmaceutically acceptable salt or solvate thereof,
(wherein Y is hydroxy or a group of the formula: -NH-SO₂-Y' (wherein Y' is optionally substituted aryl or optionally substituted alkyl),
R¹ is hydrogen, optionally substituted alkyl, optionally substituted amino, nitro, optionally substituted alkoxy, halogen, optionally substituted alkenyl or optionally substituted alkynyl,
R² is hydrogen, halogen, nitro, optionally substituted amino, cyano, optionally substituted alkyl, optionally substituted carbamoyl, optionally substituted alkoxy, hydroxy, optionally substituted alkenyl, optionally substituted alkynyl or optionally substituted aryl, or
R¹ and R² can be taken together with the neighboring carbon atom to form an optionally substituted 5 or 6-membered ring optionally containing heteroatom(s),
R³ is hydrogen, halogen, cyano, optionally substituted sulfamoyl, optionally substituted carbamoyl, optionally substituted amino, nitro, optionally substituted alkyl, optionally substituted alkoxy, optionally substituted alkenyl, optionally substituted aryl, optionally substituted heteroaryl, optionally substituted nonaromatic heterocycle or
a group of the formula: -C≡C-R³ (wherein R^{3'} is hydrogen, optionally substituted aryl, optionally substituted heteroaryl, hydroxy or optionally substituted alkyl),
R⁴ is hydrogen, halogen, optionally substituted alkyl, optionally substituted alkoxy, optionally substituted alkenyl, optionally substituted alkynyl or optionally substituted alkylthio,
R⁵ is a group of the formula: -X'-R⁵' (wherein X' is -C≡C-, and R⁵ is substituted aryl or optionally substituted alkyl),
R⁶ is hydrogen, alkyl or halogen,
R⁷ is hydrogen, optionally substituted alkoxy, halogen, cyano, nitro, optionally substituted alkyl, optionally substituted alkenyl or optionally substituted alkynyl,
R⁸ is hydrogen, nitro, optionally substituted amino, halogen, optionally substituted alkyl, alkoxy, optionally substituted alkenyl, optionally substituted alkynyl, cyano or haloalkoxy, and
R⁹ hydrogen, alkyl, halogen or optionally substituted aryl).

10. The compound of claim 9 wherein R² is halogen and R⁷ is haloalkyl, a pharmaceutically acceptable salt or solvate thereof.

11. A compound of the formula II: 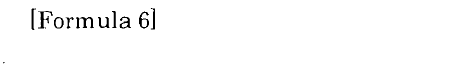 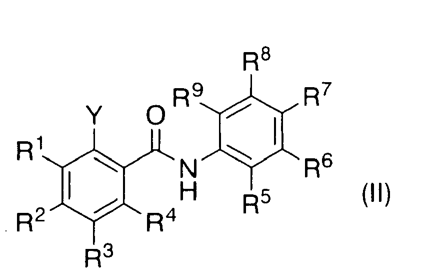 a pharmaceutically acceptable salt or solvate thereof,
(wherein Y is hydroxy or a group of the formula: -NH-SO₂-Y' (wherein Y' is optionally substituted aryl or optionally substituted alkyl),
R¹ is hydrogen, optionally substituted alkyl, optionally substituted amino, nitro, optionally substituted alkoxy, halogen, optionally substituted alkenyl or optionally substituted alkynyl,
R² is hydrogen, halogen, nitro, optionally substituted amino, cyano, optionally substituted alkyl, optionally substituted carbamoyl, optionally substituted alkoxy, hydroxy, optionally substituted alkenyl, optionally substituted alkynyl or optionally substituted aryl, or
R¹ and R² can be taken together with the neighboring carbon atom to form an optionally substituted 5 or 6-membered ring optionally containing heteroatom(s),
R³ is substituted aryl or optionally substituted heteroaryl,
R⁴ is hydrogen, halogen, optionally substituted alkyl, optionally substituted alkoxy, optionally substituted alkenyl, optionally substituted alkynyl or optionally substituted alkylthio,
R⁵ is hydrogen, carbamoyl, cyano, nitro, halogen, alkyl, alkenyl, alkoxycarbonylamino, alkoxy, haloalkoxy, alkylthio, alkylsulfinyl, alkylsulfonyl, optionally substituted aryl, optionally substituted heteroaryl, optionally substituted nonaromatic heterocycle,
a group of the formula: -X'-R^{5'} (wherein X' is -C=C-, and R^{5'} is optionally substituted aryl, optionally substituted heteroaryl, optionally substituted nonaromatic heterocycle, optionally substituted alkyl, alkoxy, hydroxy or hydrogen) or
a group of the formula: -X"-R^{5"} (wherein X" is -O-Z-, -S-Z-, -C(=O)-, -SO-Z-, -SO₂-Z-, -NRSO₂-, -NRC(=O)-, -SO₂NR-, -C(=O)NR-, -CR(OH)-, -SO₂O- or -NR-, R^{5"} is optionally substituted aryl, optionally substituted heteroaryl or optionally substituted nonaromatic heterocycle, R is hydrogen or alkyl, and Z is a bond or alkylene),
R⁶ is hydrogen, alkyl or halogen,
R⁷ is hydrogen, optionally substituted alkoxy, halogen, cyano, nitro, alkyl, haloalkyl, optionally substituted alkenyl or optionally substituted alkynyl,
R⁸ is hydrogen, nitro, optionally substituted amino, halogen, optionally substituted alkyl, alkoxy, optionally substituted alkenyl, optionally substituted alkynyl, cyano or haloalkoxy, and
R⁹ is hydrogen, alkyl, halogen or optionally substituted aryl, provided that, one of R⁷ and R⁸ is not hydrogen).

12. The compound of claim 11 wherein R⁸ is haloalkyl, a pharmaceutically acceptable salt or solvate thereof.

13. A compound of the formula II: 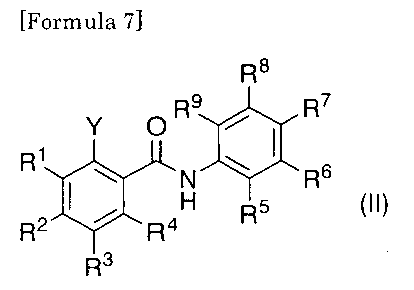 a pharmaceutically acceptable salt or solvate thereof,
(wherein Y is hydroxy or a group of the formula: -NH-SO₂-Y' (wherein Y' is optionally substituted aryl or optionally substituted alkyl),
R¹ is hydrogen, optionally substituted alkyl, optionally substituted amino, nitro, optionally substituted alkoxy, halogen, optionally substituted alkenyl or optionally substituted alkynyl,
R² is hydrogen, halogen, nitro, optionally substituted amino, cyano, optionally substituted alkyl, optionally substituted carbamoyl, hydroxy, optionally substituted alkenyl, optionally substituted alkynyl, optionally substituted aryl or
a group of the formula: -O-R^{2'}(wherein R^{2'} is optionally substituted alkyl, alkylsulfonyl, cycloalkyl, optionally substituted nonaromatic heterocycle or heteroaryl), or
R¹ and R² can be taken together with the neighboring carbon atom to form an optionally substituted 5 or 6-membered ring optionally containing heteroatom(s),
R³ is hydrogen, halogen, cyano, optionally substituted sulfamoyl, optionally substituted carbamoyl, optionally substituted amino, nitro, optionally substituted alkyl, optionally substituted alkoxy, optionally substituted alkenyl, optionally substituted aryl, optionally substituted heteroaryl, optionally substituted nonaromatic heterocycle or
a group of the formula: -C≡C-R^{3'} (wherein R³ is hydrogen, optionally substituted aryl, optionally substituted heteroaryl, hydroxy or optionally substituted alkyl),
R⁴ is hydrogen, halogen, optionally substituted alkyl, optionally substituted alkoxy, optionally substituted alkenyl, optionally substituted alkynyl or optionally substituted alkylthio,
R⁵ is substituted aryl,
R⁶ is hydrogen, alkyl or halogen,
R⁷ is hydrogen, optionally substituted alkoxy, halogen, cyano, nitro, optionally substituted alkyl, optionally substituted alkenyl or optionally substituted alkynyl,
R⁸ is hydrogen, nitro, optionally substituted amino, halogen, optionally substituted alkyl, alkoxy, optionally substituted alkenyl, optionally substituted alkynyl, cyano or haloalkoxy, and
R⁹ is hydrogen, alkyl, halogen or optionally substituted aryl).

14. The compound of claim 13 wherein either R⁷ or R⁸ is haloalkyl or haloalkoxy, a pharmaceutically acceptable salt or solvate thereof.

15. The compound of claim 14 wherein R² is halogen, a pharmaceutically acceptable salt or solvate thereof.

16. The compound of claim 14 wherein R¹ is optionally substituted alkyl, a pharmaceutically acceptable salt or solvate thereof.

17. The compound of claim 14 wherein R³ is halogen or substituted aryl, a pharmaceutically acceptable salt or solvate thereof.

18. A compound of the formula II: 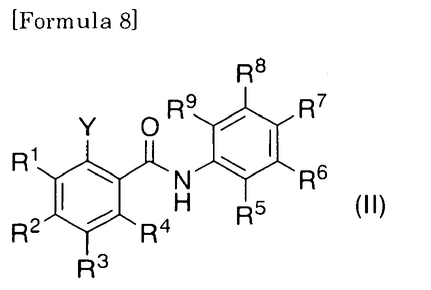 a pharmaceutically acceptable salt or solvate thereof,
(wherein Y is hydroxy or a group of the formula: -NH-SO₂-Y' (wherein Y' is optionally substituted aryl or optionally substituted alkyl),
R¹ is hydrogen, optionally substituted alkyl, optionally substituted amino, nitro, optionally substituted alkoxy, halogen, optionally substituted alkenyl or optionally substituted alkynyl,
R² is halogen, nitro, optionally substituted amino, cyano, optionally substituted alkyl, optionally substituted carbamoyl, hydroxy, optionally substituted alkenyl, optionally substituted alkynyl, optionally substituted aryl or
a group of the formula: -O-R^{2'} (wherein R^{2'} is optionally substituted alkyl, alkylsulfonyl, cycloalkyl, optionally substituted nonaromatic heterocycle or heteroaryl), or
R¹ and R² can be taken together with the neighboring carbon atom to form an optionally substituted 5 or 6-membered ring optionally containing heteroatom(s),
R³ is hydrogen, halogen, cyano, optionally substituted sulfamoyl, optionally substituted carbamoyl, optionally substituted amino, nitro, optionally substituted alkyl, optionally substituted alkoxy, optionally substituted alkenyl, optionally substituted aryl, optionally substituted heteroaryl, optionally substituted nonaromatic heterocycle or
a group of the formula: -C≡C-R^{3'} (wherein R^{3'} is hydrogen, optionally substituted aryl, optionally substituted heteroaryl, hydroxy or optionally substituted alkyl),
R⁴ is hydrogen, halogen, optionally substituted alkyl, optionally substituted alkoxy, optionally substituted alkenyl, optionally substituted alkynyl or optionally substituted alkylthio,
R⁵ is optionally substituted nonaromatic heterocycle,
R⁶ is hydrogen, alkyl or halogen,
R⁷ is hydrogen, optionally substituted alkoxy, halogen, cyano, nitro, optionally substituted alkyl, optionally substituted alkenyl or optionally substituted alkynyl,
R⁸ is halogen or haloalkyl, and
R⁹ is hydrogen, alkyl, halogen or optionally substituted aryl).

19. The compound of claim 18 wherein R² is halogen, a pharmaceutically acceptable salt or solvate thereof.

20. A compound of the formula II: 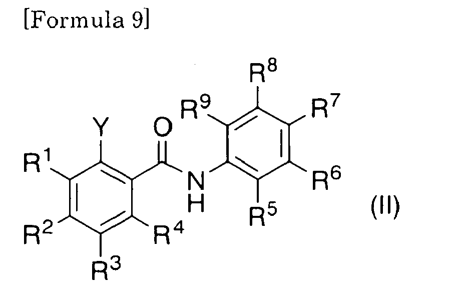 a pharmaceutically acceptable salt or solvate thereof,
(wherein Y is hydroxy or a group of the formula: -NH-SO₂-Y' (wherein Y' is optionally substituted aryl or optionally substituted alkyl),
R¹ is optionally substituted alkyl, optionally substituted amino, nitro, optionally substituted alkoxy, halogen, optionally substituted alkenyl or optionally substituted alkynyl,
R² is hydrogen, halogen, nitro, optionally substituted amino, cyano, optionally substituted alkyl, optionally substituted carbamoyl, optionally substituted alkoxy, hydroxy, optionally substituted alkenyl, optionally substituted alkynyl or optionally substituted aryl, or
R¹ and R² can be taken together with the neighboring carbon atom to form an optionally substituted 5 or 6-membered ring optionally containing heteroatom(s),
R³ is hydrogen, halogen, cyano, optionally substituted sulfamoyl, optionally substituted carbamoyl, optionally substituted amino, nitro, optionally substituted alkyl, optionally substituted alkoxy, optionally substituted alkenyl, optionally substituted aryl, optionally substituted heteroaryl, optionally substituted nonaromatic heterocycle or
a group of the formula: -C≡C-R^{3'} (wherein R^{3'} is hydrogen, optionally substituted aryl, optionally substituted heteroaryl, hydroxy or optionally substituted alkyl),
R⁴ is hydrogen, halogen, optionally substituted alkyl, optionally substituted alkoxy, optionally substituted alkenyl, optionally substituted alkynyl or optionally substituted alkylthio,
R⁵ is optionally substituted nonaromatic heterocycle,
R⁶ is hydrogen, alkyl or halogen,
R⁷ is hydrogen, optionally substituted alkoxy, halogen, cyano, nitro, optionally substituted alkyl, optionally substituted alkenyl or optionally substituted alkynyl,
R⁸ is halogen or haloalkyl, and
R⁹ is hydrogen, alkyl, halogen or optionally substituted aryl).

21. The compound of claim 20 wherein R¹ is alkyl, a pharmaceutically acceptable salt or solvate thereof.

22. The compound of claim 21 wherein R³ is halogen, a pharmaceutically acceptable salt or solvate thereof.

23. A compound of the formula II: 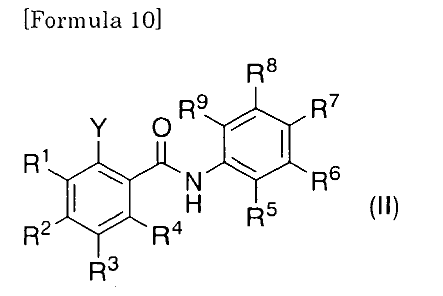 a pharmaceutically acceptable salt or solvate thereof,
(wherein Y is hydroxy or a group of the formula: -NH-SO₂-Y' (wherein Y' is optionally substituted aryl or optionally substituted alkyl),
R¹ is hydrogen, optionally substituted alkyl, optionally substituted amino, nitro, optionally substituted alkoxy, halogen, optionally substituted alkenyl or optionally substituted alkynyl,
R² is halogen,
R³ is hydrogen, halogen, cyano, optionally substituted sulfamoyl, optionally substituted carbamoyl, optionally substituted amino, nitro, optionally substituted alkyl, optionally substituted alkoxy, optionally substituted alkenyl, optionally substituted aryl, optionally substituted heteroaryl, optionally substituted nonaromatic heterocycle or
a group of the formula: -C≡C-R^{3'} (wherein R^{3'} is hydrogen, optionally substituted aryl, optionally substituted heteroaryl, hydroxy or optionally substituted alkyl),
R⁴ is hydrogen, halogen, optionally substituted alkyl, optionally substituted alkoxy, optionally substituted alkenyl, optionally substituted alkynyl or optionally substituted alkylthio,
R⁵ is a group of the formula: -X"-R^{5"} (wherein X" is -C(=O)-, -NHSO₂- -NHC(=O)-, -CH(OH)- or -NR-, R^{5"} is substituted aryl, and R is hydrogen or alkyl),
R⁶ is hydrogen, alkyl or halogen,
R⁷ is haloalkyl or haloalkoxy,
R⁸ is hydrogen, nitro, optionally substituted amino, halogen, optionally substituted alkyl, alkoxy, optionally substituted alkenyl, optionally substituted alkynyl, cyano or haloalkoxy, and
R⁹ is hydrogen, alkyl, halogen or optionally substituted aryl).

24. A compound of the formula II: 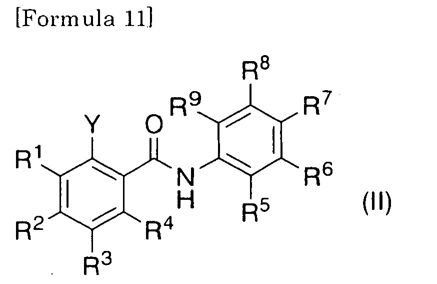 a pharmaceutically acceptable salt or solvate thereof,
(wherein Y is hydroxy,
R¹ is hydrogen,
R² is a group of the formula: -O- R^{2'} (wherein R² is optionally substituted nonaromatic heterocycle),
R³ is hydrogen,
R⁴ is hydrogen,
R⁵ is halogen, aryl or optionally substituted heteroaryl,
R⁶ is hydrogen,
R⁷ is hydrogen,
R⁸ is haloalkyl, and
R⁹ is hydrogen).

25. A compound of the formula II: 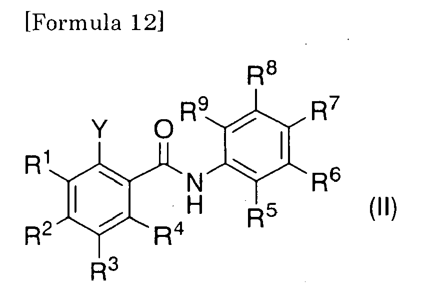 a pharmaceutically acceptable salt or solvate thereof,
(wherein Y is a group of the formula: -NH-SO₂-Y' (wherein Y' is optionally substituted aryl),
R¹ is hydrogen,
R² is hydrogen, halogen, nitro, cyano, optionally substituted carbamoyl or a group of the formula: -O-R^{2'} (wherein R² is optionally substituted alkyl),
R³ is hydrogen, halogen, nitro, cyano, optionally substituted aryl or nonaromatic heterocycle,
R⁴ is hydrogen,
R⁵ is hydrogen, halogen, optionally substituted alkyl, alkoxy, optionally substituted amino or optionally substituted nonaromatic heterocycle,
R⁶ is hydrogen, optionally substituted alkyl or halogen,
R⁷ is hydrogen, halogen or optionally substituted nonaromatic heterocycle,
R⁸ is hydrogen, halogen, haloalkyl or haloalkoxy, and
R⁹ is hydrogen).

26. The compound of any one of claim 13 to 17 wherein R⁵ is 2,4-dihalogenophenyl, a pharmaceutically acceptable salt or solvate thereof.

27. The compound of any one of claim 13 to 19, 24 and 26 wherein R² is a group of the formula: 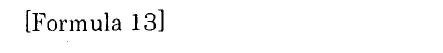 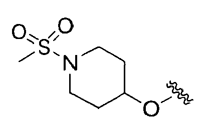 a pharmaceutically acceptable salt or solvate thereof.

28. A pharmaceutical composition comprising the compound of any one of claims 6 to 27, a pharmaceutically acceptable salt or solvate thereof.

29. The CTGF inhibitor of claim 1, wherein R² is a group of the formula:
